# EUROPEAN PATENT APPLICATION

(11) **EP 4 650 363 A1**
(43) Date of publication of application: **19.11.2025**
(21) Application number: 25176752.1
(22) Date of filing: 15.05.2025
(51) Int. Cl.: C07K 14/47, C12N 5/075, C12N 15/89

(54) **RESCUE OF CHROMOSOME COHESION IN AGED MAMMALIAN OOCYTES AND EGGS**

(30) Priority: 16.05.2024 EP 24176293; 26.02.2025 EP 25160208
(71) Applicant: Max-Planck-Gesellschaft zur Förderung der Wissenschaften e.V., 80539 München (DE)
(72) Inventor: SCHUH, Melina, 37070, Göttingen (DE); DEBOJIT, Saha, 37070, Göttingen (DE)
(74) Representative: Wichmann, Hendrik

(57) **Abstract**

The invention is in the technical field of assisted reproductive technologies. Mammalian eggs often contain an incorrect number of chromosomes, a condition referred to as aneuploidy. The inventors identified mechanisms that lead to aneuploidy in mammalian eggs, which guided the development of methods to prevent aneuploidy in mammalian oocytes and eggs. This invention relates to the in vitro introduction of several cohesion protection factors, such as Shugoshin (Sgo1), Shugoshin 2 (Sgo2), PP2A and/or pericentromeric RNA, individually and in combination, into the oocyte or egg, as these factors protect sister chromatid cohesion and thus reduce aneuploidy. Sgo1 and/or Sgo2 are also described for use in a method of preventing chromosomal aberrations.

## Description

### Background of invention

Mammalian eggs often contain an incorrect number of chromosomes, a condition referred to as aneuploidy. Aneuploidy results from chromosome segregation errors during the meiotic divisions of the egg's progenitor cell, called an oocyte (1,2). These errors increase dramatically with age, leading to an increased risk of miscarriage, infertility, and congenital disorders (3-7). It is well established in the art that a decline in female fertility is associated with an increase in aneuploidy in eggs. This is particularly relevant for aged eggs. As a result, women over the age of 35 often fail to conceive. *In vitro* fertilization (IVF) and related assisted reproductive technologies (ART) have helped to restore fertility in couples who are unable to conceive or infertile. However, higher rates of aneuploidy in aged eggs lead to lower IVF success rates. A leading age-related cause of aneuploidy is the premature separation of sister chromatids (PSSC) (2,3,6-8). The premature separation of sister chromatids is assumed to be the leading cause of aneuploidy in mammalian eggs in general. Currently, there are no established methods to prevent aneuploidy in eggs used in assisted reproductive technologies.

Single chromatids have been observed in both aged mouse and human eggs, but the timing and mechanism of their formation are incompletely understood (9-11). Once dissociated, the individual chromatids segregate randomly, causing chromosome segregation errors during both meiosis I (MI) and meiosis II (MII) (7,10,12). When the aneuploid egg or an egg with PSSC is fertilized, the errors are passed on to the embryo (13). In humans, this often leads to a halt of embryonic development, implantation failure, miscarriages, or lead to a variety of congenial disorders, in particular trisomies such as Down syndrome, Patau syndrome, Edwards syndrome or monosomies such as Turner syndrome.

Aneuploidy in eggs has been linked to an age-related loss of cohesin from chromosomes. Cohesin, a multisubunit protein complex, links chromosomes during the process of meiosis and is essential for accurate chromosome segregation (14-16). In mammalian oocytes, cohesin is loaded onto chromosomes at the fetal stage, with little or no turnover later (17,18). With age, cohesin is gradually lost from chromosomes. This loss of cohesin has been suggested to cause the premature dissociation of chromosomes in oocytes (19-21), as well as additional changes in chromosome architecture, including an increase in interkinetochore distances and kinetochore fragmentation, which further promote oocyte aneuploidy by allowing for the formation of incorrect kinetochore-microtubule attachments (3,8,21-24).

The mechanisms that lead to the loss of cohesin from chromosomes with age are not well understood. However, whether specific age-related molecular changes promote cohesin loss is unclear. More is known about the mechanisms that drive the controlled release of cohesin from chromosomes at anaphase onset. During meiosis, the meiosis-specific alpha-kleisin subunit of the cohesin ring, Rec8, is cleaved in a stepwise manner by a protease called separase (25-28), facilitating chromosome segregation during anaphase. Rec8 is cleaved along the chromosome arms during anaphase I, but must be protected in the centromeric and pericentromeric region until metaphase II to ensure that sister chromatids remain associated until anaphase II (27,29,30).

The protection of centromeric cohesin during anaphase I in mouse oocytes is mediated by the protein Shugoshin 2 (Sgo2) (31-33). As a result, only homologous chromosome pairs are separated at meiosis I, whereas sister chromatids are segregated at meiosis II.

The Shugoshin protein family contains another protein called Shugoshin 1 (Sgo1) (31,32,34). Sgo1 has been reported to be important for accurate chromosome segregation during mitosis (35-37). Although Sgo1 is expressed in mouse oocytes, its function has remained unclear. Previous RNA interference studies in mammalian oocytes have reported conflicting results for a role of Sgo1 during meiosis in mammalian oocytes. While one study reports depletion of Sgo1 leads to minor errors and no change in PP2A recruitment to the centromeres, the other study reports an increase in aneuploidy upon Sgo1 depletion in mouse oocytes (32,38).

Centromeres have traditionally been thought to be heterochromatic, but it has recently been shown that centromeres are transcriptionally active (36-44). Active centromeric transcription is required for kinetochore assembly, function, and stability by recruiting centromeric proteins through opening of centromeric chromatin (40,44-46). Centromeric transcription is tightly regulated during mouse spermatogenesis, where MIWI and Dicer-mediated cleavage of satellite repeats prevents overexpression of these repeats, thereby ensuring normal kinetochore assembly and chromosome segregation (47,48).

It has been proposed that centromeric transcription may help Sgo1 localize to the inner centromere and maintain centromeric cohesion in human mitotic cells (46,49,50). Minor satellite transcripts regulate the spindle assembly checkpoint in response to centromere structural integrity during female meiosis (51). However, it is unknown if centromeric transcription is important for protecting sister chromatid cohesion in mammalian oocytes.

Hence, there is a need in the art to prevent aneuploidy in aged mammalian oocytes and eggs. In particular, there is a need in the art to prevent premature sister chromatid separation in aged mammalian oocytes and eggs.

### Summary of the invention

The inventors identified the mechanisms that lead to PSSC in aged mammalian eggs. Elucidating these molecular mechanisms behind PSSC guided the development of methods preventing aneuploidy in mammalian oocytes and eggs as disclosed herein, which address a long-standing unmet need in the field of assisted reproductive technologies.

Herein, the inventors demonstrate that several cohesion protection factors, such as Shugoshin (Sgo1), Shugoshin 2 (Sgo2), PP2A and/or pericentromeric RNA are reduced in aged oocytes. Aged oocytes have a higher frequency of PSSC and occurrence of single chromatids, which are the main causes of aneuploidy. The inventors found that the introduction of Sgo1, Sgo2 and/or centromeric RNA to oocytes, individually and in combination, can protect sister chromatid cohesion and reduce PSSC in aged oocytes.

The invention is defined by the claims.

### Description of the Figures

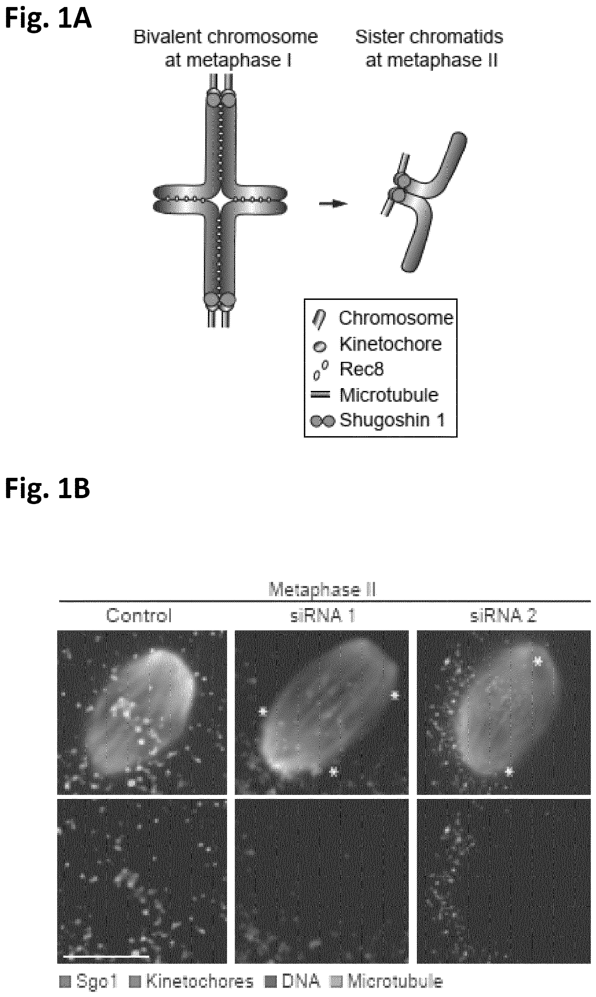

**Fig. 1A** Schematic representation of the localization of the cohesin subunit Rec8 and the centromeric cohesin protector Sgo1 in mouse oocytes at metaphase I (left panel) and eggs at metaphase II (right panel).
**Fig. 1B** Loss of Sgo1 from early-stage eggs results in increased PSSC. Representative images of metaphase II chromosomes from eggs treated with negative control (left panel), Sgo1 siRNA 1 (middle panel) and Sgo1 siRNA 2 (right panel) show that Sgo1 is lost upon knockdown. White asterisks indicate the presence of single chromatids. Kinetochores (CREST), DNA (Hoechst) and microtubules are shown in the top panel. Scale bar: 10 µm.

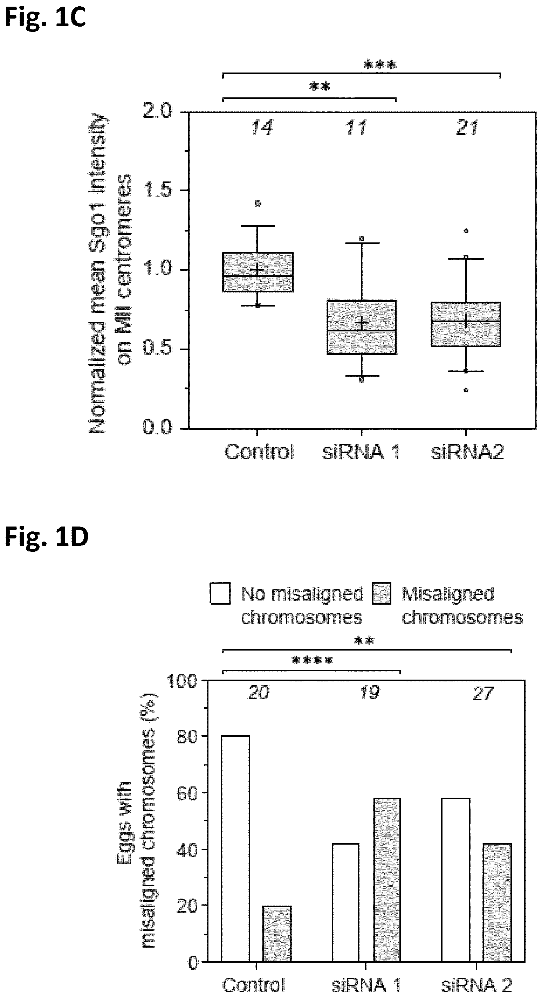

**Fig. 1C** Mean fluorescence intensity of Sgo1 at centromeres of MII eggs after Sgo1 knockdown. Plots show n = number of eggs analyzed at the top. mean (plus), median (horizontal black line), 25th and 75th percentiles (boxes), 10th and 90th percentiles (whiskers), and the 1st and 99th percentiles (circles). Statistical significance was measured by the Mann-Whitney test, **p≤0.01, ***p≤0.001.
**Fig. 1D** The percentage of eggs with misaligned chromosomes increases after Sgo1 knockdown with siRNA 1 and siRNA 2. Plots show n = number of eggs analyzed at top. Statistical significance was measured by Fisher's exact test on the number of eggs with or without misaligned chromosomes, ****p (Control vs siRNA1) = 0.0189; **p (Control vs siRNA2) = 0.2065.

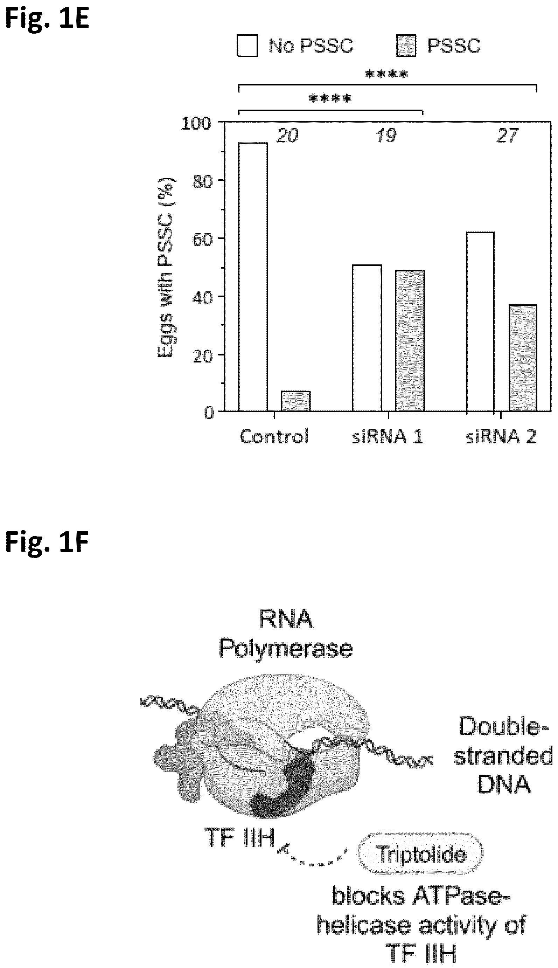

**Fig. 1E** The percentage of eggs with PSSC increases after Sgo1 knockdown with siRNA 1 and siRNA 2. Plots show n = number of eggs analyzed at top. Statistical significance was measured by Fisher's exact test on the number of eggs with or without PSSC, p-value (Control vs siRNA 1) = 0.0033, p-value (Control vs siRNA 2) = 0.0141.
**Fig. 1F** Schematic representation of triptolide function in inhibiting transcription by RNA polymerase.

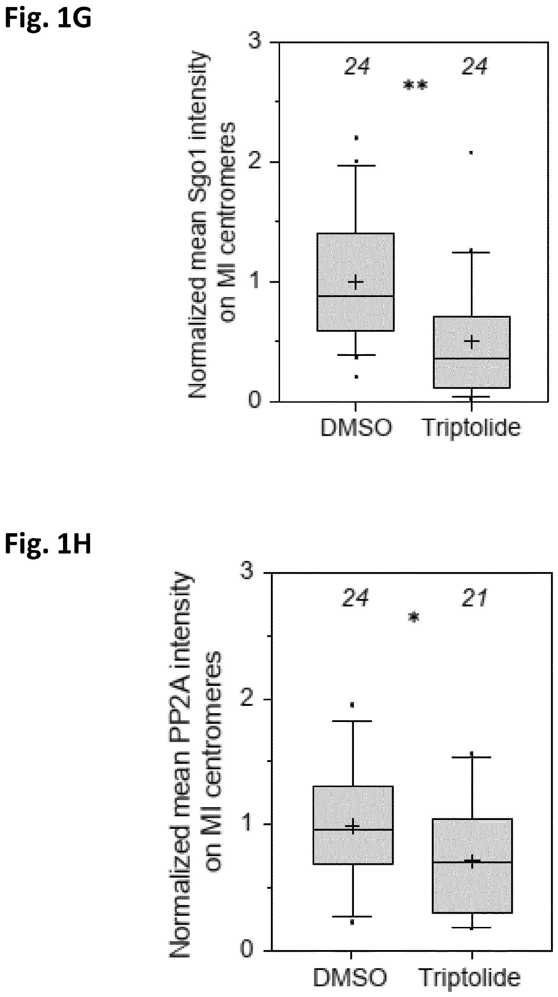

**Fig. 1G** Triptolide reduces Sgo1 on metaphase I centromeres compared to control. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test, **p≤0.01.
**Fig. 1H** Triptolide reduces PP2A on metaphase I centromeres compared to control. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test. *p≤0.05.

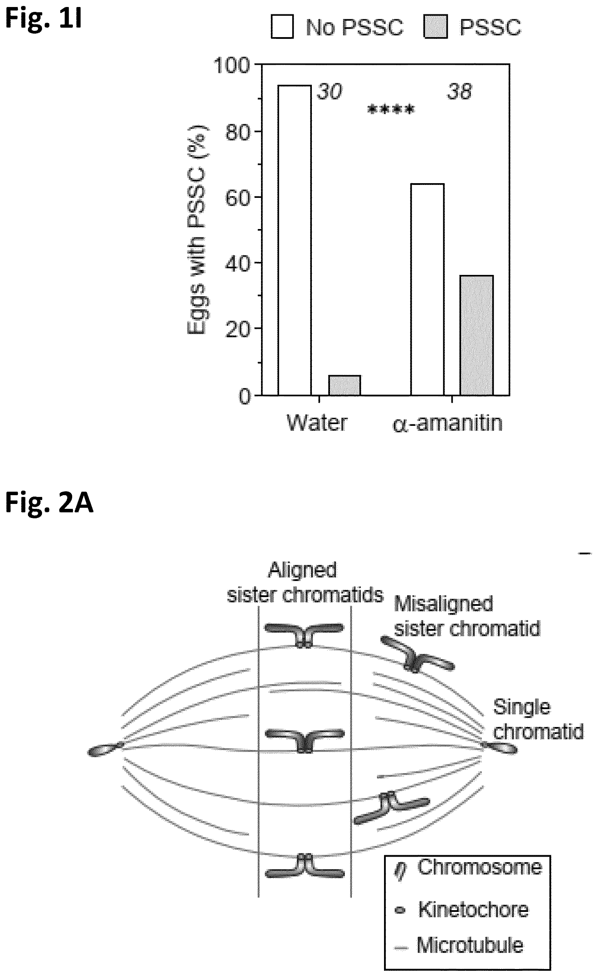

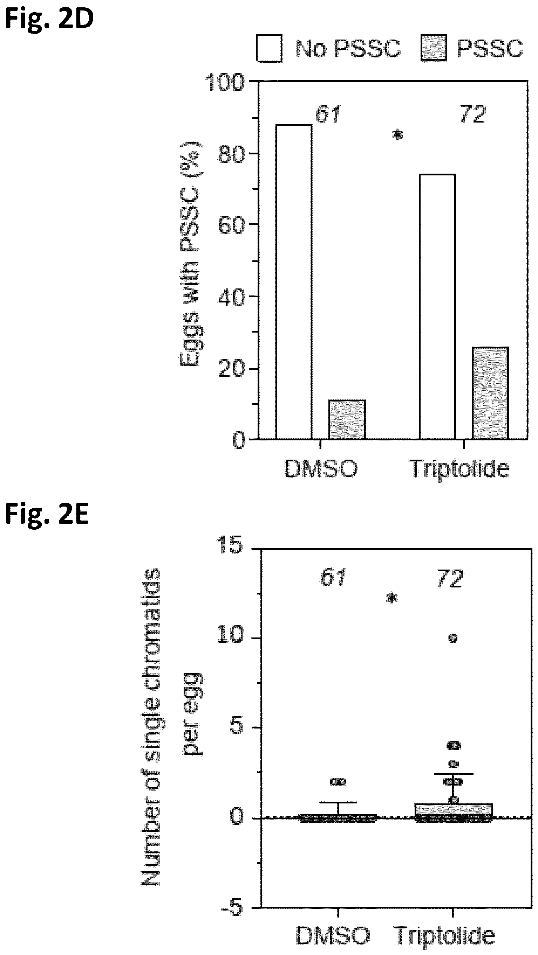

**Fig. 1I** Frequency of PSSC with α-amanitin measured by scoring the percentage of eggs with PSSC, similar to levels observed with triptolide (Fig. 2E). Plots show number of eggs analyzed on top. Statistical significance is measured by Fisher's exact test on the number of eggs with or without PSSC, p-value (water vs α-amanitin) = 0.1491.
**Fig. 2A** Schematic of sister chromatid alignment at the mouse metaphase II spindle. While sister chromatids are usually accurately aligned at the metaphase plate, prematurely separated chromatids are often misaligned due to unbalanced spindle microtubule tension.

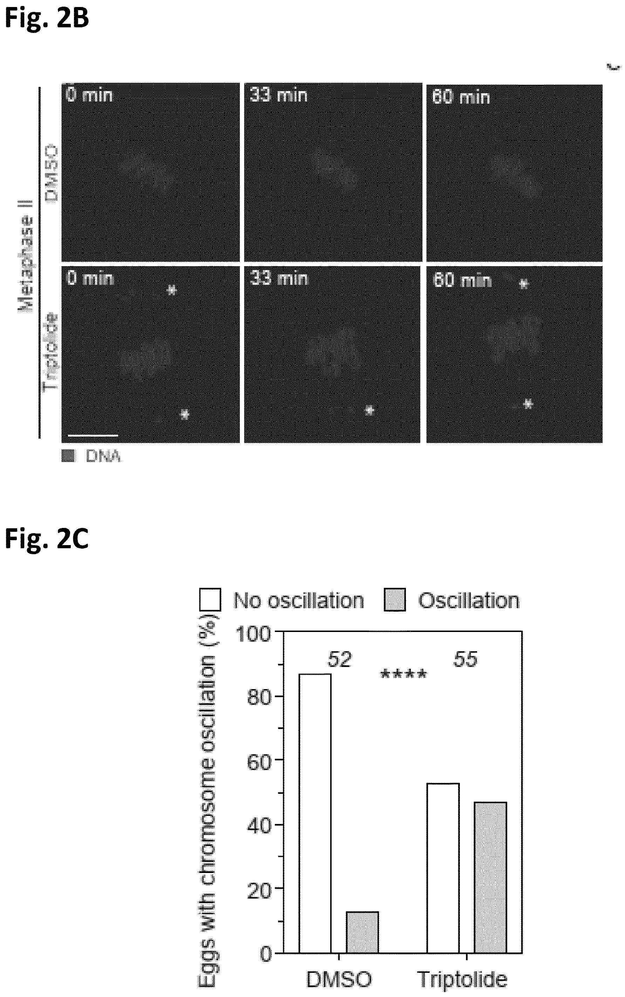

**Fig. 2B** Metaphase II chromosomes in DMSO (control) (upper panel) and triptolide-treated (lower panel) eggs imaged by time-lapse imaging at T= 0, 33, and 60 min at metaphase II. White asterisks indicate oscillating chromosomes outside the metaphase plate, scored according to the scheme in (Fig. 2A).
**Fig. 2C** The percentage of eggs with oscillating chromosomes in control and triptolide-treated oocytes. Plots show number of eggs analyzed at top. Statistical significance measured by Fisher's exact test on the number of eggs with or without oscillating chromosomes, p-value (DMSO vs Triptolide) = 0.0002.
**Fig. 2D** Frequency of PSSC measured by scoring the percentage of eggs with PSSC. Plots show number of eggs analyzed on top. Statistical significance is measured by Fisher's exact test on the number of eggs with or without PSSC, p-value (DMSO vs Triptolide) = 0.0744.
**Fig. 2E** Number of single chromatids in eggs treated with DMSO and triptolide. Bars show the mean and standard deviation (black vertical line), individual values (dots). Statistical significance was measured by Kolmogorov-Smirnov test, *p≤0.05.

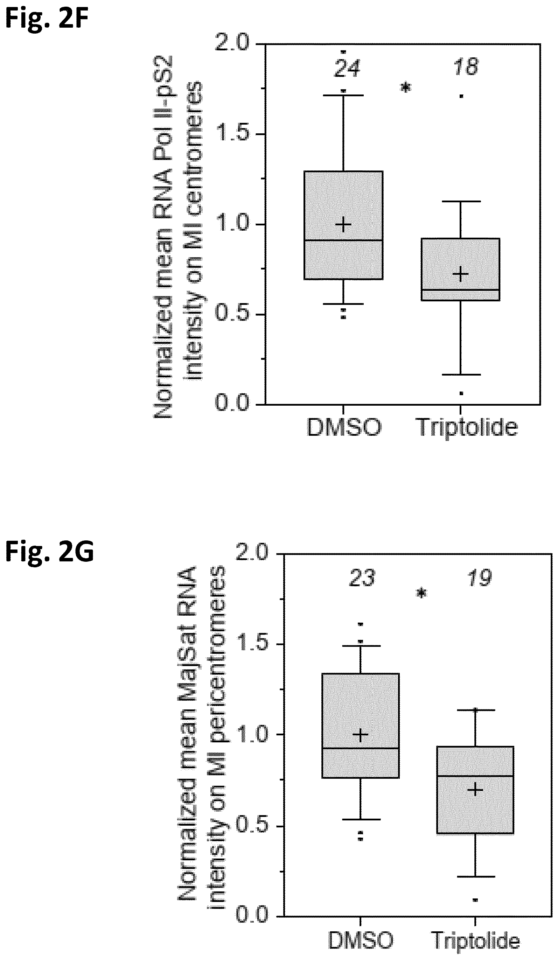

**Fig. 2F** Centromeric transcription is reduced in triptolide-treated oocytes compared to controls. Relative intensity of the centromeric pool of actively elongating RNA Pol II from DMSO- and triptolide-treated oocytes, plots show n = number of oocytes analyzed at top, mean (plus), median (horizontal black line), 25th and 75th percentiles (boxes), 10th and 90th percentiles (whiskers), and the 1st and 99th percentiles (circles). statistical significance measured by Kolmogorov-Smirnov test, *p≤0.05.
**Fig. 2G** The relative intensity of MajSat RNA at the pericentromere from DMSO- and triptolide-treated oocytes, plots shown, number of oocytes analyzed at top, showing MajSat RNA reduction upon triptolide treatment. Statistical significance measured by Mann-Whitney test. *p≤0.05

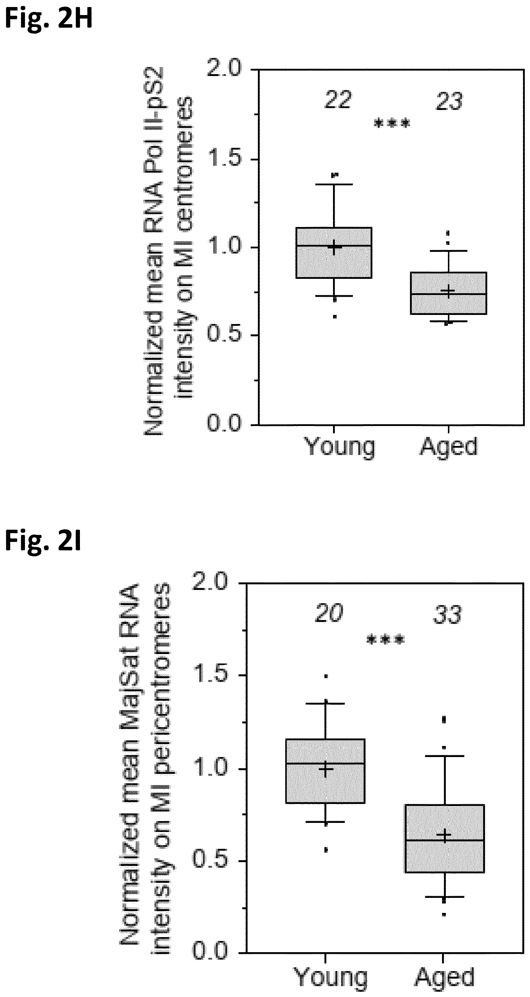

**Fig. 2H** Relative intensity of the pericentromeric pool of actively elongating RNA Pol II from young and aged MI oocytes, plots show plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test, ***p≤0.001.
**Fig. 2I** MajSat RNA is significantly reduced on pericentromeres in aged MI oocytes compared to young oocytes. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test, ***p≤0.001.

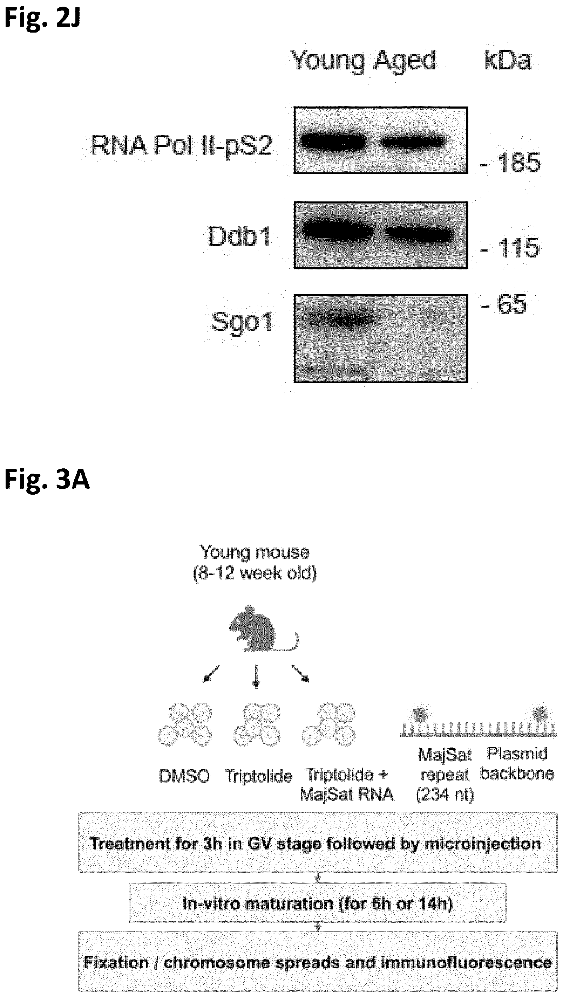

**Fig. 2J** Levels of RNA Pol II-pS2, Ddb1 and Sgo1 observed on immunoblots of young and aged oocytes at the germinal vesicle stage. Sgo1 protein levels show a pronounced reduction in aged oocytes, whereas RNA Pol II-pS2 shows a partial reduction compared to the control (Ddb1).
**Fig. 3A** Schematic of chromosome error-reduction assay in mouse eggs upon triptolide treatment.

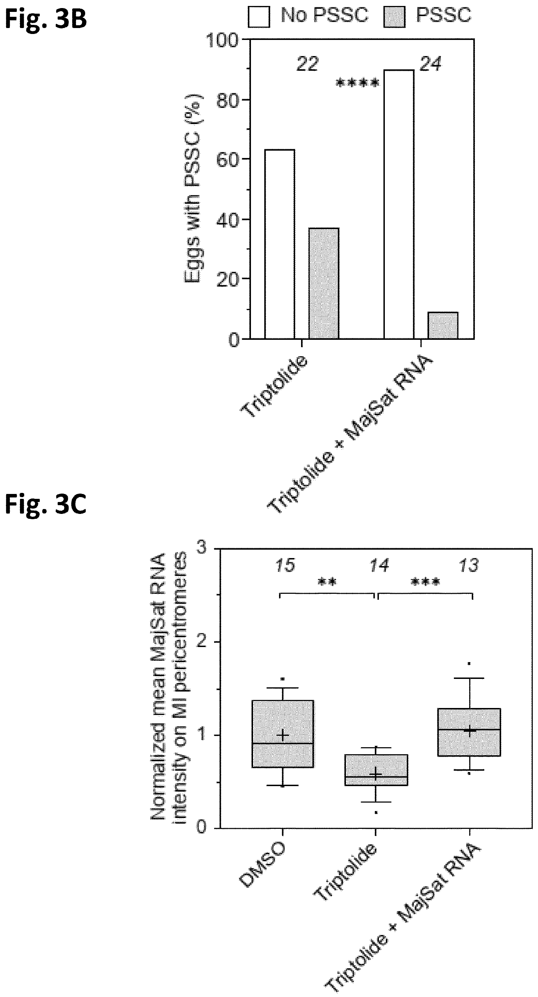

**Fig. 3B** Frequency of PSSC measured by scoring the number of single chromatids per egg in triptolide-treated eggs and triptolide-treated eggs rescued with MajSat RNA, showing the effect of MajSat supplementation in eggs with reduced transcription. Plots show number of oocytes analyzed on top. Statistical significance is measured by Fisher's exact test on the number of eggs with or without PSSC, p-value (Triptolide vs Triptolide + Majsat RNA) = 0.0304.
**Fig. 3C** Rescue of MajSat RNA levels in triptolide-treated oocytes after exogenous delivery of MajSat RNA. The relative intensity of MajSat RNA at the pericentromere from DMSO and both uninjected and injected triptolide-treated MI oocytes. Plots shown, number of oocytes analyzed at top, statistical significance measured by Mann-Whitney test. **p≤0.01, ***p≤0.001.

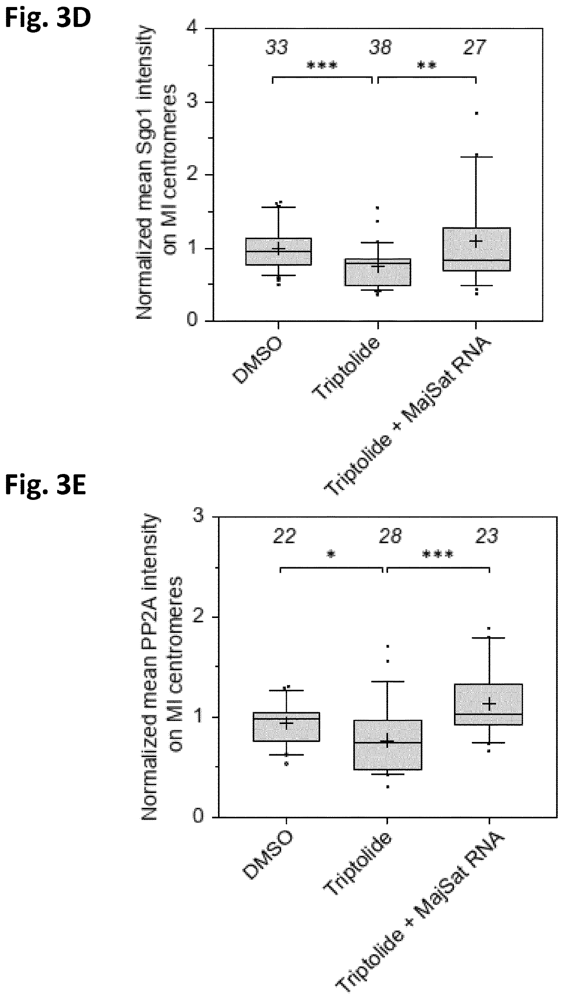

**Fig. 3D** Normalized mean Sgo1 intensity on MI centromeres in DMSO-treated, triptolide-treated and triptolide-treated after exogenous delivery of MajSat RNA oocytes. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test. **p≤0.01, ***p≤0.001.
**Fig. 3E** Normalized mean PP2A intensity on MI centromeres in DMSO-treated, triptolide-treated and triptolide-treated after exogenous delivery of MajSat RNA oocytes. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance measured by Mann-Whitney test. *p≤0.05, ***p≤0.001.

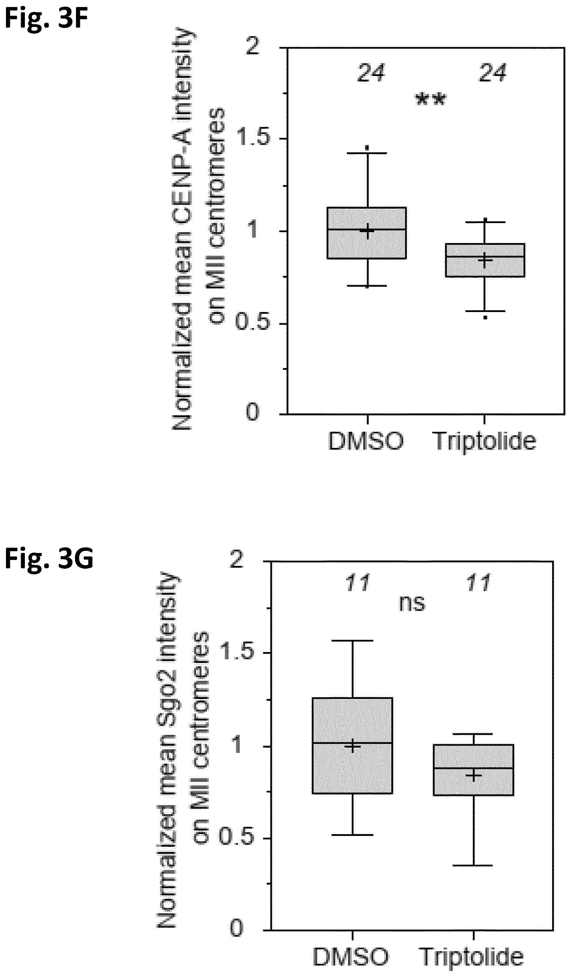

**Fig. 3F** Triptolide reduces CENP-A at metaphase II centromeres. Plots show n = number of eggs analyzed at top, mean (plus), median (horizontal black line), 25th and 75th percentiles (boxes), 10th and 90th percentiles (whiskers), and the 1st and 99th percentiles (circles). Statistical significance is measured by Mann-Whitney test, **p≤0.01.
**Fig. 3G** Normalized Sgo2 intensity in DMSO-treated and Triptolide-treated eggs. Plots shown n = number of eggs analyzed at top, mean (plus), median (horizontal black line), 25th and 75th percentiles (boxes), 10th and 90th percentiles (whiskers), and the 1st and 99th percentiles (circles). Statistical significance is measured by Mann-Whitney test, ns = not significant, p = 0.48.

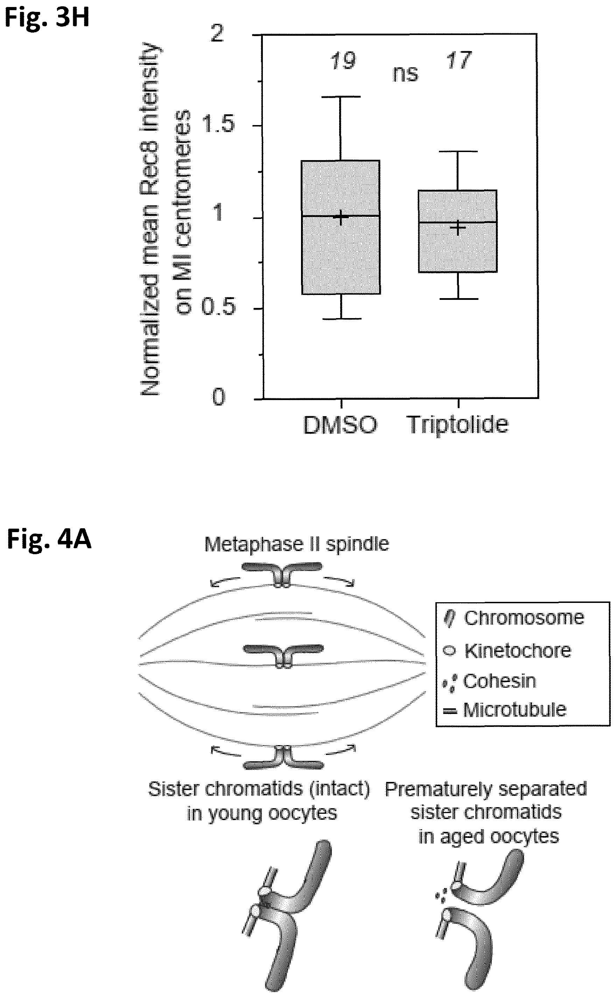

**Fig. 3H** Normalized Rec8 intensity in DMSO- and triptolide treated oocytes in metaphase I. Plots shown n = number of oocytes analyzed at top, mean (plus), median (horizontal black line), 25th and 75th percentiles (boxes), 10th and 90th percentiles (whiskers), and the 1st and 99th percentiles (circles). Statistical significance is measured by Mann-Whitney test, ns = not significant, p = 0.64.
**Fig. 4A** Schematic of sister chromatids on the mouse metaphase II spindle (top panel). Centromeric cohesion holds sister chromatids together in young eggs (lower left panel). Cohesin is lost from chromosomes with age, leading to premature separation of sister chromatids (PSSC) (lower right panel).

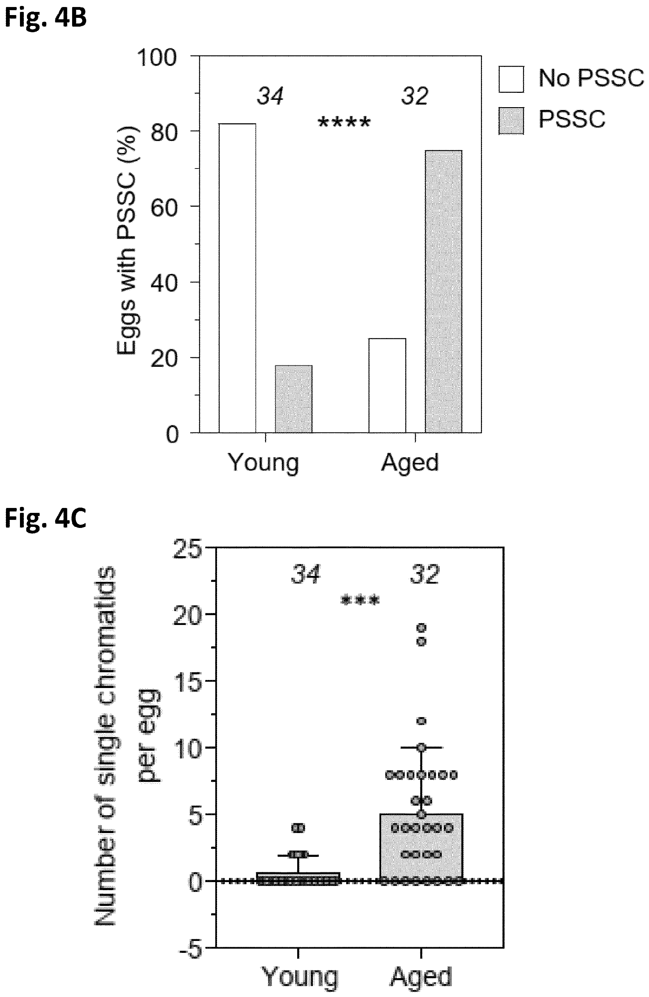

**Fig. 4B** The percentage of eggs with PPSCs in eggs from young (8-12 weeks old) and aged mice (60-65 weeks old). n, number of eggs analysed at top, statistical significance measured by Fisher's exact test on the number of eggs with or without PSSC. ****p-value (Young vs Aged) ≤0.0001.
**Fig. 4C** Frequency of PSSC measured by scoring the number of single chromatids per egg. Bars show the mean and standard deviation (black vertical line), individual values (blue dots). Statistical significance was measured using the Kolmogorov-Smirnov test. ***p≤0.001.

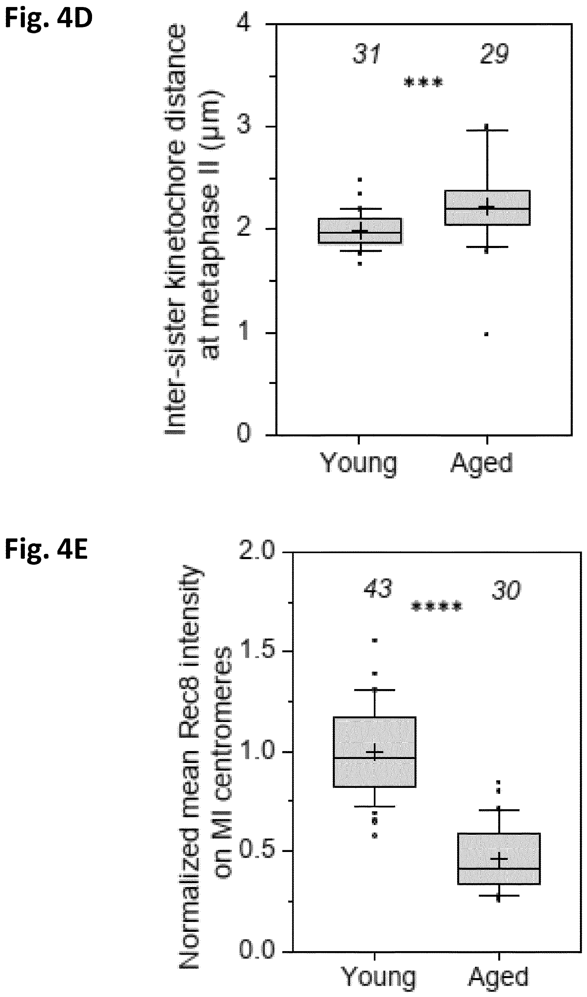

**Fig. 4D** Age-related increase in interkinetochore distance. Plots show mean (plus), median (horizontal black line), 10th - 90th percentiles. Statistical significance was measured using the Mann-Whitney test. ***p≤0.001.
**Fig. 4E** The relative intensity of the centromeric pool of Rec8 from young and aged oocytes, showing significant reduction of Rec8 in aged oocytes, compared to young oocytes. Plots show n, number of oocytes analysed at top, statistical significance measured by Kolmogorov-Smirnov test, ****p≤0.0001.

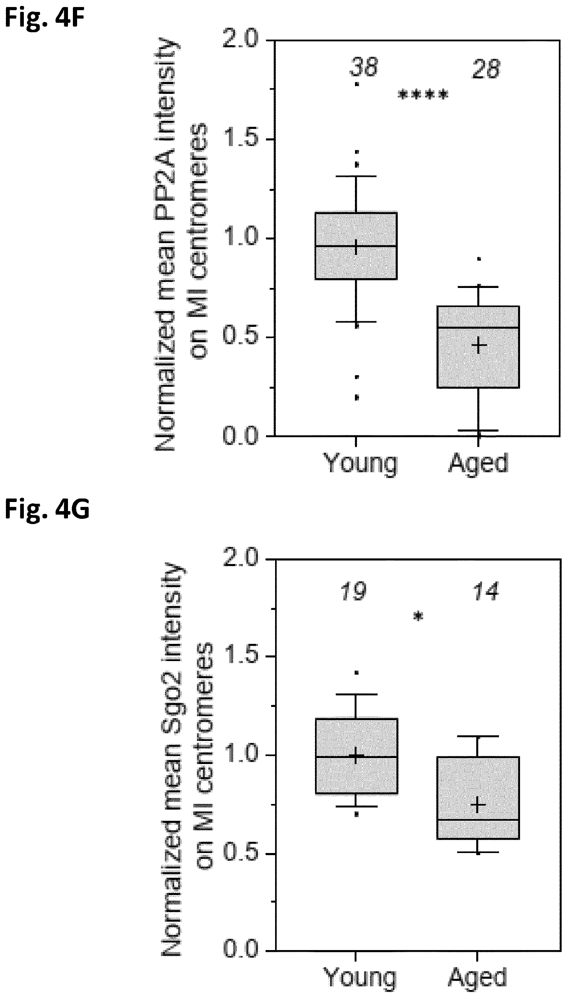

**Fig. 4F** The relative intensity of the centromeric pool of PP2A from young and aged oocytes, plots show n, number of oocytes analysed on top, statistical significance measured by Kolmogorov-Smirnov test, ****p≤0.0001.
**Fig. 4G** The relative intensity of the centromeric pool of Sgo2 from young and aged oocytes, plots show n, number of oocytes analysed on top, statistical significance measured by Kolmogorov-Smirnov test, *p≤0.05.

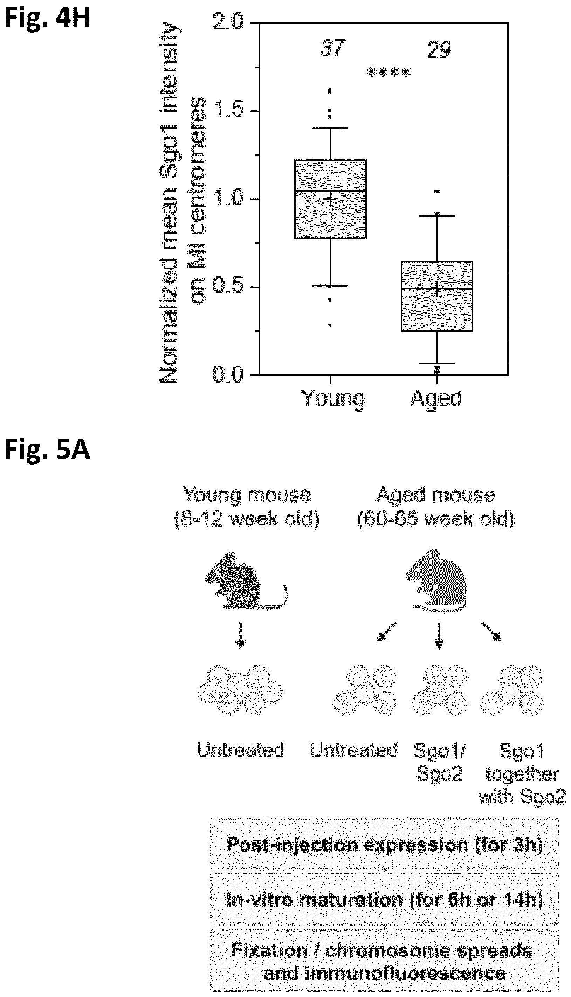

**Fig. 4H** The relative intensity of the centromeric pool of Sgo1 from young and aged oocytes, plots show n, number of oocytes analysed on top, statistical significance measured by Kolmogorov-Smirnov test, ****p≤0.0001.
**Fig. 5A** Schematic of the assay for PSSC reduction in aged mouse eggs.

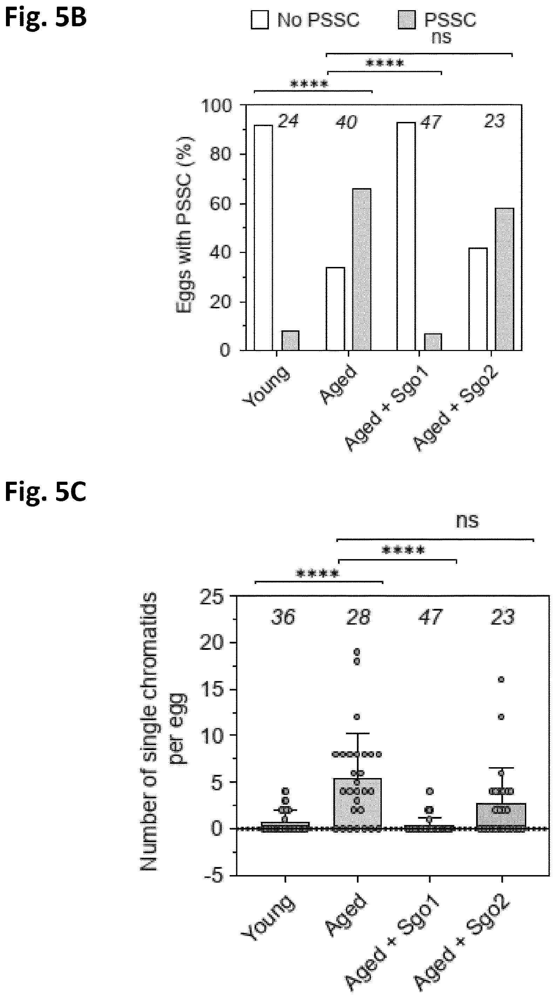

**Fig. 5B** Percentage of eggs with PSSC after supplementation with Sgo1 or Sgo2. Plots show n = number of eggs analyzed at top. Statistical significance is measured by Fisher's exact test on the number of eggs with or without PSSC . p-value (Young vs Aged) ≤0.0001, p-value (Aged vs Aged + Sgo1) ≤0.0001, p-value (Aged vs Aged + Sgo2) = 0.3034.
**Fig. 5C** Frequency of PSSC in aged eggs supplemented with Sgo1 or Sgo2. Plots show n = number of eggs analyzed at top, mean and standard deviation (black vertical line), individual values (dots). Statistical significance is measured by Kolmogorov-Smirnov test. ns = not significant, ****p≤0.0001

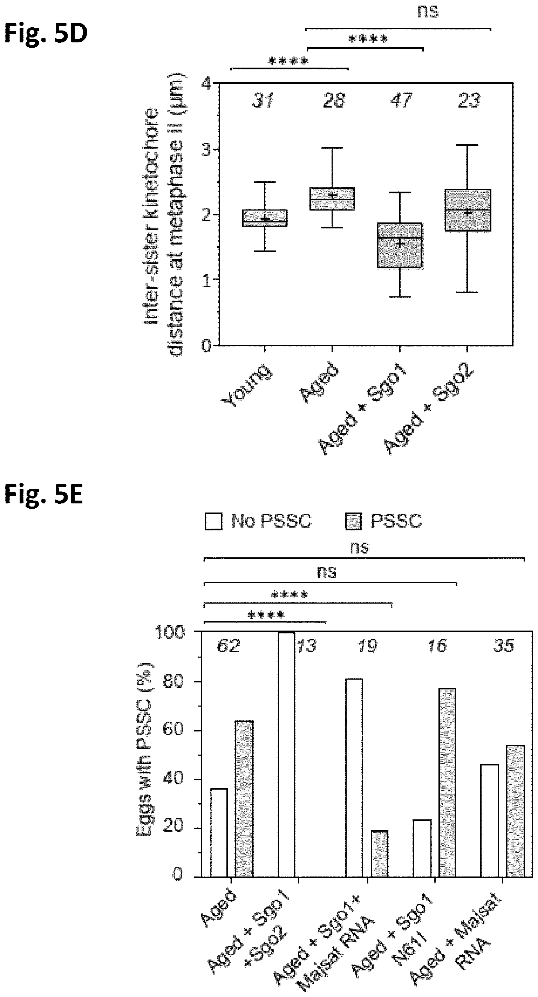

**Fig. 5D** Measurements of interkinetochore distances between sister chromatids of metaphase II chromosomes in mouse eggs injected with the combination of RNAs indicated in (5C). Plots show n = number of eggs analyzed at top, mean (plus), median (horizontal black line), 25th and 75th percentiles (boxes), 10th and 90th percentiles (whiskers), and the 1st and 99th percentiles (circles). Statistical significance was measured by Kolmogorov-Smirnov test. ns = not significant, ****p≤0.0001.
**Fig. 5E** Percentage of eggs with PSSC in aged eggs supplemented with Sgo1 together with Sgo2, Sgo1 together with MajSat RNA, Sgo1 N61I mutant, and MajSat RNA alone. Plots show n = number of eggs analyzed at top. Statistical significance is measured by Fisher's exact test on the number of eggs with or without PSSC. p-value (Aged vs Aged + Sgo1 + Sgo2) ≤0.0001, p-value (Aged vs Aged + Sgo1 + Majsat RNA) = 0.0002, p-value (Aged vs Aged + Sgo1 N61I) > 0.9999, p-value (Aged vs Aged + Majsat RNA) = 0.3837.

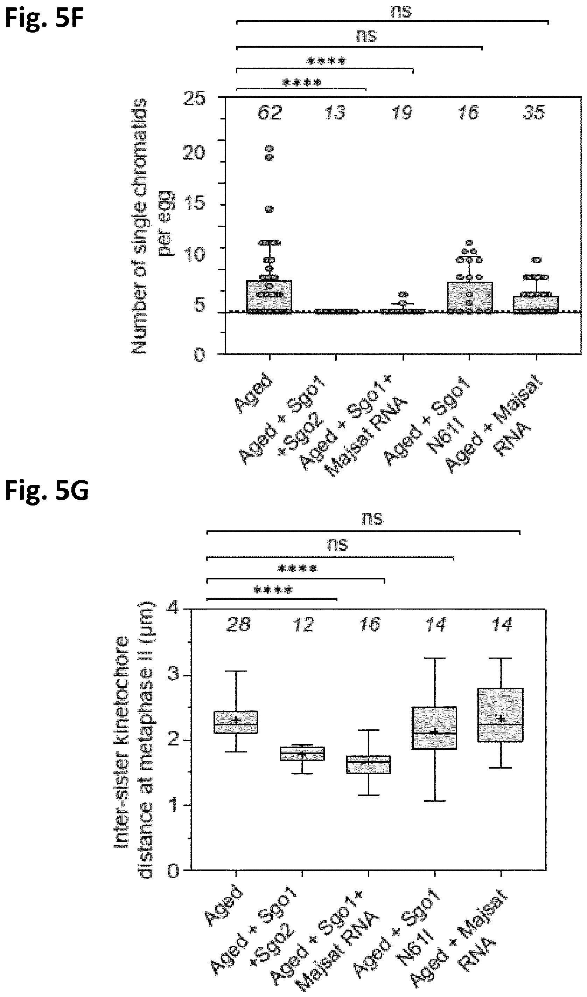

**Fig. 5F** Number of single chromatids per egg in aged eggs supplemented with Sgo1 together with Sgo2, Sgo1 together with MajSat RNA, Sgo1 N61I mutant, and MajSat RNA alone. Scatter plots show n = number of eggs analyzed at top, mean and standard deviation (black vertical line), individual values (dots). Statistical significance is measured by Kolmogorov-Smirnov test. ns = not significant, ****p≤0.0001
**Fig. 5G** Measurements of interkinetochore distances between sister chromatids of metaphase II chromosomes in mouse eggs injected Sgo1 together with Sgo2, Sgo1 together with MajSat RNA, Sgo1 N61I mutant, and MajSat RNA alone. Plots show n = number of eggs analyzed at top, mean (plus), median (horizontal black line), 25th and 75th percentiles (boxes), 10th and 90th percentiles (whiskers), and the 1st and 99th percentiles (circles). Statistical significance was measured by Kolmogorov-Smirnov test. ns = not significant, ****p≤0.0001.

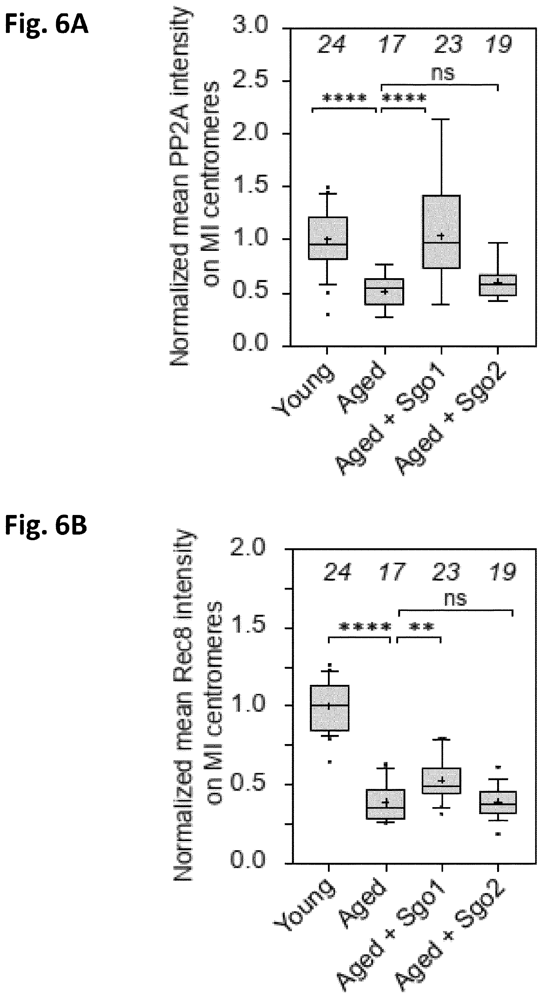

**Fig. 6A** **-** **Fig. 6B** Sgo1 rescues centromeric Rec8 and PP2A levels in aged oocytes, but Sgo2 does not. Plots show n = number of oocytes analyzed at top, mean (plus), median (horizontal black line), 25th and 75th percentiles (boxes), 10th and 90th percentiles (whiskers), and the 1st and 99th percentiles (circles). Statistical significance is measured by Kolmogorov-Smirnov test. ns = not significant, *p≤0.05, **p≤0.01, ****p≤0.0001.

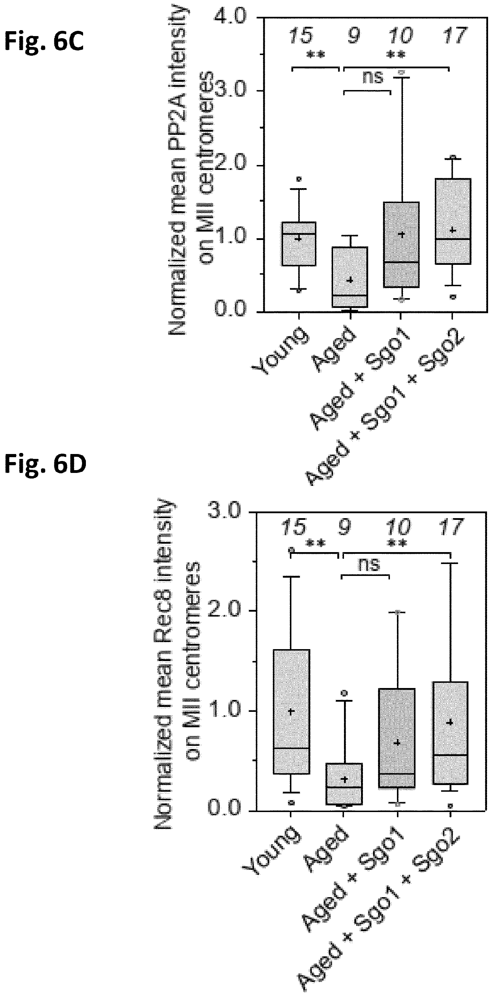

**Fig. 6C** **-** **Fig. 6D****.** Co-expression of Sgo1 and Sgo2 rescues centromeric Rec8 and PP2A levels in aged eggs, but Sgo1 alone does not. Plots show n = number of eggs analyzed at top, mean (plus), median (horizontal black line), 25th and 75th percentiles (boxes), 10th and 90th percentiles (whiskers), and the 1st and 99th percentiles (circles). Statistical significance is measured by Kolmogorov-Smirnov test. ns = not significant, *p≤0.05, **p≤0.01, ****p≤0.0001.

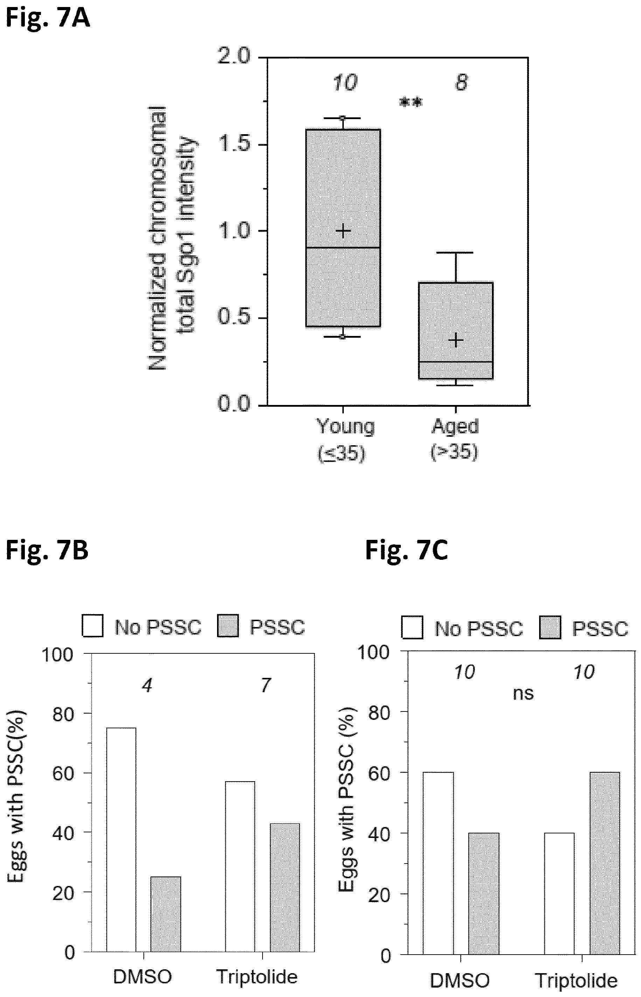

**Fig. 7A** The relative intensity of chromosomal Sgo1 in metaphase I oocytes from young and aged women shows a significant reduction in Sgo1 levels. Plots show mean (plus), median (horizontal black line), 25th and 75th percentiles (boxes), 10th and 90th percentiles (whiskers), and the 1st and 99th percentiles (circles). P values were calculated using the Kolmogorov-Smirnov test, P = 0.007.
**Fig. 7B and 7C** Frequency of PSSC scored as percentage of eggs with PSSC in DMSO- and triptolide-treated human eggs. Plots show n = number of eggs analyzed at top. For Fig. 7C, P value was calculated using Fisher's exact test on the number of eggs with or without PSSC, ns (DMSO vs Triptolide) = 0.6563.

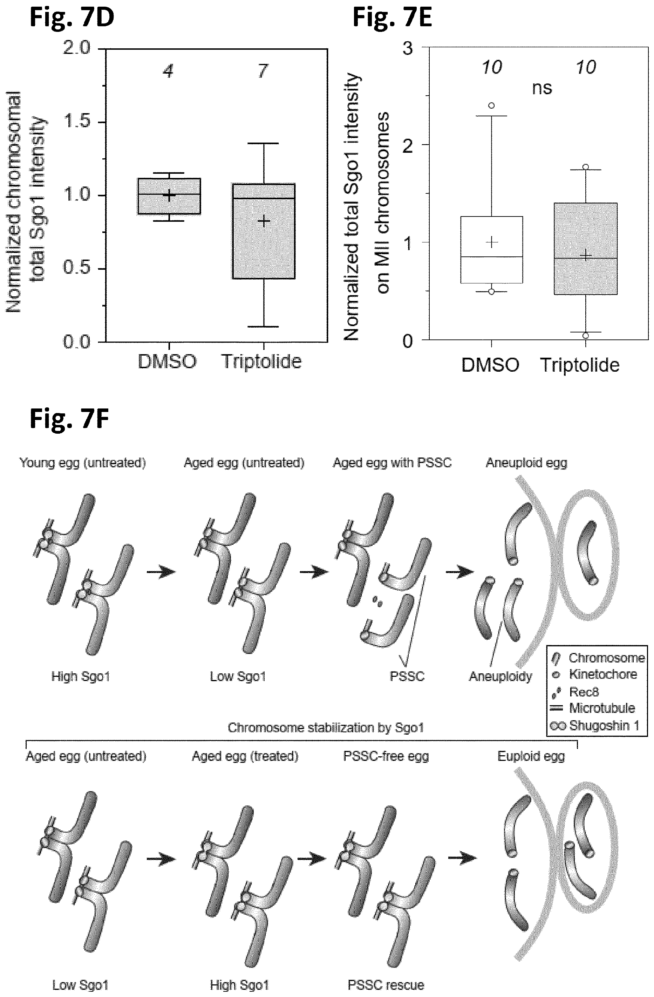

**Fig. 7D and 7E** Bar graph showing the relative intensity of the chromosome pool of Sgo1 in human eggs treated with DMSO or triptolide. Plots show n = number of eggs analyzed at top, mean (plus), median (horizontal black line), 25th and 75th percentiles (boxes), 10th and 90th percentiles (whiskers), and the 1st and 99th percentiles (circles). For Fig. 7E, P value was calculated using the Mann-Whitney test, ns (DMSO vs Triptolide) = 0.6305.
**Fig. 7F** Schematic representation of error-prone chromosome segregation during meiotic progression in aged oocytes leading to PSSC. Sgo expression can rescue PSSC errors in aged oocytes by protecting sister chromatid cohesion, thereby reducing aneuploidy.

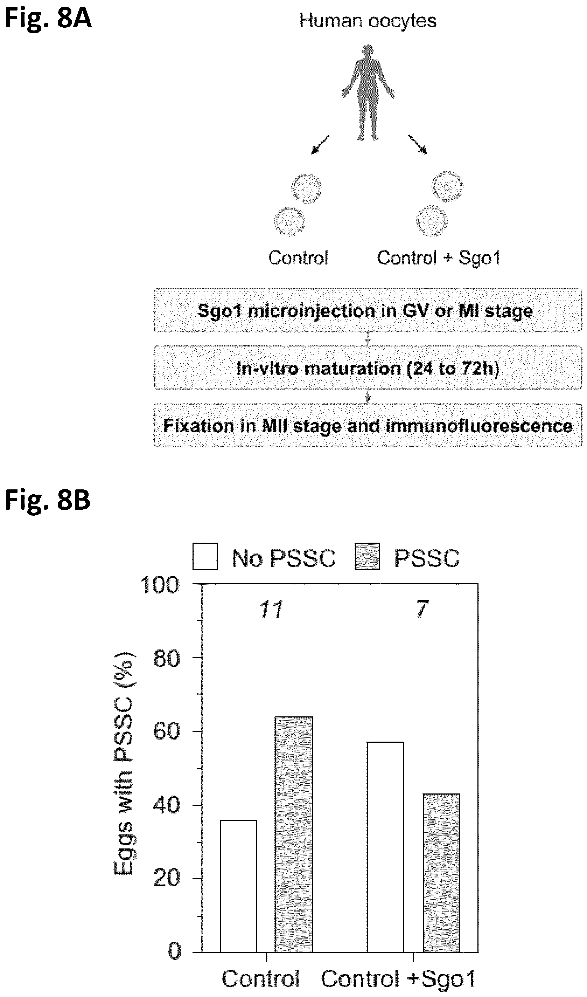

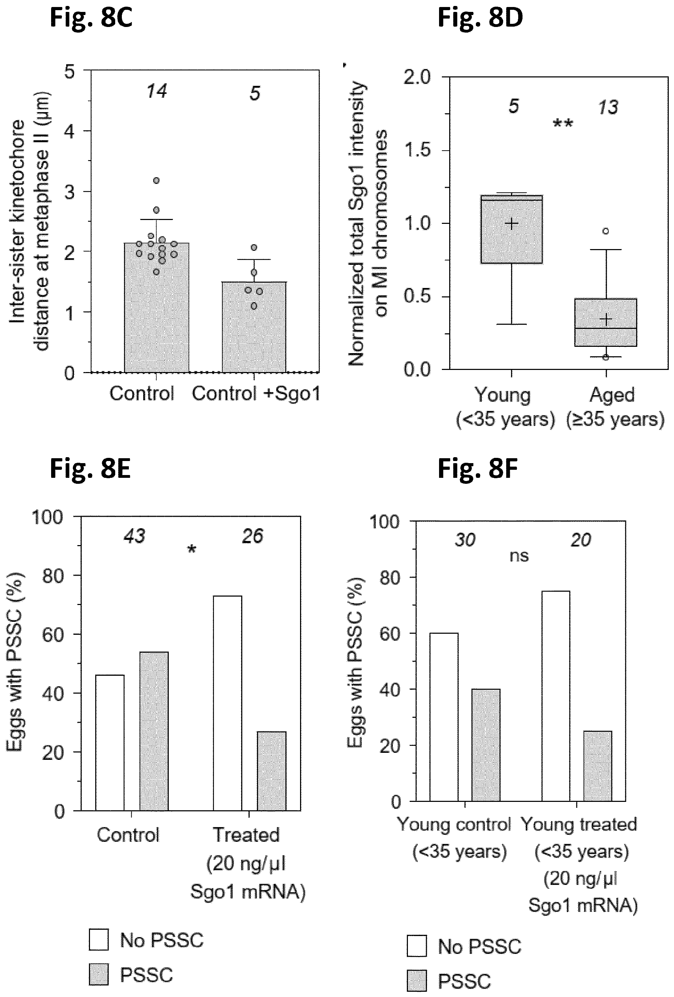

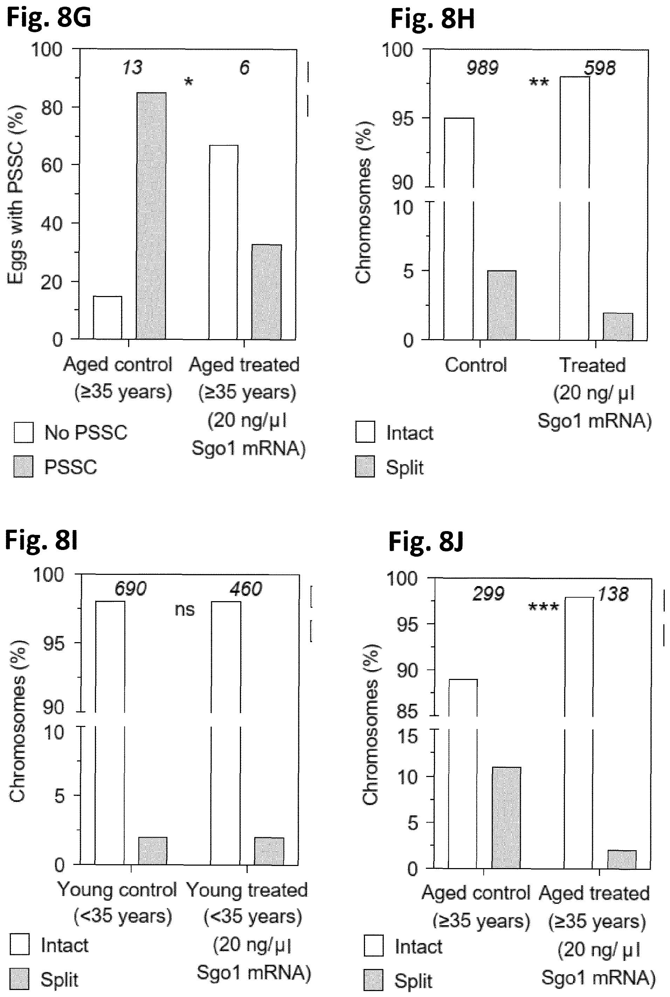

**Fig. 8A** Exogenous Sgo1 can protect sister chromatid cohesion in human eggs. Schematic of the assay for PSSC reduction in human eggs.
**Fig. 8B** Percentage of eggs with PSSC in untreated eggs and eggs supplemented with Sgo1. Plots show n = number of eggs analyzed at top.
**Fig. 8C** Measurements of inter-kinetochore distances between sister chromatids of metaphase II chromosomes in untreated eggs and eggs supplemented with Sgo1. Plots show n = number of oocytes analyzed at top, mean and standard deviation (black vertical lines), and individual values (dots).
**Fig. 8D** Normalized total intensity of chromosomal Sgo1 in metaphase I oocytes from young (<35 years) and aged (≥35 years) women. The analyzed oocytes were the same as in Figure 7A, but the age boundary was shifted in this figure. Sgo1 is significantly reduced at the pericentromere region in aged human oocytes compared to young oocytes. Plots show mean (plus), median (horizontal black line), 25th and 75th percentiles (boxes), 10th and 90th percentiles (whiskers), and the 1st and 99th percentiles (circles). P values were calculated using the Mann-Whitney test, P = 0.007.
**Fig. 8E-G** Eggs from young (<35 years) and aged (≥35 years) women were microinjected with 4 pl of Sgo1 mRNA (20 ng/µl) or kept untreated. Treated and untreated eggs were scored for PSSC. The percentage of eggs with PSSC is shown for young and aged women together (E), as well as for the same young (<35 years, F) and aged (≥35 years, G) women, separately. Plots show n = number of eggs analyzed at top. P values were calculated using Fisher's exact test on the number of eggs with or without PSSC. *p≤0.05, ns = not significant.
**Fig. 8H-J** Sgo1 mRNA (20 ng/µl) supplementation reduces the frequency of PSSC in human eggs. Percentage of chromosomes with PSSC in untreated eggs and eggs supplemented with Sgo1 mRNA (young and aged eggs together (H), in young eggs alone (<35 years, I) and aged eggs alone (≥35 years, J). Plots show n = number of chromosomes analyzed at top. P values were calculated by Fisher's exact test on the number of chromosomes being either split or intact. **p≤0.01, ***p≤0.001, ns = not significant.

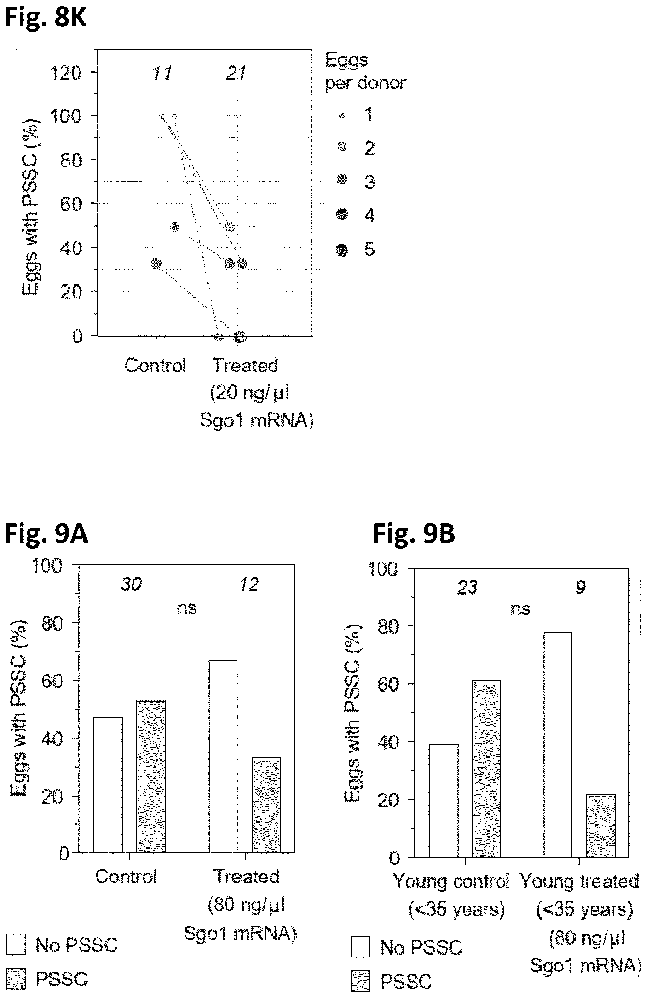

**Fig. 8K** Eggs from the same donor were microinjected with 4 pl of Sgo1 mRNA (20 ng/µl) or kept untreated. The percentage of PSSC in the treated and untreated eggs is shown for each donor (linked by a line). The size of the circle corresponds to the number of eggs that was analyzed. Plots show n = number of eggs analyzed at top, each dot represents percentage of eggs with PSSC per donor. Scale bars: 10 µm.

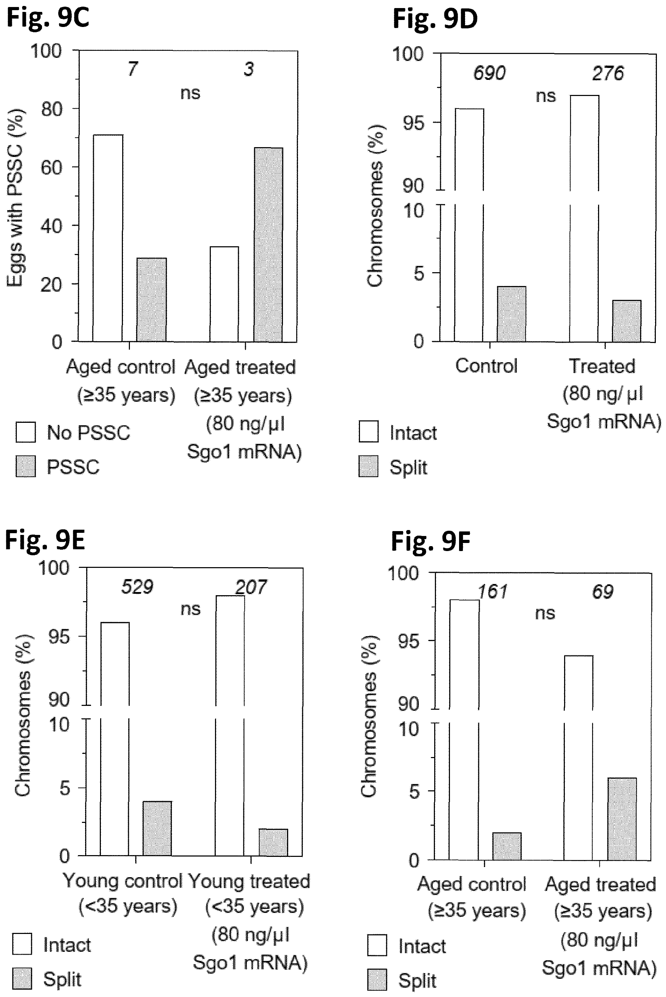

**Fig. 9A-C** Exogenous Sgo1 can protect sister chromatid cohesion in human eggs. Sgo1 expression reduces PSSC in human eggs. Eggs from young (<35 years) and aged (≥35 years) women were microinjected with 4 pl of Sgo1 mRNA (80 ng/µl) or kept untreated. Treated and untreated eggs were scored for PSSC. The percentage of eggs with PSSC is shown for young and aged women together (A), as well as for the same young (<35 years, B) and aged (≥35 years, C) women, separately. Plots show n = number of eggs analyzed at top. P values were calculated using Fisher's exact test on the number of eggs with or without PSSC. ns = not significant.
**Fig. 9D-F** Sgo1 mRNA (80 ng/µl) supplementation reduces frequency of PSSC in human eggs. Percentage of chromosomes with PSSC in untreated eggs and eggs supplemented with 80 ng/µl Sgo1 mRNA (young and aged eggs together (D), in eggs from young (<35 years, E) and aged (≥35 years, F) women. Plots show n = number of chromosomes analyzed at top. P values were calculated by Fisher's exact test on the number of chromosomes being either split or intact. ns = not significant.

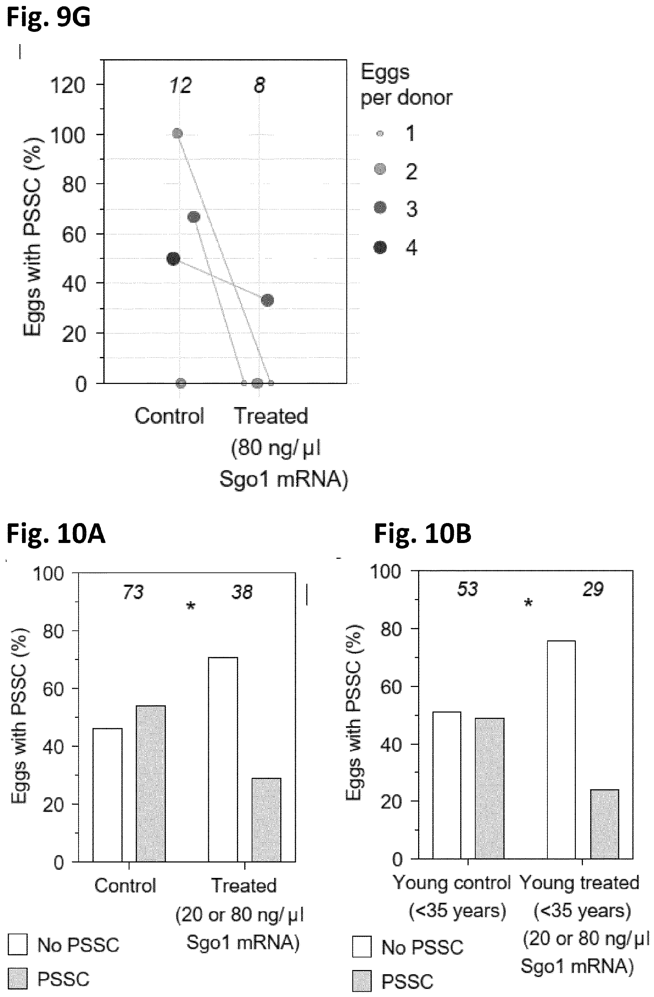

**Fig. 9G** Eggs from the same donor were microinjected with 4 pl of Sgo1 mRNA (80 ng/µl) or kept untreated. The percentage of PSSC in the treated and untreated eggs is shown for each donor (linked by a line). The size of the circle corresponds to the number of eggs that was analyzed. Plots show n = number of eggs analyzed at top, each dot represents percentage of eggs with PSSC per donor. Scale bars: 10 µm.

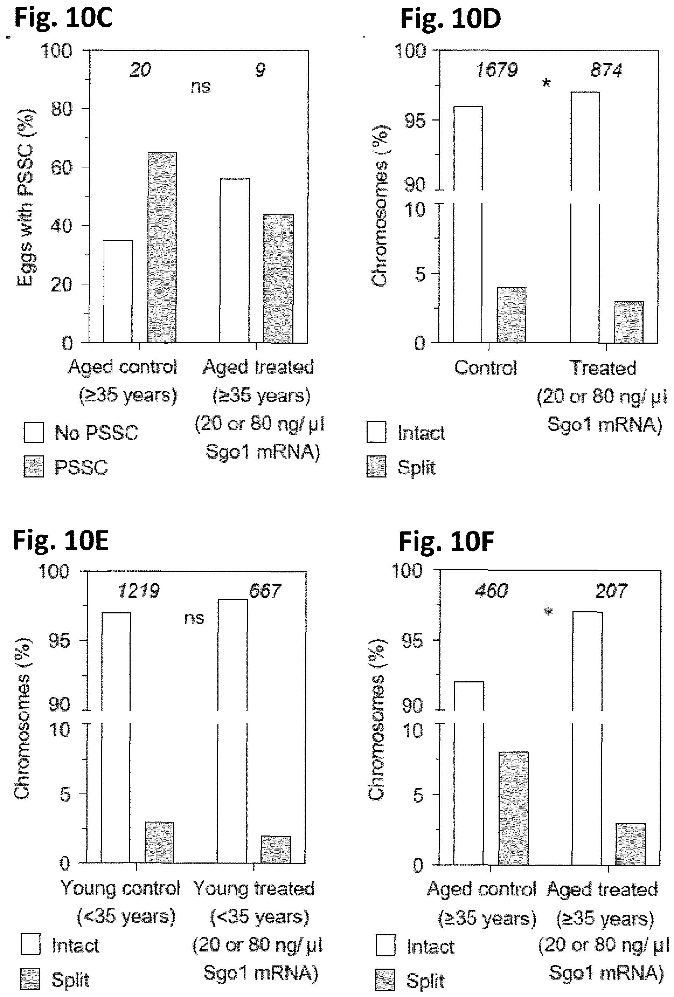

**Fig. 10A-C** Exogenous Sgo1 can protect sister chromatid cohesion in human eggs. Eggs from young (<35 years) and aged (≥35 years) women were microinjected with 4 pl of Sgo1 mRNA (20 or 80 ng/µl) or kept untreated. Treated and untreated eggs were scored for PSSC. The percentage of eggs with PSSC is shown for young and aged women together (A), as well as for the same young (<35 years, B) and aged (≥35 years, C) women, separately. Plots show n = number of eggs analyzed at top. P values were calculated using Fisher's exact test on the number of eggs with or without PSSC. *p≤0.05, ns = not significant.
**Fig. 10D-F** Sgo1 mRNA expression (20 or 80 ng/µl) reduces frequency of PSSC in human eggs. Percentage of chromosomes with PSSC in untreated eggs and eggs supplemented with 4 pl of 20 or 80 ng/µl Sgo1 mRNA (young and aged eggs together (D), in eggs from young (<35 years, E) and aged (≥35 years, F) women. Plots show n = number of chromosomes analyzed at top. P values were calculated by Fisher's exact test on the number of chromosomes being either split or intact. *p≤0.05, ns = not significant.

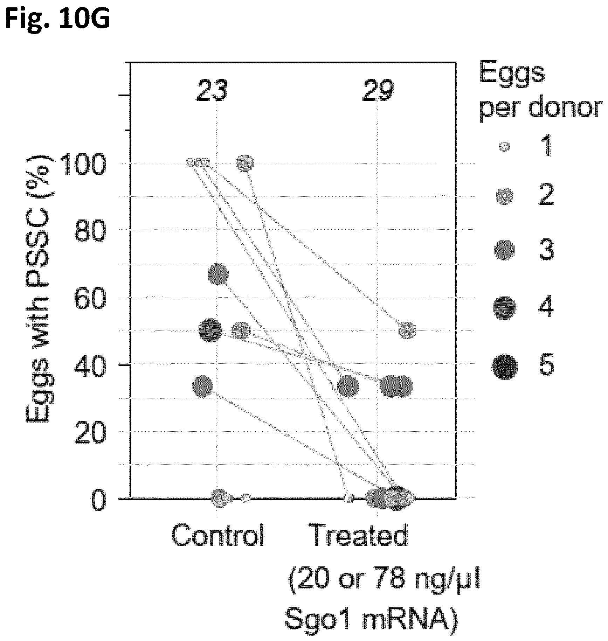

**Fig. 10G** Eggs from the same donor were microinjected with 4 pl of Sgo1 mRNA (20 or 80 ng/µl) or kept untreated. The percentage of PSSC in the treated and untreated eggs is shown for each donor (linked by a line). The size of the circle corresponds to the number of eggs that was analyzed. Plots show n = number of eggs analyzed at top, each dot represents percentage of eggs with PSSC per donor.

The asterisks in Figs. 1D, 1E, 1I, 2C, 2D, 3B, 4B, 5B, 5E indicate the calculated statistical significance, which was determined using Fisher's Exact Test based on percentages. A re-analysis was conducted using the absolute oocyte/egg numbers for Fisher's Exact Test, resulting in p-values as listed in the above description of the Figures. These p-values are used in the interpretation of results.

### Detailed description

The introduction of exogenous Sgo1, Sgo2 and/or satellite RNA into oocytes to improve centromeric cohesion and reduce aneuploidy was shown to be effective in mammalian oocytes and eggs. This introduction represents an *in vitro* method that can be used in assisted reproductive technologies to prevent chromosomal aberrations such as aneuploidy in eggs.

An *in vitro* method of introducing (i) mRNA encoding Shugoshin 1 ("Sgo1") or (ii) Sgo1 protein into an oocyte or an egg is disclosed, as further defined in the claims.

The term "introducing" used herein refers to bringing a biological molecule, such as a protein or an oligonucleotide, into a cell, e.g. an oocyte or egg. The preferred biological molecule is a protein or an mRNA. The person skilled in the art is familiar with the methods by which a biological molecule can be introduced into a cell and such methods have been described in the literature, such as using microinjection, electroporation, or transfection. A biological molecule introduced to a cell may be referred to as "exogenous" herein. The term "endogenous" refers to a biological molecule produced inside a cell, wherein the cell has not been genetically manipulated to produce the biological molecule.

mRNA encoding Sgo1 or Sgo1 protein can be introduced into an oocyte or egg, for example, by microinjection, electroporation or transfection. Preferably, the method of introduction is microinjection. In the Example 1 provided herewith, the inventors successfully introduced mRNA encoding Sgo1 protein by microinjection. After introduction, an oocyte or an egg comprises the detectable level of protein or oligonucleotide that can be detected by, for example, fluorescence or polarized microscopy, Western blot. Oligonucleotide such as mRNA can be detected by RNA FISH, RNA sequencing or Northern blot. Introduction of exogenous protein or mRNA to an oocyte with decreased endogenous levels of said protein or mRNA may restore the levels of said protein or mRNA in the oocyte. Exogenous protein or mRNA may have the same function in an oocyte as corresponding endogenous protein or mRNA.

In general, the term *"in vitro"*as used herein means outside a living organism such as a human or animal body. For example, a method may be carried out after obtaining an oocyte from, e.g., a woman and by microinjecting Sgo1 protein into the oocyte. In other words, the methods described herein are performed *ex vivo.* This means that these methods are not performed on a living subject, such as a human being.

An "oocyte" is a female gametocyte or germ cell involved in reproduction. In other words, it is an immature ovum, or immature egg, which is known in the art. The term oocyte refers to the stage of the female gamete until metaphase II of meiosis. The term "egg" or "ovum", also known as "egg cell" or "mammalian egg", refers to a mature oocyte, i.e. after the oocyte has undergone the first meiotic division and is arrested in metaphase II. The term "zygote" refers to a fertilised egg, i.e. the result of the union of a female gamete (egg or ovum) with a male gamete (sperm). The terms "oocyte", "egg" and "zygote" are known in the art (70). According to the present invention, the methods as disclosed herein can be carried out using an oocyte or an egg

It is preferred that the methods disclosed herein reduce aneuploidy. Aneuploidy is a condition wherein a cell has a wrong number of chromosomes, which is a condition known in the art. The reduction of aneuploidy is referred to the reduction of occurrence of aneuploidy in eggs or probability of occurrence of aneuploidy. Therefore, the reduction of aneuploidy means reduction of frequency or incidence of aneuploidy occurring in cells, e.g. oocytes or eggs. The person skilled in the art is aware of the methods detecting aneuploidy, such as karyotyping or microscopic imaging. Hence, the person skilled in the art is able to detect and compare aneuploidy incidence in the oocytes or eggs having exogeneous protein or mRNA encoding exogenous protein introduced with oocytes not having exogeneous protein or mRNA encoding exogenous protein introduced. In particular, the skilled person is able to compare aneuploidy incidence in the oocytes having Sgo1 protein or mRNA encoding Sgo1 protein introduced with oocytes not having Sgo1 protein or mRNA encoding Sgo1 protein introduced.

Shugoshin 1 protein, referred to herein as 'Sgo1', is the protein encoded by the Sgo1 gene in eukaryotic species and is well known to those skilled in the art. Endogenous Sgo1 protein interacts and co-localizes with the PP2A protein complex at centromeric regions of sister chromatids during mitosis and it functions in the centromeric protection of cohesin by recruiting PP2A, as was previously established in the art. PP2A is a phosphatase, i.e. it catalyzes removal of phosphate groups from proteins, the process also referred to as dephosphorylation. PP2A maintains Rec8 protein dephosphorylated during meiosis, which prevents its cleavage and premature chromatid segregation. This mechanism constitutes a plausible role by which Sgo1 is expected to contribute to protection of centromeric cohesion.

Co-localization of proteins in a cell, for example of Sgo1 and PP2A or other proteins, can be tested by methods known in the art, such as fluorescence microscopy, preferably wherein the target proteins are detected with antibodies. The primary antibody for detection is anti-Sgo1 antibody. Anti-Sgo1 antibody may be labelled with fluorophore for fluorescence detection (also referred to as immunofluorescence). Alternatively, the primary antibody may be not fluorescently labelled and a secondary fluorophore-labelled antibody against the primary antibody may be used for detection. The person skilled in the art is well aware of the methods involving antibody-based detection of a protein in a cell.

The Sgo1 protein has an N-terminal coil-coil domain and a C-terminal basic domain (60). The N-terminal domain of Sgo1 was shown to form a homodimer and bind PP2A subunits B and C. Hence the interaction of Sgo1 with PP2A can be tested by methods known in the art. Such methods may include, for example, pull-down assays, isothermal calorimetry or capillary thermophoresis. The terms 'binding' and 'interaction' are used herein interchangeably and mean association of two biological molecules, such as protein-protein interactions or protein-oligonucleotide interactions; or biological molecule and any other chemical molecule, such as protein and small molecule.

The gene encoding Sgo1 can be transcribed to mRNA encoding Sgo1 protein and such mRNA encoding Sgo1 protein can be translated into Sgo1 protein. Both the processes of transcription and translation are well defined in the art. Ideally, translated mRNA encoding Sgo1 protein or Sgo1 protein has PP2A binding activity and co-localizes with PP2A (see e.g., Examples herein).

In preferred embodiments, mRNA encoding Sgo1 protein or Sgo1 protein is introduced to an oocyte or an egg in an effective amount to reduce aneuploidy. The person skilled in the art is capable of determining an effective amount of (i) mRNA encoding Sgo1 protein or (ii) Sgo1 protein. For example, the person skilled in the art may perform a titration experiment with various amounts of (i) mRNA encoding Sgo1 protein or (ii) Sgo1 protein, which guides the skilled person. Furthermore, the titration experiment may detect aneuploidy in oocytes or eggs, which can be assessed by microscopy or polarized light microscopy or karyotyping. Such titration experiment with aneuploidy detection might compare the frequency of aneuploidy in oocytes or eggs having (i) mRNA encoding Sgo1 protein or (ii) Sgo1 protein introduced compared to oocytes or eggs without having (i) mRNA encoding Sgo1 protein or (ii) Sgo1 protein. It is clear to the skilled person that oocytes would be compared to oocytes and eggs would be compared to eggs. In contrast, oocytes are ideally not compared to eggs. In such an experiment, the skilled person may observe no or non-significant difference between these two groups, when too little (below effective amount) of (i) mRNA encoding Sgo1 protein or (ii) Sgo1 protein has been introduced. At the same time, the person skilled in the art might observe disturbances in chromosome segregation when an excessive amount of (i) mRNA encoding Sgo1 protein or (ii) Sgo1 protein has been introduced.

Exemplary effective amounts of Sgo1 protein introduced into an oocyte or an egg may be from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg.

Exemplary effective amounts of Sgo1 protein may be introduced into an oocyte at a concentration from 0.1 to 30 mg/ml, more preferably from 0.5 to 15 mg/ml, even more preferably from 1 to 10 mg/ml.

In preferred embodiments, the Sgo1 protein is recombinantly produced. Alternatively, Sgo1 may be extracted from non-human mammalian species. Recombinant Sgo1 protein may be produced by a standard procedure in the art. For example, HEK 293 cells may be used for recombinant expression of Sgo1. Alternatively, Sgo1 may be recombinantly expressed in CHO cells, insect cells, yeast cells or bacterial cells. Furthermore, the skilled person is well aware of the methods of recombinant protein purification, such as affinity chromatography (such as nickel affinity chromatography, performed, for example using HisTrap FF, GE Healthcare), followed by size exclusion chromatography (for example using ÄKTA pure equipped with HiLoad 26/600 Superdex 200 column, GE Healthcare). The person skilled in the art is aware of the recombinant protein expression protocols.

The Sgo1 protein may be human Sgo1 protein or non-human Sgo1 protein. In preferred embodiments, Sgo1 protein is a human protein. It is also preferred that the Sgo1 protein comprises, and preferably consists of, a sequence being at least 70% identical to SEQ ID NO:1, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91 %, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 1.

The mRNA encoding Sgo1 protein translates to an amino acid sequence comprising, preferably consisting of, a sequence having at least 70% identity to SEQ ID NO: 1, preferably at least 75%, more preferably at least 80%, more preferably at least 81 %, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91 %, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 1.

The person skilled in the art is well aware of the methods to compare sequence identity of proteins and oligonucleotides. The person skilled in the art may use the Basic Local Alignment Search Tool (BLAST) to compare sequence identity.

Exemplary effective amount of mRNA encoding Sgo1 protein introduced into an oocyte or an egg may be from is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 100 to 1000 fg per oocyte or egg, and most preferably and most preferably from 140 to 750 fg per oocyte or egg. Ideally, the effective amount of mRNA encoding Sgo1 introduced to the oocyte or the egg is from 1 to 4000 fg per oocyte or egg, preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 70 to 700 fg per oocyte or egg, even more preferably from 70 to 500 fg per oocyte or egg, even more preferably from 75 to 400 fg per oocyte or egg, even more preferably from 80 to 320 fg per oocyte or egg, and even more preferably 80 fg or 320 fg per oocyte or egg. Ideally, the effective amount of mRNA encoding Sgo1 protein introduced into an oocyte or an egg is 80 fg.

Exemplary effective amount of mRNA encoding Sgo1 protein may be introduced into an oocyte at a concentration from 6.5 to 650 ng/µl, more preferably from 10 to 100 ng/µl, even more preferably from 30 to 80 ng/µl, even more preferably from 20 to 80 ng/µl, and most preferably wherein the effective amount of mRNA encoding Sgo1 is introduced at the concentration of 20 ng/µl or 80 ng/µl.

In preferred embodiments, (a) mRNA encoding Sgo2 protein or (b) Sgo2 protein is additionally introduced into the oocyte. The term 'additionally introduced' means herein that (A) mRNA encoding Sgo1 protein or Sgo1 protein and (B) mRNA encoding Sgo2 protein or Sgo2 protein are introduced to an oocyte or egg simultaneously or sequentially. Preferably, (A) and (B) are introduced simultaneously. As was described above, the person skilled in the art is aware of the methods of introduction of biological molecule to the oocyte or egg. Additional introduction of (i) mRNA encoding Sgo2 protein or (ii) Sgo2 protein may further improve centromeric cohesion and reduce aneuploidy in oocytes and consequently lead to less chromosomal aberrations.

It is preferred that Sgo2 protein binds centromeres. The Sgo2 may bind centromeres directly or indirectly, through binding to other proteins at centromeres. The person skilled in the art is able to assess the binding of Sgo2 to centromeres with methods established in the art. For example, the person skilled in the art may test localization of Sgo2 at centromeres with microscopic imaging, as was described above and applicable herein.

Ideally, Sgo2 protein is recombinantly produced. Alternatively, Sgo2 may be extracted from non-human mammalian species. The recombinant Sgo2 protein may be produced by any standard procedure in the art. For example, HEK 293 cells may be used for recombinant expression of Sgo2. Alternatively, Sgo2 may be recombinantly expressed in CHO cells, insect cells, yeast cells or bacterial cells. Furthermore, the skilled person is well aware of the methods of recombinant protein purification, such as affinity chromatography (such as nickel affinity chromatography, for example using HisTrap FF, GE Healthcare), followed by size exclusion chromatography (for example using ÄKTA pure equipped with HiLoad 26/600 Superdex 200 column, GE Healthcare).

For example, the mRNA encoding Sgo2 protein or the Sgo2 protein is introduced to an oocyte or an egg in an effective amount to reduce aneuploidy. The person skilled in the art is capable of determining an effective amount of (a) mRNA encoding Sgo2 protein or (b) Sgo2 protein. For example, the person skilled in the art may perform a titration experiment with various amount of (a) mRNA encoding Sgo2 protein or (b) Sgo2 protein as was described above and applicable herein, which guides the skilled person.

Exemplary effective amount of Sgo2 protein introduced into an oocyte or an egg may be from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg.

Exemplary effective amount of Sgo2 protein may be introduced into an oocyte at a concentration from 0.1 - 30 mg/ml, more preferably 0.5-15 mg/ml, even more preferably 1-10 mg/ml.

The Sgo2 protein may be human Sgo2 protein or non-human Sgo2 protein. In preferred embodiments, Sgo2 protein is a human protein. It is also preferred that the Sgo2 protein comprises, and preferably consists of, a sequence being at least at least 70% identical to SEQ ID NO: 2, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 2.

The mRNA encoding Sgo1 protein translates to an amino acid sequence comprising, preferably consisting of, a sequence having at least 70% identity to SEQ ID NO:2, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 2.

Exemplary effective amount of mRNA encoding Sgo2 protein introduced into an oocyte or an egg may be from is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 100 to 1000 fg per oocyte or egg, and most preferably and most preferably from 140 to 750 fg per oocyte or egg. Ideally, the effective amount of mRNA encoding Sgo2 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 70 to 700 fg per oocyte or egg, even more preferably from 70 to 500 fg per oocyte or egg, even more preferably from 75 to 400 fg per oocyte or egg, even more preferably from 80 to 320 fg per oocyte or egg, and even more preferably 80 fg or 320 fg per oocyte or egg. Ideally, the effective amount of mRNA encoding Sgo2 protein introduced into an oocyte or an egg is 80 fg.

Exemplary effective amount of mRNA encoding Sgo2 protein may be introduced into an oocyte at a concentration from 6.5 to 650 ng/µl, more preferably from 10 to 100 ng/µl, even more preferably from 30 to 80 ng/µl, even more preferably from 20 to 80 ng/µl, and even more preferably wherein the effective amount of mRNA encoding Sgo2 is introduced at the concentration of 20 ng/µl or 80 ng/µl. Ideally, the effective amount of mRNA encoding Sgo2 protein is introduced into an oocyte at a concentration 20 ng/µl.

In preferred embodiments, a satellite RNA may be additionally introduced into the oocyte or egg. For example, a satellite RNA may be introduced together with (A) Sgo1 protein or mRNA encoding Sgo1 protein; and/or (B) Sgo2 protein or mRNA encoding Sgo2 protein. The term "satellite RNA" means herein an RNA that is transcribed from tandem repeated sequences of centromeres and pericentromeric regions. The skilled person is also aware of this satellite RNA (81). A satellite RNA can be an *in vitro* generated transcript from a non-coding centromeric or pericentromeric DNA region of a mammal (for example, DNA encoding such repeats SEQ ID NO: 12-14). The skilled person is aware of satellite RNA in different species. For example, in Drosophila, the 359 bp satellite III (SAT III) repeat on the X chromosome produces a long non-coding RNA (82) . The frog centromeric repeat 1 (fcr1) repeats in Xenopus and point centromeres in budding yeast, are also transcriptionally active (83; 84). In addition, tandem satellite arrays have been reported in centromeres of domestic cattle (*Bos taurus)* and horses (85; 86). In mouse, examples of satellite RNA are minor and major satellite repeats (MajSat) RNA (SEQ ID NO: 6 and 7). In human and primates, an example of satellite RNA is alpha satellite RNA (SEQ ID NO: 3). Ideally, the satellite RNA is alpha satellite RNA. Preferably, the sequences of the Sgo1 (mRNA or protein), Sgo2 (mRNA or protein) and/or the satellite RNA are sequences of one species. Exemplary sequences for human and mouse are provided in the sequence listing herein. In other words, it is preferred that the protein or mRNA sequence of Sgo1, Sgo2 and/or the satellite RNA are from the same species. It is strongly preferred that human Sgo1, human Sgo2 and/or human alpha satellite RNA are combined. For species having major and minor satellite RNAs, it is preferred that a major and/or a minor satellite RNA may be additionally introduced into the oocyte or egg. For example, a major satellite RNA such as MajSat for mouse, may be additionally introduced. Alternatively, a minor satellite RNA may be additionally introduced. It is also contemplate that a major and a minor satellite RNA is additionally introduced.

The oocyte used in the methods disclosed herein may be a mammalian oocyte or a mammalian egg. It is preferred that the oocyte is a human oocyte or a human egg. The oocyte or egg can be any mammalian oocyte or any mammalian egg, for example from a livestock mammal, more preferably wherein the livestock mammal is cattle, a horse, a goat, a rabbit, a sheep, a pig, a donkey, a llama, a camel, or an alpaca. The oocytes or eggs can be obtained by oocyte retrieval, wherein the oocyte retrieval as such is not part of the invention. The method of introducing any of Sgo1 protein, mRNA encoding Sgo1 protein, Sgo2 protein, mRNA encoding Sgo2 protein, centromeric RNA, and combinations thereof, is an *in vitro* method. In other words, said method is carried out *ex vivo.*

The oocyte or the egg may be a young or aged oocyte or egg. For example, a young oocyte or egg is an oocyte or egg that is less than 35 years. Also, in young oocytes or young eggs, the methods as disclosed herein may reduce PSSC and thus may prevent chromosomal aberrations. For example, the methods as disclosed herein may be performed on oocytes or eggs from women in their early 30s, such as oocytes or eggs being around 30, 31, 32, 33, or 34 years old.

An oocyte can be an aged oocyte. An egg may be an aged egg. The term 'aged oocyte' or 'aged egg' used herein means that the oocyte or the egg is of age higher than reproductive prime age. The reproductive prime age for human is an age from 18 to 35 years, more preferred from 18 to 29 years. The fertility decline in a woman older than 35 years has been well described in the art. Therefore, the human oocyte or egg can be an aged oocyte or an aged egg, wherein the aged oocyte or the aged egg is at least 35 years old. Preferably, the aged oocyte or the aged egg is from 35 to 50 years old, more preferably from 36 to 45 years old, more preferably from 37 to 40 years old. Ideally, the aged oocyte or the aged egg is from 35 to 45 years old. Example 3 herein demonstrates that human oocytes older than 35 years have lower Sgo1 intensity detected by fluorescence microscopy than oocytes in reproductive prime age. The age of an oocyte herein is the age of the individual from which the oocyte was retrieved at the time of the oocyte retrieval. The oocyte retrieval is not the part of any methods disclosed herein.

An oocyte can be from a human having a medical record of at least one failed *in vitro* fertilization cycle. The term 'medical record' means a medical history of an individual, summarizing all information on care or treatment received, results of such treatments or care and diagnoses. The medical record may be in written or non-written form. A medical record of an individual might be obtained from the individual's medical care provider or from the individual's oral disclosure.

*In vitro* fertilization is known by the person skilled in the art. The term *"in vitro* fertilization cycle" means that oocytes and/or eggs have been retrieved once and that at least one egg has been fertilized with a sperm. In general, women can undergo several IVF cycles. An IVF cycle is considered failed if it does not result in a live birth.

An oocyte or egg can be from a human with a medical history of being unable to conceive for a period of at least two years. The term 'being unable to conceive' refers herein to attempts to conceive that do not result in live birth.

An oocyte can be a mammalian oocyte from an endangered animal. An endangered animal is an animal which is at risk of extinction because of decrease in its population or loss of its habitat. An animal can be considered as endangered at a regional, national or international level. The person skilled in the art may consult International Union for Conservation of Nature (IUCN) Red List of Threatened Species to define whether an animal is endangered. An endangered animal may be listed at any category of IUCN Red List (e.g. Extinct, Threatened or Lower Risk). The person skilled in the art might consult with any national authority for nature conservation to define whether an animal is endangered nationally or regionally. One example of national authority for nature conservation is German Federal Agency for Nature Conservation.

Examples of endangered species are: a panda, an orangutan, a saola, a tiger, a rhinoceros, a leopard, a gorilla, an elephant, a bonobo, or a dolphin, a tapir, a zebra, a gibbon, a chimpanzee, a lemur, a macaque, a monkey, a deer, a giraffe, a whale, a lynx, a seal, a gazelle, an antelope, a possum, a wombat, a wolf, a pangolin, a sloth, and a cheetah.

In one embodiment, an oocyte or egg prior to carrying out the method may have increased probability of having a premature separation of sister chromatids (PSSC). PSSC is premature sister chromatid separation, wherein sister chromatid pairs separate from each other prior to anaphase I and segregate independently, and often incorrectly, during anaphase I. PSSC is the primary cause of aneuploidy in oocytes and eggs. In Examples 1-3, PSSC was detected based on presence of single chromatids with single kinetochores. A person skilled in the art is aware of the methods to detect single chromatids in oocytes or eggs, such as microscopy with staining for DNA and components of kinetochore and microtubules. An increase in probability of having a premature separation of sister chromatids is assumed when a higher percentage of oocytes show PSSC compared to an average oocyte or egg in reproductive prime age. In preferred embodiments, an oocyte has at least a 5% increased probability of having a premature separation of sister chromatids (PSSC), preferably at least 10%, more preferably at least 15%, more preferably at least 20%, more preferably at least 25%, and even more preferably at least 35% compared to an average oocyte in reproductive prime age.

In an embodiment, the oocyte or the egg may have a reduction in natural levels of Sgo1 protein compared to an average oocyte in reproductive prime age. Natural levels of Sgo1 refer to the amount of Sgo1 expressed in a cell which has not been genetically manipulated to express Sgo1. The term 'expression' as used herein refers to the concentration of Sgo1 (amount per volume) in a cell, the term 'to express' means to produce a protein in a cell.

Specifically, the oocyte or egg may have at least 5% reduction in natural Sgo1 levels, preferably at least 10% reduction, more preferably at least 15% reduction, even more preferably at least 25% reduction and even more preferably at least 35% reduction compared to an average oocyte in reproductive prime age.

The natural levels of Sgo1 (i.e. amount of Sgo1 in a cell) may be assessed by Western blot with quantification of band intensity. Naturally, the person skilled in the art is well-versed in the standard protocols to perform Western Blot. In this technique, proteins, having undergone separation in a gel via electrophoresis, are transferred onto membranes such as PVDF or nitrocellulose. Subsequently, these proteins are identified through their interaction with labeled antibodies. The antibody for detection of mouse Sgo1 may be mouse anti-Sgol1 HOO151648-M01 (Abnova). Of course, the skilled person is aware that primary antibodies towards Sgo1, as well as secondary antibodies, can be commercially obtained from many commercial providers. Natural levels of Sgo1 protein in an oocyte can be assessed by fluorescence microscopy or polarized light microscopy, wherein Sgo1 protein is detected with anti-Sgo1 antibody. Alternatively, the natural levels of Sgo1 in the oocyte or the egg may be determined with other methods, such as mass-spectrometry. Moreover, the person skilled in the art can assess the expression of mRNA encoding Sgo1 through various techniques such as RNA-sequencing, reverse transcription-polymerase chain reaction (RT-PCR), Northern blot, or in situ microarray hybridization. The person skilled in the art may conduct a comparison between the natural levels of Sgo1 in a specific oocyte or egg and the levels observed in an average oocyte or egg. An average oocyte or egg may be retrieved from a female individual, human or animal, in the prime reproductive age. To establish the natural level of Sgo1 in an average oocyte of reproductive prime age, the skilled person may determine Sgo1 levels across a set of oocytes in the reproductive prime age (18-35 years for humans) and subsequently calculate the average natural level of Sgo1 within this sample set.

The oocyte or the egg after having carried out the method may have reduced probability of having a premature separation of sister chromatids (PSSC) compared to an oocyte or an egg not undergoing the method. A reduced probability of having a premature separation of sister chromatids is assumed when a lower percentage of oocytes show PSSC compared to an oocyte not undergoing the method. The oocyte or the egg not undergoing the method is a comparable oocyte or egg, of approximately the same age. Reduction of probability of having a premature separation of sister chromatids (PSSC) may be determined by the skilled person as was described herein.

The oocyte or egg after having carried out the method has at least a 5% reduced probability of having a premature separation of sister chromatids (PSSC), preferably at least 10%, more preferably at least 15%, more preferably at least 20%, more preferably at least 25%, and even more preferably at least 35% compared to an oocyte or an egg not undergoing the method.

The oocyte or the egg prior to carrying out the method as described herein may have reduction in natural Sgo2 levels compared to an average oocyte or egg in reproductive prime age. The term 'natural levels' has been previously defined and is also applicable for this embodiment. Additionally, the methodologies outlined for determining the natural levels of a protein are equally applicable. Specifically, Sgo2 protein may be detected by Western blot or fluorescence or polarized light microscopy, wherein Sgo2 protein is detected with rabbit anti-Sgol2 antibody, for example with rabbit anti-Sgol2 antibody from Biorbyt (orb499806).

The oocyte or the egg prior to carrying out the method may have a reduction in natural Sgo2 levels of at least 10%, preferably at least 20%, more preferably at least 30%, and even more preferably at least 40% compared to an average oocyte or an average egg in reproductive prime age.

The oocyte or the egg prior to carrying out the method may have a reduction in PP2A levels compared to an average oocyte in reproductive prime age. The methodologies outlined for determining the levels of a protein are equally applicable here. Specifically, PP2A may be detected by using rabbit anti-PP2A catalytic subunit antibody number 05-421 from Merck or with rabbit anti-PP2A catalytic subunit antibody number 2038S from Cell Signaling.

The oocyte or the egg prior to carrying out the method may have at least 10% reduction in PP2A levels, preferably at least 20% reduction, more preferably at least 35% reduction and even more preferably at least 50% reduction compared to an average oocyte or average egg cell in reproductive prime age.

In preferred embodiments, the oocyte or the egg prior to carrying out the method may have a reduction in Rec8 levels at centromeres. As for other proteins discussed above, the same methods may be applied to determine the Rec8 levels. Specifically, Rec8 may be detected with rabbit anti-Rec8 antibody from Cambridge Research Biochemicals.

The oocyte or the egg prior to carrying out the method may have at least 15% reduction in Rec8 levels at centromeres, preferably at least 25% reduction, more preferably at least 35% reduction and even more preferably at least 55% reduction compared to an average oocyte or an average egg in a reproductive prime age.

The oocyte or the egg prior to carrying out the method may have reduced transcription during meiotic divisions. Transcription is a well-defined biological process of RNA synthesis from a DNA template. Reduced transcription or reduced transcription level is a decrease in coding and non-coding RNA synthesis in a cell. The person skilled in the art may determine the level of transcription by using fluorescence or polarized light microscopy with anti-RNA Pol II Sp2 antibody detection, which detects elongating (hence, transcribing) RNA Pol II. Alternatively, centromeric transcription may be assessed by detecting centromeric RNA transcripts by RNA fluorescence in situ hybridization (RNA FISH). The skilled person is well aware of the current protocols on performing RNA FISH.

The oocyte or the egg prior to carrying out the method may have reduced transcription during meiotic divisions, in particular centromeric transcription, by at least 10%, preferably 20%, more preferably at least 30% compared to an average oocyte or an average egg in reproductive prime age.

In an embodiment, the oocyte or the egg prior to carrying out the method may have an increased interkinetochore distance. Interkinetochore distance as defined herein is a distance between sister kinetochores. Increased distance between sister kinetochores is associated with loss of cohesion and aneuploidy. In Example 2, the inventors measured the interkinetochore distance using fluorescence microscopy with antibody detection of kinetochores, wherein interkinetochore distances were obtained by manual pairwise measurements between detected spots of kinetochores (see also exemplary Figs. 4D, 5D and 5G). In Example 3, the inventors introduced Sgo1 into human oocytes, which resulted in decrease of interkinetochore distance in MII stage, likely due to increased cohesion in the centromeric region (Fig. 8C).

An increased interkinetochore distance compared to an average oocyte or an average egg in reproductive prime age therefore may be a characteristic of the oocyte or the egg. Increased interkinetochore distance is typical for an aged oocyte and/or oocytes with loss of sister chromatid cohesion. Similarly, increased interkinetochore distance is typical for aged eggs and/or eggs with loss of sister chromatid cohesion.

The oocyte or egg prior to carrying out the method may have an increased interkinetochore distance by at least 10% when compared to an average oocyte or an average egg in reproductive prime age, more preferably by at least 20%, and the most preferably by at least 30% when compared to an average oocyte or an average egg in reproductive prime age.

The oocyte or the egg may be compared to an oocyte or an egg in a reproductive prime age. The reproductive prime age for a human oocyte may be from 18 to 29 years, more preferably from 20 to 27 years, even more preferably from 23 to 25 years.

In preferred embodiments, the mRNA encoding Sgo1 protein or the Sgo1 protein, preferably in combination with the (i) mRNA encoding Sgo2 protein or the Sgo2 protein and/or (ii) the satellite RNA may be introduced to the oocyte during various stages. In particular, the mRNA encoding Sgo1 protein or Sgo1 protein, preferably in combination with the (i) mRNA encoding Sgo2 protein or Sgo2 protein and/or (ii) the satellite RNA may be introduced to the oocyte or the egg during prophase arrest, meiosis I, or meiosis II. As known in the art, meiosis I (MI) and meiosis II (MII) can be subdivided into different stages. It is also known in the art that prophase arrest includes germinal vesicle stage.

In oogenesis, oocytes have a prolonged resting phase called dictyate, in which oocytes are arrested in prophase I. During this stage, an immature nucleus of an oocyte is referred to as germinal vesicle. Upon maturation stimuli, the oocyte resumes meiosis, which requires breakdown of the germinal vesicle (GVBD). Therefore, the stage at which an oocyte is arrested prior to resuming meiosis is referred to as germinal vesicle stage.

The person skilled in the art is able to determine the GV stage of oocytes by morphological features using light microscopy. In preferred embodiments, the mRNA encoding Sgo1 or the Sgo1 protein, preferably in combination with the (i) mRNA encoding Sgo2 protein or Sgo2 protein and/or (ii) the satellite RNA may be introduced to the oocyte during germinal vesicle stage.

After germinal vesicle stage (also known as dictyate-arrested prophase I), maturation of an oocyte continues with germinal vesicle breakdown and meiotic spindle formation, metaphase I, anaphase I, and telophase I.

It is preferred that the mRNA encoding Sgo1 protein or Sgo1 protein, preferably in combination with the (i) mRNA encoding Sgo2 protein or Sgo2 protein and/or (ii) the satellite RNA may be introduced to the oocyte or the egg during the germinal vesicle stage, meiosis I prometaphase, meiosis I metaphase, meiosis I anaphase, meiosis I telophase, or meiosis II metaphase. Ideally, the mRNA encoding Sgo1 protein or Sgo1 protein, preferably in combination with the (i) mRNA encoding Sgo2 protein or Sgo2 protein and/or (ii) the satellite RNA is introduced to the oocyte or the egg between the germinal vesicle stage and anaphase I. It is most preferred that the mRNA encoding Sgo1 protein or Sgo1 protein, preferably in combination with the (i) mRNA encoding Sgo2 protein or Sgo2 protein and/or (ii) the satellite RNA is introduced during germinal vesicle stage, meiosis I prometaphase or meiosis I metaphase.

The mRNA encoding Sgo1 protein or Sgo1 protein, preferably in combination with the (i) mRNA encoding Sgo2 protein or Sgo2 protein and/or (ii) the satellite RNA may be introduced to the oocyte during meiosis I prometaphase, meiosis I metaphase, meiosis I anaphase, meiosis I telophase, preferably during germinal vesicle stage, meiosis I anaphase or meiosis I telophase. The mRNA encoding Sgo1 protein or Sgo1 protein, preferably in combination with the (i) mRNA encoding Sgo2 protein or Sgo2 protein and/or (ii) the satellite RNA may be introduced during meiosis I anaphase or meiosis I telophase. Preferably, the mRNA encoding Sgo1 protein or the Sgo1 protein, ideally in combination with the (i) mRNA encoding Sgo2 protein or Sgo2 protein and/or (ii) the satellite RNA may be introduced in metaphase I, germinal vesicle stage or during meiosis II (MII). Ideally, the mRNA encoding Sgo1 protein or the Sgo1 protein, ideally in combination with the (i) mRNA encoding Sgo2 protein or Sgo2 protein and/or (ii) the satellite RNA may be introduced in metaphase I or germinal vesicle stage. It is strongly preferred that the mRNA encoding Sgo1 protein or the Sgo1 protein, ideally in combination with the (i) mRNA encoding Sgo2 protein or Sgo2 protein and/or (ii) the satellite RNA may be introduced in metaphase I.

The skilled person has solid understanding of these cell cycle stages from common general knowledge. Moreover, the skilled person is also able to differentiate between these stages. The mRNA encoding Sgo1 or the Sgo1 protein, preferably in combination with the (i) mRNA encoding Sgo2 protein or Sgo2 protein and/or (ii) the satellite RNA may be introduced into an egg, i.e. into a mature oocyte during metaphase II arrest.

In preferred embodiments, the introduction of mRNA encoding Sgo1 protein or the Sgo1 protein, preferably in combination with the (i) mRNA encoding Sgo2 protein or Sgo2 protein and/or (ii) the satellite RNA may be carried out by microinjection. The mRNA encoding Sgo1 protein or the Sgo1 protein, preferably in combination with the (i) mRNA encoding Sgo2 protein or Sgo2 protein and/or (ii) the satellite RNA, such as an alpha satellite RNA, may be introduced during intracytoplasmic sperm injection (ICSI), preferably in a medium suitable for ICSI. The skilled person is aware of the standard protocols for microinjection into oocytes or eggs and ICSI. Furthermore, microinjection has been used by the inventors and described in Materials and Methods herein. Alternatively, said introduction may be carried out by electroporation, according to protocols known in the art or said introduction may be carried out by transfection as was described in the art. Additionally, introduction may be carried out using microfluidics device, wherein the use of microfluidics device is combined with any known method of introduction, such as electroporation, microinjection, or transfection. Furthermore, the introduction may be carried out using a lipid nanoparticle. The most preferred way of introduction is microinjection.

Ideally, none of the embodiments disclosed herein include nuclear transfer, such as 'somatic cell nuclear transfer', also known as SCNT. In 'nuclear transfer', an enucleated oocyte or egg is generated and a donor nucleus from another cell, e.g. a somatic cell, is introduced into the oocyte or egg cell. It is preferred that in none of the embodiments herein, an oocyte or an egg cell is enucleated. Additionally, it is preferred that in none of the embodiments herein, an enucleated oocyte or an enucleated egg cell is used in a method disclosed herein. In other words, it is preferred that the oocyte or the egg cell according to the embodiments has not been enucleated.

It is preferred that all methods disclosed herein are used in assisted reproductive technologies. The term 'assisted reproductive technologies' refers to procedures related to the conception, such as in vitro fertilization. Assisted reproductive technology is often used in treatment of infertility, miscarriage, implantation failure, or embryonic arrest. The methods disclosed herein may be used *in vitro* as a part of any suitable assisted reproductive technology. Assisted reproductive technology does not include a somatic cell nuclear transfer. For example, any methods disclosed herein may be carried out on an oocyte or an egg *in vitro* as a part of an *in vitro* fertilization protocol. The person skilled in the art is familiar with methods of assisted reproductive technologies. Specifically, the skilled person knows how to carry out an *in vitro* fertilization.

An *in vitro* method of introducing (a) mRNA encoding Sgo2 protein or (b) Sgo2 protein into an oocyte or an egg is also disclosed. The mRNA encoding Sgo2 protein or the Sgo2 protein is defined herein and preferred embodiments are disclosed above and said embodiments apply equally to this aspect of the invention. Furthermore, the oocyte or the egg is defined herein and preferred embodiments are disclosed above and said embodiments apply equally to this aspect of the invention. Moreover, a satellite RNA may be additionally introduced into the oocyte or the egg. The satellite RNA is defined herein and preferred embodiments are disclosed above and said embodiments apply equally to this aspect of the invention. Ideally, the mRNA encoding Sgo2 protein or the Sgo2 protein, preferably in combination with the satellite RNA, is/are introduced during prophase arrest, meiosis I, or meiosis II, in particular during the germinal vesicle stage, meiosis I prometaphase, meiosis I metaphase, meiosis I anaphase, meiosis I telophase, meiosis II metaphase, even more preferably between the germinal vesicle stage and anaphase I, and most preferably during germinal vesicle stage, meiosis I prometaphase or meiosis I metaphase. Experimental evidence for the technical effect on the reduction of PSSC after introduction of Sgo2 alone is provided in Fig. 5B (see also Example 2).

Furthermore, (i) the mRNA encoding Sgo1 or (ii) the Sgo1 protein for use in preventing infertility is also disclosed. Ideally, said use of (i) the mRNA encoding Sgo1 protein or (ii) Sgo1 protein improves sister chromatid cohesion. Moreover, said use reduces PSSC in an oocyte, an egg, or a zygote. Ideally, said use of (i) the mRNA encoding Sgo1 protein or (ii) the Sgo1 protein may reduce aneuploidy in the oocyte, egg or zygote. Also, the use of (i) the mRNA encoding Sgo1 or (ii) the Sgo1 protein may reduce risk of miscarriage after *in vitro fertilization.* Additionally, the use of (i) the mRNA encoding Sgo1 protein or (ii) the Sgo1 protein may reduce risk of infertility, hence preventing infertility. The term *'infertility'* as referred herein and as defined by World Health Organization is failure to achieve a pregnancy after 12 months or more of regular unprotected sexual intercourse.

Additionally, the use of (i) the mRNA encoding Sgo1 protein or (ii) the Sgo1 protein may reduce risk of miscarriage, hence preventing a miscarriage. As known in the art, a "miscarriage" is the death and expulsion of an embryo or fetus before it can survive independently before 20 weeks of gestation. A key cause of miscarriage are chromosomal aberrations such as aneuploidy.

Furthermore, the use of (i) the mRNA encoding Sgo1 protein or (ii) the Sgo1 protein may reduce risk of implantation failure, hence preventing an implantation failure. As known in the art, an "implantation failure" means that the after fertilization of the egg, the zygote/early embryo does not implant into the uterus and does not continue its development. As known in the art, implantation failure is diagnosed around ten days after the transfer by measuring the hormone levels of β-hCG and/or by the absence of a gestational sac developing within the uterine cavity by ultrasound. Implantation failure occurs when the β-hCG assay is negative or when the ultrasound examination does not reveal the presence of an embryo in the endometrium. A key cause of implantation failure are chromosomal aberrations such as aneuploidy.

Moreover, the use of (i) the mRNA encoding Sgo1 protein or (ii) the Sgo1 protein may reduce the risk of delays in embryonic development and embryonic arrest, hence preventing embryonic arrest. As known in the art, "embryonic arrest" is referred to a halt or severe delay in embryonic development. Arrested or delayed embryos have a poor developmental capacity and are therefore unlikely to give rise to successful pregnancies in assisted reproductive technologies. A key cause of embryonic arrest and embryonic developmental delays are chromosomal aberrations such as aneuploidy.

The (i) mRNA encoding Sgo1 protein or (ii) the Sgo1 protein may be used in preventing infertility, miscarriage, implantation failure, or embryonic arrest; preferably wherein the infertility, miscarriage, implantation failure, or embryonic arrest is caused by a chromosomal aberration, more preferably wherein the chromosomal aberration is aneuploidy. The (i) mRNA encoding Sgo1 protein or (ii) the Sgo1 protein may be used in preventing infertility, miscarriage, implantation failure, or embryonic arrest; preferably wherein the infertility, miscarriage, implantation failure, or embryonic arrest is associated with a chromosomal aberration, more preferably wherein the chromosomal aberration is aneuploidy. In particular, the (i) mRNA encoding Sgo1 protein or (ii) the Sgo1 protein may be used in preventing chromosomal aberration associated infertility, chromosomal aberration associated miscarriage, chromosomal aberration associated implantation failure, and/or chromosomal aberration associated embryonic arrest.

The (i) mRNA encoding Sgo1 protein or (ii) the Sgo1 protein for use in a method of preventing chromosomal aberrations is also disclosed herein. Furthermore, the (i) mRNA encoding Sgo1 protein or (ii) the Sgo1 protein for use in a method of preventing a chromosomal aberration associated disease is disclosed herein. Furthermore, the (i) mRNA encoding Sgo1 protein or (ii) the Sgo1 protein for use in a method of preventing a chromosomal aberration associated disorder is disclosed herein. In other words, a method for preventing chromosomal aberration or chromosomal aberration associated disease or disorder by introducing (i) the mRNA encoding Sgo1 protein or (ii) the Sgo1 protein is also disclosed. The term 'chromosomal aberrations' refers to a missing, extra or irregular portion of chromosomal DNA. Generally, chromosomal aberrations lead to a chromosomal aberration associated disease or disorder. One example of a chromosomal aberration is aneuploidy, wherein a cell contains an abnormal number of chromosomes. Aneuploidy may be monosomy, trisomy, tetrasomy or pentasomy. For example, (i) mRNA encoding Sgo1 protein or (ii) Sgo1 protein for use in a method of preventing aneuploidy is disclosed herein. Preferably, the (i) mRNA encoding Sgo1 protein or (ii) the Sgo1 protein for use in a method of preventing trisomy or monosomy is disclosed herein. A trisomy may be trisomy 21 (Down syndrome), trisomy 13 (Edwards syndrome), trisomy 18 (Patau syndrome), trisomy 7, trisomy 9, trisomy 11, trisomy 15, trisomy 16, trisomy 17, trisomy 19, trisomy 20, trisomy 22, trisomies of all other autosomes, trisomies of sex chromosomes. A monosomy may be Turner syndrome (X chromosome monosomy). Also, aneuploidy combinations of trisomies, monosomies or other types of aneuploidies where multiple chromosomes are simultaneously present in incorrect numbers are frequently observed in early embryos and most commonly result from chromosome segregation errors during the meiotic divisions of the egg. Typically, autosome monosomies do not result in live birth and lead to miscarriage. The skilled person is able to detect chromosomal aberrations, in particular, aneuploidy, using the methods established in the art. For example, aneuploidy may be detected using karyotyping, fluorescent in situ hybridization (FISH), DNA sequencing, and quantitative PCR. Detection of chromosomal aberrations may be performed prenatally. For example, the material for prenatal detection of chromosomal aberrations may be obtained through amniocentesis. Alternatively, chromosomal aberrations may be detected using analysis of maternal serum cell-free fetal DNA.

In preferred embodiments, the (I) the mRNA encoding Sgo1 protein or the Sgo1 protein is introduced in combination with (II) the mRNA encoding Sgo2 protein or the Sgo2 protein. The (I) mRNA encoding Sgo1 protein or the Sgo1 protein; and (II) the mRNA encoding Sgo2 protein or the Sgo2 protein may be introduced together or sequentially, using the methods of introduction described above, such as microinjection, electroporation or transfection. In the Examples, the inventors introduced the mRNA encoding Sgo1 protein in combination with mRNA encoding Sgo2 protein to an oocyte using microinjection, as described in the Method section.

The (i) mRNA encoding Sgo1 protein or (ii) the Sgo1 protein may be introduced in combination with satellite RNA. The satellite RNA may be alpha satellite RNA such as the sequence of SEQ ID NO: 3.

The (i) mRNA encoding Sgo2 protein or (ii) the Sgo2 protein may be for use in preventing infertility, miscarriage, implantation failure, or embryonic arrest; preferably wherein the infertility, miscarriage, implantation failure, or embryonic arrest is caused by a chromosomal aberration, more preferably wherein the chromosomal aberration is aneuploidy. The (i) mRNA encoding Sgo2 protein or (ii) the Sgo2 protein may be for use in preventing infertility, miscarriage, implantation failure, or embryonic arrest; preferably wherein the infertility, miscarriage, implantation failure, or embryonic arrest is associated with a chromosomal aberration, more preferably wherein the chromosomal aberration is aneuploidy. In particular, the (i) mRNA encoding Sgo2 protein or (ii) the Sgo2 protein may be for use in preventing chromosomal aberration associated infertility, chromosomal aberration associated miscarriage, chromosomal aberration associated implantation failure, and/or chromosomal aberration associated embryonic arrest. The aspects of preventing infertility, miscarriage, implantation failure, or embryonic arrest are detailed above and equally apply to this part of the invention. Ideally, said use reduces PSSC in the oocyte, the egg, or the zygote. Ideally, said use of (i) the mRNA encoding Sgo2 protein or (ii) the Sgo2 protein reduces aneuploidy in the oocyte, egg or zygote.

The (i) mRNA encoding Sgo2 protein or (ii) Sgo2 protein may be for use in a method of preventing chromosomal aberrations. The (i) mRNA encoding Sgo2 protein or (ii) Sgo2 protein may be used in a method of preventing a chromosomal aberration associated disease or disorder. The chromosomal aberration may be an aneuploidy, more preferably a trisomy or a monosomy. Exemplary trisomies are trisomy 21 (Down syndrome), trisomy 13 (Edwards syndrome) or trisomy 18 (Patau syndrome). An exemplary monosomy is Turner syndrome (X chromosome monosomy). The methods by which the skilled person can detect an aneuploidy are outlined above, which also applies to this embodiment.

Disclosed herein is a pharmaceutical composition comprising an mRNA encoding Sgo1 or the Sgo1 protein. Ideally, said pharmaceutical composition additionally comprises (I) an mRNA encoding Sgo2 protein or a Sgo2 protein, (II) an mRNA encoding PP2A or PP2A protein and/or (III) a satellite RNA, and wherein the pharmaceutical composition is suitable for introduction into an oocyte, an egg or a zygote. The Sgo1 protein and the Sgo2 protein may form a complex. The Sgo1 protein and the PP2A protein may form a complex. Ideally, the Sgo1 protein and the satellite RNA may form a complex. It is also preferred that the Sgo2 protein and the satellite RNA form a complex. It is preferred that any such complex is formed after introduction into the oocyte or the egg. This is for example the case when the mRNA encoding Sgo1 and/or Sgo2 and the mRNA encoding PP2A is introduced, or when the mRNA encoding Sgo1 and/or Sgo2 and the satellite RNA are introduced. It is also contemplated that the Sgo1 protein and/or Sgo2 and the PP2A protein form a complex in the pharmaceutical composition. It is further contemplated that the Sgo1 protein and/or Sgo2 protein and the satellite RNA form a complex in the pharmaceutical composition. Ideally, the Sgo1 and/or Sgo2, the PP2A and the satellite RNA are forming a complex. It is even more preferred that the Sgo1 and/or Sgo2, the PP2A and the satellite RNA form a complex after introduction into the oocyte or the egg.

The person skilled in the art is capable to assess the complex formation between Sgo1, Sgo2, PP2A and/or satellite RNA, such as an alpha satellite RNA or a major satellite RNA, by the methods established in the art. For example, the skilled person can carry out isothermal titration calorimetry, capillary electrophoresis with fluorescently-labelled alpha satellite RNA, fluorescence polarization assay with labelled alpha satellite RNA.

The pharmaceutical composition as described above may include satellite RNA as defined herein. In a preferred embodiment, the satellite RNA is a human alpha-satellite RNA (such as SEQ ID NO: 3). A satellite RNA may be synthesized or in vitro transcribed from DNA of centromeric or pericentromeric chromosomal regions.

The pharmaceutical composition may be in an effective amount to reduce aneuploidy. The person skilled in the art can determine an effective amount and outlined above. Exemplary effective amounts of the pharmaceutical composition to be administered to an oocyte are from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg.

The pharmaceutical composition comprising the mRNA encoding Sgo1 protein or the Sgo1 protein, wherein the pharmaceutical composition additionally comprises (I) an mRNA encoding Sgo2 protein or a Sgo2 protein, (II) an mRNA encoding PP2A or PP2A protein and/or (III) a satellite RNA may be in an effective amount to be introduced at a concentration from 0.1 - 30 mg/ml, more preferably 0.5-15 mg/ml, even more preferably 1-10 mg/ml.

Ideally, the pharmaceutical composition comprises the mRNA encoding Sgo1 or the Sgo1 protein, wherein the pharmaceutical composition additionally comprises the mRNA encoding Sgo2 protein or the Sgo2 protein. In such a case the satellite RNA may be absent from the pharmaceutical composition. It is preferred that the pharmaceutical composition comprises an mRNA encoding Sgo1 or the Sgo1 protein, wherein the pharmaceutical composition additionally comprises (I) an mRNA encoding Sgo2 protein or a Sgo2 protein, and (II) a satellite RNA. The mRNA encoding Sgo2, the Sgo2 protein and the satellite RNA have been described in detail herein and all embodiments regarding said molecules apply equally to this aspect of the invention.

A needle, capillary or injection pen for microinjection comprising the pharmaceutical composition are disclosed herein. The composition may be in a form of solution or lyophilized. The composition may be encapsulated in a nanoparticle, such as lipid nanoparticle.

Finally, a kit a comprising a pharmaceutical composition as defined in the embodiments is provided. The kit may comprise the pharmaceutical composition in a form of solution or lyophilized material, such as a powder. The kit may comprise a needle, a capillary or an injection pen for microinjection comprising the pharmaceutical composition as defined herein. Additionally, the kit comprises instructions for use.

The technical advantage of the invention is corroborated by the extensive experimental evidence presented in the Example section. First, the inventors investigated the role of Sgo1 in maintaining sister chromatid cohesion in oocyte meiosis. The inventors depleted Sgo1 from mouse oocytes by injecting siRNAs against Sgo1 and examined the oocytes using microscopic imaging with immunofluorescent staining for Sgo1, kinetochores, microtubules and DNA. The inventors observed a significant increase in PSSC in Sgo1-depleted oocytes compared to untreated oocytes (Fig. 1C -1E). This finding led to the conclusion that Sgo1 protein protects sister chromatid cohesion in mouse oocytes.

The inventors subsequently investigated whether centromeric transcription affects sister chromatid cohesion. The inventors treated mouse oocytes with the transcription inhibitors triptolide and alpha-amanitin and examined the oocytes using microscopic imaging. Treatment with the transcriptional inhibitor, such as triptolide, resulted in significantly reduced Sgo1 levels in oocytes compared to control oocytes treated with DMSO. Levels of PP2A, an interactor of Sgo1, were also significantly reduced. The inventors observed an increase in chromosome oscillation, PSSC and single chromatids during metaphase II in triptolide-treated eggs (Fig. 2C,2D).

Triptolide-treated oocytes also had reduced intensity of RNA Pol II-pS2 (transcribing RNA polymerase II) and MajSat RNA, a non-coding RNA that is transcribed from the pericentromeric region of chromatids in metaphase I. When the inventors compared young (8-12 weeks) and aged (60-65 weeks) mouse oocyte, the inventors found that RNA Pol II Sp2 intensity and MajSat RNA intensity were reduced in aged mouse oocytes when compared to young oocytes (Fig. 2H - 2J). These findings led the inventors to conclude that centromeric transcription regulates sister chromatid cohesion during meiosis in oocytes and that reduction of centromeric transcription contributes to PSSC.

The inventors then introduced MajSat RNA into triptolide-treated oocytes and observed that introduction of MajSat RNA into triptolide-treated oocytes reduced the percentage of oocytes with PSSC when compared to triptolide-treated oocytes (Fig 3B). Accordingly, the introduced exogenous satellite repeats, e.g. major satellite RNA such as MajSat RNA, localized to centromeric regions and restored the intensity of MajSat RNA at the pericentromere in triptolide-treated oocytes to the levels of control DMSO-treated oocytes (Fig. 3C). Introduction of MajSat RNA into triptolide-treated oocytes resulted in a significant increase in Sgo1 and PP2A intensity at the pericentromere compared to triptolide-treated oocytes (Fig. 3D, 3E). Thus, the inventors showed that introduction of exogenous centromeric transcripts into oocytes can rescue decreased levels of Sgo1 and PP2A at the centromere.

The inventors then compared young and aged mouse eggs. PSSC, the number of single chromatids per oocyte and the inter-kinetochore distance were significantly increased during metaphase II in aged eggs when compared to young eggs (Fig. 4B-4D). The inventors then quantified the intensity of Rec8, a cohesin subunit, at the pericentromeres of young and aged oocytes and found that Rec8 was also significantly reduced in aged oocytes compared to young oocytes (Fig. 4E). Similarly, aged oocytes had reduced intensity of PP2A, Sgo1 and Sgo2 compared to young oocytes (Fig. 4F-4H).

As corroborated in the Examples, aged eggs with introduced Sgo1, Sgo2, Sgo1 + Sgo2 and Sgo1 + MatSat RNA showed significantly reduced incidence of single chromatids (percentage of oocytes with single chromatids), single chromatid number per egg, and inter-sister kinetochore distance in metaphase II compared to aged eggs without exogenous introduction (Fig. 5E - 5G). As an additional control, the inventors also tested mRNA encoding Sgo1 protein with the N61I mutation, which abolishes Sgo1 interaction with PP2A. To the knowledge of the inventors, this is the first time Sgo1 alone, Sgo2 alone, or Sgo1 in combination with Sgo2 and/or satellite repeat RNA has been introduced into aged oocytes or aged eggs in order to decrease PSSC and thereby aneuploidy. As known in the art, single chromatids separated prematurely cause chromosome segregation errors during both meiosis I (MI) and meiosis II (MII), leading to aneuploidy. Thereby, the inventors have experimentally demonstrated that the introduction of Sgo1, Sgo2 and MajSat RNA reduces the risk of aneuploidy in aged oocytes and eggs. Hence, the current invention is concerned with reducing the risk of aneuploidy in oocytes or eggs by introducing exogeneous (I) mRNA encoding Sgo1 protein or Sgo1 protein, and/or (II) mRNA encoding Sgo2 protein or Sgo2, (III) and/or satellite RNA into oocytes or eggs.

Next, the inventors looked at the effect of introducing Sgo1, Sgo2 and satellite RNA, e.g. MajSat RNA, and detected the levels of Rec8 and PP2A in aged oocytes. Surprisingly, introduction of exogenous Sgo1 to aged oocytes significantly increased the intensity of PP2A and Rec8 at pericentromeres in metaphase I (Fig. 6A, 6B). Introduction of both Sgo1 and Sgo2 significantly increased the intensity of PP2A and Rec8 at centromere in metaphase II (Fig. 6E, 6D). Hence, introduction of Sgo1 and Sgo2 improves sister chromatid cohesion.

Importantly, the reduction in Sgo1 intensity in metaphase I was detected in aged human oocytes (>35 years old) compared to young human oocytes (≤35 years old) (Fig. 7A). This agreement between the data obtained in mouse and human oocytes shows that the loss of Sgo1 with age is a phenomenon conserved in mammalian oocytes (Fig. 7C). Additionally, this data shows the risk of aneuploidy in oocytes or eggs is reduced by introducing exogeneous (I) mRNA encoding Sgo1 protein or Sgo1 protein, (II) mRNA encoding Sgo2 protein or Sgo2 protein, (III) and/or centromeric RNA into human oocytes or human eggs. In addition, exogenous mRNA encoding PP2A protein or PP2A protein may be introduced.

Thus, the inventors described the mechanisms of cohesion protection involving Sgo1, Sgo2 and centromeric RNA that are universal to mammalian oocytes. The inventors also showed that the levels of Sgo1, Sgo2, and pericentromeric transcription decrease with age, leading to PSSC, higher incidence of single chromatids, higher number of single chromatids per egg and increased inter-kinetochore distances in eggs, and therefore, increasing the risk of aneuploidy.

When mRNA encoding Sgo1 was microinjected into human oocytes, as shown in the Examples, the Sgo1 protein that was translated from the exogenous mRNAs localized to the human chromosomes at metaphase I, and metaphase II, similar to the localization observed with immunofluorescence images of endogenous Sgo1.

When Sgo1 encoding mRNA was introduced into human GV or MI stage oocytes (Fig. 8A), and oocytes were in vitro matured to the MII stage, the percentage of eggs with PSSC was prominently reduced (Fig. 8B, see also Example 3 herein). In addition, the distance between sister chromatid kinetochores in MII was decreased (Fig. 8C, see also Example 3 herein). A decrease in inter sister kinetochore distances is indicative of increased cohesion in the centromeric region. Hence, the present invention provides experimental evidence from human oocytes that the introduction of at least Sgo1 decreases PSSC and decreases interkinetochore distances. This technical effect is expected to decrease the risk of infertility miscarriages, implantation failure and/or embryonic arrest in assisted reproductive technology.

The invention is also further described in the following numbered embodiments:
1. An *in vitro* method of introducing (i) Shugoshin 1 ("Sgo1") protein or (ii) mRNA encoding Sgo1 into an oocyte or an egg.
2. The method of embodiment 1, wherein the mRNA encoding Sgo1 protein or the Sgo1 protein reduces aneuploidy compared to an oocyte or an egg cell not having the mRNA encoding Sgo1 protein or the Sgo1 protein introduced.
3. The method of embodiment 1 or 2, wherein Sgo1 protein is introduced to the oocyte or the egg in an effective amount to reduce aneuploidy compared to an oocyte or an egg not having Sgo1 protein introduced.
4. The method of embodiment 3, wherein the effective amount of Sgo1 protein introduced to the oocyte or the egg is from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg.
5. The method of embodiments 3 or 4, wherein the effective amount of Sgo1 protein is introduced at a concentration from 0.1 to 30 mg/ml, more preferably from 0.5 to 15 mg/ml, even more preferably from 1 to 10 mg/ml.
6. The method of any of the preceding embodiments, wherein Sgo1 protein binds PP2A protein and/or at the centromere, preferably assessed by microscopic co-localization.
7. The method of any of the preceding embodiments, wherein the Sgo1 protein is a recombinant protein.
8. The method of any of the preceding embodiments, wherein the Sgo1 protein comprises, and preferably consists of, a sequence having at least 70% identity to SEQ ID NO:1, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 1.
9. The method of embodiments 1 or 2, wherein the mRNA encoding Sgo1 translates to an amino acid sequence comprising, preferably consisting of, a sequence having at least 70% identity to SEQ ID NO: 1, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identity to SEQ ID NO: 1.
10. The method of any of embodiments 1-2 or 9, wherein the mRNA encoding Sgo1 is introduced to the oocyte or the egg in an effective amount to reduce aneuploidy compared to an oocyte or an egg not having such mRNA introduced.
11. The method of embodiment 10, wherein the effective amount of mRNA encoding Sgo1 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 100 to 1000 fg per oocyte or egg, and most preferably and most preferably from 140 to 750 fg per oocyte or egg; or wherein the effective amount of mRNA encoding Sgo1 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 70 to 700 fg per oocyte or egg, even more preferably from 70 to 500 fg per oocyte or egg, even more preferably from 75 to 400 fg per oocyte or egg, even more preferably from 80 to 320 fg per oocyte or egg, even more preferably 80 fg or 320 fg per oocyte or egg, and most preferably 80 fg per oocyte or egg.
12. The method of embodiments 10 or 11, wherein the effective amount of mRNA encoding Sgo1 is introduced at a concentration from 7-700 ng/µl, more preferably from 20-100 ng/µl, even more preferably from 30 to 80 ng/µl, even more preferably from 20 to 80 ng/µl, even more preferably wherein the effective amount of mRNA encoding Sgo1 is introduced at the concentration of 20 ng/µl or 80 ng/µl, and most preferably wherein the effective amount of mRNA encoding Sgo1 is introduced at the concentration of 20 ng/µl.
13. The method of any of the preceding embodiments, wherein (i) mRNA encoding Sgo2 protein or (ii) Sgo2 protein is additionally introduced into the oocyte or the egg.
14. The method of the embodiment 13, wherein the mRNA encoding Sgo2 protein or the Sgo2 protein reduces aneuploidy compared to an oocyte or an egg not having mRNA encoding Sgo2 protein or Sgo2 protein introduced.
15. The method of embodiments 13 or 14, wherein the Sgo2 protein binds centromeres.
16. The method of any of embodiments 13-15, where the Sgo2 protein is a recombinant protein.
17. The method of any of embodiments 13-16, wherein the Sgo2 protein is introduced to the oocyte or the egg in an effective amount to reduce aneuploidy compared to an oocyte or an egg not having Sgo2 protein introduced.
18. The method of embodiment 17, wherein the effective amount of Sgo2 protein introduced to the oocyte or the egg is from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg.
19. The method of embodiments 17 or 18, wherein the effective amount of Sgo2 protein is introduced at a concentration from 0.1 - 30 mg/ml, more preferably 0.5-15 mg/ml, even more preferably 1-10 mg/ml.
20. The method of any of embodiments 13-19, wherein the Sgo2 protein comprises, and preferably consists of, a sequence being at least 70% identical to SEQ ID NO: 2, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 2.
21. The method of any of the embodiments 13 or 14, wherein the mRNA encoding Sgo2 translates to an amino acid sequence comprising, preferably consisting of, a sequence having at least 70% identity to SEQ ID NO:2, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identical to SEQ ID NO: 2.
22. The method of any of the embodiments 13-14 or 21, wherein the Sgo2 mRNA is introduced to the oocyte or the egg in an effective amount to reduce aneuploidy compared to an oocyte or an egg not having such mRNA introduced.
23. The method of embodiment 22, wherein the effective amount of mRNA encoding Sgo2 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte, more preferably from 50 to 1900 fg, even more preferably from 60 to 1700 fg per oocyte or egg, even more preferably from 70 to 1500 fg per oocyte or egg, even more preferably from 100 to 1000 fg per oocyte or egg, and most preferably and most preferably from 140 to 750 fg per oocyte or egg; or wherein the effective amount of mRNA encoding Sgo2 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 70 to 700 fg per oocyte or egg, even more preferably from 70 to 500 fg per oocyte or egg, even more preferably from 75 to 400 fg per oocyte or egg, even more preferably from 80 to 320 fg per oocyte or egg, even more preferably 80 fg or 320 fg per oocyte or egg, and most preferably 80 fg per oocyte or egg.
24. The method of embodiments 22 or 23, wherein the effective amount of mRNA encoding Sgo2 is introduced at a concentration from 6.5 to 650 ng/µl, more preferably from 10 to 100 ng/µl, even more preferably from 30 to 80 ng/µl, even more preferably from 20 to 80 ng/µl, even more preferably wherein the effective amount of mRNA encoding Sgo2 is introduced at the concentration of 20 ng/µl or 80 ng/µl, and most preferably wherein the effective amount of mRNA encoding Sgo2 is introduced at the concentration of 20 ng/µl.
25. The method of any of the preceding embodiments, wherein a satellite RNA is additionally introduced into the oocyte or egg.
26. The method of embodiment 25, wherein the satellite RNA is an alpha satellite RNA.
27. The method of embodiments 25 or 26, wherein the sequences of the Sgo1, Sgo2 and/or satellite RNA are sequences of one species.
28. The method of any of the embodiments 25-27, wherein the satellite RNA is an *in vitro* generated transcript from a non-coding centromeric or pericentromeric DNA region of a mammal.
29. The method of the embodiment 28, wherein the mammal is a human, a canine, a rabbit or a livestock mammal, preferably wherein the livestock mammal is cattle, a horse, a goat, a rabbit, a sheep, a pig, a donkey, a llama, a camel, or an alpaca.
30. The method of any of the embodiments 25-29, wherein the satellite RNA is the alpha satellite RNA and wherein the alpha satellite RNA comprises, and preferably consists of, a sequence having at least 70% identity to SEQ ID:3, preferably at least 75%, more preferably at least 80%, more preferably at least 81%, more preferably at least 82%, more preferably at least 83%, more preferably at least 84%, more preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, even more preferably at least 99%, and most preferably 100% identity to SEQ ID NO: 3.
31. The method of any of the embodiments 25-30, wherein the satellite RNA is introduced to the oocyte or the egg in an effective amount to reduce aneuploidy compared to an oocyte or an egg not having such satellite RNA introduced.
32. The method of embodiment 31, wherein the effective amount of satellite RNA is introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 100 to 1000 fg per oocyte or egg, and most preferably and most preferably from 140 to 750 fg per oocyte or egg; or wherein the effective amount of mRNA encoding Sgo1 introduced to the oocyte or the egg is from 1 to 4000 fg per oocyte or egg, more preferably from 5 to 3000 fg per oocyte or egg, more preferably from 20 to 2500 fg, more preferably from 35 to 2000 fg per oocyte or egg, more preferably from 50 to 1900 fg, even more preferably from 60 to 1700 fg per oocyte or egg, even more preferably from 70 to 1500 fg per oocyte or egg, even more preferably from 70 to 700 fg per oocyte or egg, even more preferably from 70 to 500 fg per oocyte or egg, even more preferably from 75 to 400 fg per oocyte or egg, even more preferably from 80 to 320 fg per oocyte or egg, even more preferably 80 fg or 320 fg per oocyte or egg, and most preferably 80 fg per oocyte or egg.
33. The method of embodiments 31 or 32, wherein the effective amount of the satellite RNA is introduced at a concentration from 4 to 400 ng/µl, more preferably from 10 to 200 ng/µl, even more preferably from 20 to 80 ng/µl, even more preferably wherein the effective amount of mRNA encoding Sgo1 is introduced at the concentration of 20 ng/µl or 80 ng/µl, and most preferably wherein the effective amount of mRNA encoding Sgo1 is introduced at the concentration of 20 ng/µl.
34. The method of any preceding embodiments, where the oocyte or the egg is a mammalian oocyte or egg, preferably wherein the mammalian oocyte or egg is from a human or from a livestock mammal, more preferably wherein the livestock mammal is cattle, a horse, a goat, a rabbit, a sheep, a pig, a donkey, a llama, a camel, or an alpaca.
35. The method of embodiment 34, where the oocyte or egg is a human oocyte or a human egg.
36. The method of embodiment 34 or 35, wherein the human oocyte or the human egg is an aged oocyte or an aged egg, preferably wherein the aged oocyte or the aged egg is at least 35 years old.
37. The method of any of embodiments 34-36, wherein the oocyte or the egg is from a human having a medical record of at least one failed *in vitro* fertilization cycle.
38. The method of any of embodiments 34-37, wherein the oocyte or the egg is from a human having a medical history of being unable to conceive for a period of at least two years.
39. The method of embodiment 34, wherein the mammalian oocyte or the mammalian egg is from an endangered animal.
40. The method of embodiment 39, wherein the endangered animal is selected from the list comprising a panda, an orangutan, a saola, a tiger, a rhinoceros, a leopard, a gorilla, an elephant, a bonobo, or a dolphin, a tapir, a zebra, a gibbon, a chimpanzee, a lemur, a macaque, a monkey, a deer, a giraffe, a whale, a lynx, a seal, a gazelle, an antelope, a possum, a wombat, a wolf, a pangolin, a sloth, and a cheetah.
41. The method of any preceding embodiments, wherein the oocyte or the egg prior to carrying out the method has at least a 5% increased probability of having a premature separation of sister chromatids (PSSC), preferably at least 10%, more preferably at least 15%, more preferably at least 20%, more preferably at least 25%, and even more preferably at least 35% compared to an average oocyte in reproductive prime age.
42. The method of any preceding embodiments, wherein the oocyte or the egg has at least 5% reduction in natural Sgo1 levels, preferably at least 10% reduction, more preferably at least 15% reduction, even more preferably at least 25% reduction and even more preferably at least 35% reduction compared to an average oocyte or average egg in reproductive prime age.
43. The method of embodiment 42, wherein the level of Sgo1 is detectable by using fluorescence microscopy or polarized light microscopy, preferably wherein Sgo1 protein is detected with mouse anti-Sgo1 antibody or nanobody, more preferably with HOO151648-M01 from Abnova; or wherein the level of Sgo1 mRNA is detectable by RNA FISH.
44. The method of any preceding embodiments, wherein the oocyte or the egg after having carried out the method has at least a 5% reduced probability of having a premature separation of sister chromatids (PSSC), preferably at least 10%, more preferably at least 15%, more preferably at least 20%, more preferably at least 25%, and even more preferably at least 35% compared to an oocyte or an egg not undergoing the method.
45. The method of any preceding embodiments, wherein the oocyte or the egg prior to carrying out the method has a reduction in natural Sgo2 levels of at least 10%, preferably at least 20%, more preferably at least 30%, and even more preferably at least 40% compared to an average oocyte or an average egg in reproductive prime age.
46. The method of embodiment 45, wherein the level of Sgo2 protein is detectable by using fluorescence microscopy or polarized light microscopy, preferably wherein Sgo2 protein is detected with rabbit anti-Sgol2 antibody or nanobody, more preferably with orb499806 from Biorbyt; or the level of Sgo2 mRNA is detectable by RNA FISH.
47. The method of any preceding embodiments, wherein the oocyte or the egg prior to carrying out the method has at least 10% reduction in PP2A levels, preferably at least 20% reduction, more preferably at least 35% reduction and even more preferably at least 50% reduction compared to an average oocyte and average egg in reproductive prime age.
48. The method of embodiment 47, wherein the level of PP2A is detectable by using fluorescence microscopy or polarized light microscopy, preferably wherein PP2A is detected by an anti-PP2A antibody or nanobody, more preferably wherein the anti-PP2A antibody is a rabbit anti-PP2A catalytic subunit antibody, even more preferably the rabbit anti-PP2A catalytic subunit antibody number 05-421 from Merck or the rabbit anti-PP2A catalytic subunit antibody number 2038S from Cell Signaling; or wherein the level of PP2A mRNA is detectable by RNA FISH.
49. The method of any preceding embodiments, wherein the oocyte or the egg prior to carrying out the method has at least 15% reduction in Rec8 levels at centromeres, preferably at least 25% reduction, more preferably at least 35% reduction and even more preferably at least 55% reduction compared to an average oocyte or an average egg in reproductive prime age.
50. The method of embodiment 49, wherein the level of Rec8 is detectable by using fluorescence microscopy or polarized light microscopy, preferably wherein the Rec8 protein is detected with rabbit anti-Rec8 antibody from Cambridge Research Biochemicals; or the level of Rec8 mRNA is detectable by RNA FISH.
51. The method of any preceding embodiments, wherein the oocyte or the egg prior to carrying out the method has reduced transcription during meiotic divisions, in particular centromeric transcription, by at least 10%, preferably 20%, more preferably at least 30% compared to an average oocyte or an average egg in reproductive prime age.
52. The method of embodiment 51, wherein the transcription level is detectable by using fluorescence or polarized light microscopy, preferably with an anti-RNA PolII Sp2 antibody; or by RNA FISH detecting alpha satellite RNA.
53. The method of any preceding embodiments, wherein the oocyte or the egg prior to carrying out the method has an increased interkinetochore distance by at least 10% when compared to an average oocyte or an average egg in reproductive prime age, more preferably by at least 20%, and the most preferably by at least 30% when compared to an average oocyte or an average egg in reproductive prime age.
54. The methods of embodiments 41-53, wherein the reproductive prime age for a human oocyte or a human egg is from 18 to 29 years, more preferably from 20 to 27 years, even more preferably from 23 to 25 years.
55. The method of any preceding embodiments, wherein the mRNA encoding Sgo1 protein or the Sgo1 protein, preferably in combination with the (i) mRNA encoding Sgo2 protein or the Sgo2 protein and/or (ii) the satellite RNA, is/are introduced to the oocyte or the egg during germinal vesicle stage, meiosis I or meiosis II, more preferably during germinal vesicle stage or late meiosis I stage. is/are introduced to the oocyte or the egg during prophase arrest, meiosis I, or meiosis II, in particular during the germinal vesicle stage, meiosis I prometaphase, meiosis I metaphase, meiosis I anaphase, meiosis I telophase, or, meiosis II metaphase.
56. The method of embodiment 55, wherein the mRNA encoding Sgo1 or the Sgo1 protein, preferably in combination with the (i) mRNA encoding Sgo2 protein or the Sgo2 protein and/or (ii) the satellite RNA, is/are introduced between the germinal vesicle stage and anaphase I, and most preferably during germinal vesicle stage, meiosis I prometaphase or meiosis I metaphase.
57. An *in vitro* method of introducing (a) mRNA encoding Sgo2 protein or (b) Sgo2 protein into an oocyte or an egg.
58. The method of embodiment 57, wherein the mRNA encoding Sgo2 protein or the Sgo2 protein is defined in any of the embodiments 13-24, and/or wherein the oocyte or the egg is further defined in any of embodiments 34-54.
59. The method of embodiments 57 or 58, wherein a satellite RNA is additionally introduced into the oocyte or the egg as further defined in any of embodiments 25-33.
60. The method of any of embodiments 57-59, wherein the mRNA encoding Sgo2 protein or the Sgo2 protein, preferably in combination with the satellite RNA, is/are introduced to the oocyte or the egg during germinal vesicle stage, meiosis I or meiosis II, more preferably during germinal vesicle stage or late meiosis I stage. is/are introduced to the oocyte or the egg during prophase arrest, meiosis I, or meiosis II, in particular during the germinal vesicle stage, meiosis I prometaphase, meiosis I metaphase, meiosis I anaphase, meiosis I telophase, or, meiosis II metaphase.
61. The method of any of embodiments 57-60, wherein the mRNA encoding Sgo2 protein or the Sgo2 protein, preferably in combination with the satellite RNA, is/are introduced between the germinal vesicle stage and anaphase I, and most preferably during germinal vesicle stage, meiosis I prometaphase or meiosis I metaphase.
62. A method comprising the steps of
   a) providing a composition comprising (i) or mRNA encoding Sgo1 protein (ii) Sgo1 protein;
   b) *in vitro* introduction of the composition of step (a) into the oocyte or the egg.
63. The method of the embodiment 62, wherein the composition in step (a) additionally comprises (i) mRNA encoding Sgo2 protein or (ii) Sgo2 protein.
64. The method of embodiments 62 or 63, wherein the Sgo1 protein or mRNA is further defined in any of embodiments 2-12, the Sgo2 protein or mRNA is further defined in any of the embodiments 13-24, and/or wherein the oocyte or the egg is further defined in any of embodiments 34-54.
65. The method of any of embodiments 62-64, wherein a satellite RNA is additionally introduced as further defined in any of embodiments 25-33.
66. The method of any of embodiments 62-65, wherein the mRNA encoding Sgo2 protein or the Sgo2 protein, preferably in combination with the satellite RNA, is/are introduced during germinal vesicle stage, meiosis I or meiosis II, more preferably during germinal vesicle stage or late meiosis I stage.
67. The method of any of embodiments 57-60, wherein the mRNA encoding Sgo2 or the Sgo2 protein, preferably in combination with the satellite RNA, is/are introduced to the oocyte or the egg during prophase arrest, meiosis I, or meiosis II, in particular during the germinal vesicle stage, meiosis I prometaphase, meiosis I metaphase, meiosis I anaphase, meiosis I telophase, meiosis II metaphase, even more preferably between the germinal vesicle stage and anaphase I, and most preferably during germinal vesicle stage, meiosis I prometaphase or meiosis I metaphase.
68. A method comprising the steps of
   a) providing a composition comprising (i) mRNA encoding Sgo2 protein or (ii) Sgo2 protein;
   b) *in vitro* introduction of the composition of step (a) into an oocyte or an egg.
69. The method of embodiment 68, wherein the Sgo2 protein or mRNA is further defined in any of the embodiments 13-24, and/or wherein the oocyte or the egg is further defined in any of embodiments 34-54.
70. The method of embodiments 68 or 69, wherein a satellite RNA is additionally introduced into the oocyte or the egg as further defined in any of embodiments 25-33.
71. The method of any of embodiments 68-70, wherein the mRNA encoding Sgo2 protein or the Sgo2 protein, preferably in combination with the satellite RNA, is/are introduced during germinal vesicle stage, meiosis I or meiosis II, more preferably during germinal vesicle stage or late meiosis I stage.
72. The method of any of embodiments 68-71, wherein the mRNA encoding Sgo2 protein or the Sgo2 protein, preferably in combination with the satellite RNA, is/are introduced during meiosis I prophase, meiosis I metaphase, meiosis I anaphase, meiosis I telophase, and more preferably during germinal vesicle stage or meiosis I anaphase or meiosis I telophase.
73. The method of any of the preceding embodiments, wherein the introduction to the oocyte or egg is carried out by microinjection, electroporation, transfection, and/or using a microfluidics device, preferably by microinjection.
74. The method of any preceding embodiments, wherein the method does not include nuclear transfer.
75. The method of any preceding embodiments, wherein the method is used in assisted reproductive technologies.
76. A (i) mRNA encoding Sgo1 protein or (ii) a Sgo1 protein for use in preventing infertility, miscarriage, implantation failure, or embryonic arrest, wherein the mRNA encoding Sgo1 protein or Sgo1 protein is introduced into an oocyte, an egg, or a zygote; preferably wherein the infertility, miscarriage, implantation failure, or embryonic arrest is caused by or associated with a chromosomal aberration, more preferably wherein the chromosomal aberration is aneuploidy.
77. A (i) mRNA encoding Sgo1 protein or (ii) a Sgo1 protein for use in a method of preventing (i) a chromosomal aberration or (ii) a chromosomal aberration associated disease or (iii) a chromosomal aberration associated disorder.
78. The (i) the mRNA encoding Sgo1 protein or (ii) Sgo1 protein of embodiment 76 or 77, wherein the (i) chromosomal aberration or (ii) chromosomal aberration associated disease or (iii) chromosomal aberration associated disorder is aneuploidy, more preferably a trisomy or a monosomy, more preferably trisomy 21 (Down syndrome), trisomy 13 (Edwards syndrome), trisomy 18 (Patau syndrome) or Turner syndrome (X chromosome monosomy).
79. The (i) mRNA encoding Sgo1 protein or (ii) the Sgo1 protein of any of embodiments 76-78, wherein (i) said mRNA encoding Sgo1 protein or (ii) said Sgo1 protein is introduced in combination with (a) mRNA encoding Sgo2 protein or (b) Sgo2 protein.
80. The (i) the mRNA encoding Sgo1 protein or (ii) Sgo1 protein of any of embodiments 76-79, wherein (i) said mRNA encoding Sgo1 protein or (ii) said Sgo1 protein is introduced in combination with a satellite RNA, preferably an alpha satellite RNA.
81. A (a) mRNA encoding Sgo2 protein or (b) a Sgo2 protein for use in preventing infertility, miscarriage, implantation failure, or embryonic arrest, wherein the mRNA encoding Sgo1 protein or Sgo1 protein is introduced into an oocyte, an egg, or a zygote; preferably wherein the infertility, miscarriage, implantation failure, or embryonic arrest is caused by or associated with a chromosomal aberration, more preferably wherein the chromosomal aberration is aneuploidy.
82. A (a) mRNA encoding Sgo2 protein or (b) Sgo2 protein for use in a method of preventing (i) a chromosomal aberration or (ii) a chromosomal aberration associated disease or (iii) a chromosomal aberration associated disorder, wherein the mRNA encoding Sgo1 protein or Sgo1 protein is introduced into an oocyte, an egg, or a zygote.
83. The (a) mRNA encoding Sgo2 protein or (b) the Sgo2 protein of embodiment 81 or 82, wherein the (i) chromosomal aberration or (ii) chromosomal aberration associated disease or (iii) chromosomal aberration associated disorder is aneuploidy, more preferably a trisomy or a monosomy, more preferably trisomy 21 (Down syndrome), trisomy 13 (Edwards syndrome), trisomy 18 (Patau syndrome) or Turner syndrome (X chromosome monosomy).
84. A pharmaceutical composition comprising an mRNA encoding Sgo1 or the Sgo1 protein, wherein the pharmaceutical composition additionally comprises (I) an mRNA encoding Sgo2 protein or a Sgo2 protein, (II) an mRNA encoding PP2A or PP2A protein and/or (III) a satellite RNA, and wherein the pharmaceutical composition is suitable for introduction into an oocyte or, an egg or a zygote.
85. The pharmaceutical composition of embodiment 84, wherein
   I. the Sgo1 and the Sgo2 form a complex after introduction into the oocyte or the egg;
   II. the Sgo1 and the PP2A form a complex after introduction into the oocyte or the egg; and/or
   III. the Sgo1 protein and the satellite RNA are forming a complex after introduction into the oocyte or the egg,
   preferably wherein the Sgo1, the Sgo2, the PP2A and/or the satellite RNA form a complex after introduction into the oocyte or the egg.
86. The pharmaceutical composition of any of the embodiments 84 or 85, wherein the satellite RNA is further defined in any of embodiments 25-33.
87. The pharmaceutical composition of any of embodiments 84-86 in an effective amount to reduce aneuploidy.
88. The pharmaceutical composition of embodiment 87, wherein the effective amount of the complex is from 1 to 250 pg per oocyte or egg, more preferably from 2 to 150 pg per oocyte or egg, more preferably from 3 to 100 pg per oocyte or egg, more preferably from 4 to 80 pg per oocyte or egg, even more preferably from 5 to 50 pg per oocyte or egg, even more preferably from 6 to 30 pg per oocyte or egg, even more preferably even more preferably from 7 to 20 pg per oocyte or egg, even more preferably from 8 to 15 pg per oocyte or egg, even more preferably from 9 to 12 pg per oocyte or egg, and most preferably around 10 pg per oocyte or egg.
89. The pharmaceutical composition of embodiment 87 or 88, wherein the effective amount is introduced at a concentration from 0.1 - 30 mg/ml, more preferably 0.5-15 mg/ml, even more preferably 1-10 mg/ml.
90. The pharmaceutical composition of any of the embodiments 84-89, comprising mRNA encoding Sgo2 protein or Sgo2 protein as further defined in any of the embodiments 13-24.
91. A needle, capillary or injection pen for microinjection comprising the pharmaceutical composition of embodiments 84-90.
92. A kit comprising a pharmaceutical composition any of the embodiments 84-90.
93. The kit of embodiment 92 comprising the needle, capillary or injection pen for microinjection of embodiment 91.
94. The kit as in embodiments 92 or 93, additionally comprising instructions for use.

### Examples

The following examples are intended to illustrate the invention further, but are not limited to it. The examples describe technical features, and the invention also relates to combinations of the technical features presented in this section.

### Example 1

### Sgo1 protects chromosome integrity in mouse eggs

As aneuploidy in eggs is an increased risk in ageing women for infertility and birth defects, there is a need to prevent premature separation of sister chromatids, the major cause of age-related aneuploidy. The inventors first investigated the molecular mechanisms that maintain sister chromatid cohesion in young oocytes. By expressing fluorescently labeled Sgo1 in mouse oocytes, it was found that Sgo1 localized to an extended region around the centromeres at both metaphase I and metaphase II stages (Fig. 1A). Previous work had established that Sgo2 is required to maintain sister chromatid cohesion in oocytes. However, Sgo1's function in oocyte meiosis was unclear. To test if Sgo1 is important for sister chromatid cohesion in eggs, Sgo1 was depleted from oocytes using long-term follicle culture (52). In particular, follicle-enclosed oocytes were microinjected with two different Sgo1 siRNAs at the MII stage (52). Both siRNAs led to a decrease of Sgo1 in metaphase II eggs (Fig. 1C). Importantly, misaligned chromosomes and PSSC were significantly increased in eggs injected with either of the two Sgo1 siRNAs compared to controls (Fig. 1D, E). The fraction of eggs with chromosome errors was substantially higher than in previous studies, where fully grown oocytes were treated with siRNAs (32). This is likely due to a more complete depletion of Sgo1 over 10 days of follicle culture compared to shorter depletion in fully grown oocytes. Together, these data show that Sgo1 protects chromosome integrity to maintain sister chromatid cohesion in mouse oocytes and eggs.

### Centromeric transcription prevents PSSC and maintains CENP-A in oocytes

Next, the inventors investigated, how Sgo1 is recruited specifically to the pericentromeric region of chromosomes. A recent study in somatic cells has shown that centromeric transcription plays a role in Sgo1 recruitment to the inner centromere (49). The inventors therefore studied if transcription was necessary for progression through meiosis and for the maintenance of sister chromatid cohesion in mouse oocytes. To test this, the meiotic maturation of oocytes was followed in the presence of the transcription inhibitor, triptolide, which inhibits transcription initiation by RNA polymerases (RNA Pols) (53). Time-lapse imaging revealed that triptolide lead to increased chromosome misalignment at the MII stage compared to controls (DMSO-treated) (Fig. 2C). Furthermore, using high-resolution microscopy, a significant increase in PSSC was observed (Fig. 2E). Another transcription inhibitor, α-amanitin, also led to a significant increase in PSSC, at a level similar to that seen with triptolide (Fig. 1I).

Triptolide significantly reduced actively elongating RNA Pol II at the centromere (Fig. 2F). The mouse inner centromere and pericentromere regions are composed of more ordered minor satellite (MinSat) and less ordered major satellite (MajSat) repeats, respectively, and both regions are reported to be transcriptionally active to generate non-coding RNAs (47, 51-54). The inventors found that triptolide significantly reduced major satellite repeat RNAs, suggesting that pericentromeric transcription is reduced upon triptolide treatment (Fig. 2J). In addition, triptolide significantly reduced CENP-A at the centromere, suggesting a role for transcription in maintaining centromere organization and kinetochore assembly (Fig. 3F). Taken together, these data demonstrate that transcription is required to maintain robust sister chromatid cohesion in mouse oocytes, which is expected to be the case in all mammalian oocytes.

### Sgo1 and PP2A recruitment to centromeres requires centromeric transcription

Work in yeast, fly, Xenopus and mammals showed that Sgo proteins protect cohesion in the pericentromeric region by recruiting a protein phosphatase 2A (PP2A) (34,59-61). PP2A locally dephosphorylates the Rec8 subunit of the cohesion complex so that it is no longer susceptible to separase cleavage (26,31,32,59,60,62-65). While hSgo1 only binds to the AB'C PP2A holoenzyme, hSgo2 was reported to make stable interaction with PP2A's C subunit and also with individual PP2A subunits along with AC core complex (65). This further demonstrates that PP2A recruited by different Shugoshin proteins might regulate phosphorylation of distinct substrates.

Therefore, the inventors asked whether triptolide treatment affects sister chromatid cohesion by affecting the levels of Sgo1 and Sgo2 at the centromere. The inventors found that Sgo1 levels were significantly reduced at the mouse pericentromere, whereas Sgo2 levels were not (Fig. 1G; Fig. 3G). Quantification of PP2A-α levels after triptolide treatment showed that triptolide significantly reduced PP2A levels in comparison to controls (Fig. 1H). Together, these findings show that transcription is essential to maintain high levels of Sgo1 and PP2A in the centromeric region.

### PSSC upon triptolide treatment can be rescued with exogenous MajSat RNA

In human mitotic cells, Sgo1 binds to centromeric α-satellite transcripts and RNA polymerase II with its basic region (49). Therefore, the inventors tested whether the PSSC defects in triptolide-treated oocytes could be rescued by exogenous delivery of MajSat RNA, which was reduced upon transcriptional inhibition. Using time-lapse imaging, it was found that exogenous MajSat RNA localized to pericentromeres, similar to the localization of Sgo1. Co-localization of MajSat RNA and Sgo1 was further confirmed by performing RNA FISH followed by immunofluorescence. The exogenous delivery of MajSat RNA significantly reduced the incidence of PSSC in Triptolide-treated oocytes (Fig. 3B). Furthermore, exogenous delivery of MajSat RNA resulted in the rescue of MajSat RNA, Sgo1, and PP2A levels at the pericentromere, in further support of a role for MajSat RNA in Sgo1 protein function at the centromere (Fig. 3C-E). The inventors conclude that exogenous MajSat transcripts can rescue Sgo1 and PP2A levels upon inhibition of transcription, and reduce PSSC in triptolide-treated young oocytes.

### PSSC in aged oocytes is accompanied by a reduction of centromeric transcription, Sgo1 and PP2A

Next, the mechanism that causes increased PSSC in aged oocytes was investigated. At first, the incidence of PSSC in young (8-12 weeks old) and aged mouse eggs (60-65 weeks old) was quantified (Fig. 4B-4C). The incidence and frequency of PSSC increase significantly in aged eggs, consistent with its role as a major contributor to the age-dependent increase in aneuploidy. In addition, the distance between sister kinetochores increases significantly in eggs from aged mice compared to young ones (Fig. 4D).

The inventors used high-resolution microscopy to systematically study levels of cohesins and proteins that are responsible for cohesion protection on chromosome spreads of MI chromosomes. Previous studies had shown that chromosome-associated Rec8 levels are reduced in aged oocytes compared to young oocytes (19-21). It was found that Rec8 not only decreased at chromosome arms, but it was also significantly reduced at aged centromeres compared to young centromeres (Fig. 4E).

Sgo1 was greatly reduced at the centromeres of aged oocytes (Fig. 4H). Also, Sgo2 was reduced, consistent with a previous study (66). Although Sgo2 is known to protect centromeric cohesion in meiosis, a more significant reduction in Sgo1 levels than that of Sgo2 was found (31,32,38). Interestingly, Sgo1 was localized to a much larger area around the pericentromere, whereas Sgo2 is localized to the inner centromere. Furthermore, the inventors found that Sgo1 protein levels are greatly reduced in aged germinal vesicle (GV) oocytes when compared to young oocytes on a Western blot, consistent with a reduction of the total Sgo1 pool.

PP2A is recruited to the centromere by Shugoshin to counteract centromeric Rec8 phosphorylation during MI. PP2A was dramatically reduced at aged kinetochores, which may lead to premature Rec8 cleavage and further aneuploidy (Fig. 4F). Furthermore, centromeric transcription was significantly reduced in aged oocytes compared to young oocytes (Fig. 2H, 2I).

Taken together, these data demonstrate that many arms of pericentromeric cohesion protection - Sgo1, Sgo2, PP2A and pericentromeric transcription - are all dramatically reduced in aged oocytes and all of them individually and in combination represent a promising therapeutic target for prevention of chromosomal aberrations, such as aneuploidy.

### Example 2

### Sgo1 and Sgo2 supplementation rescues PSSC in aged eggs

Next, the inventors aimed to develop a strategy to reduce PSSC in aged eggs. Having established which molecules that protect sister chromatid cohesion decline in aged oocytes, the inventors next examined, whether reintroduction of any of these molecules can reduce or even prevent PSSC (Fig. 5A).

First, the incidence of PSSC and inter-kinetochore distance were examined in aged eggs following introduction by microinjection of either Sgo1 or Sgo2 or both. PSSC and inter-kinetochore distances were strikingly reduced when Sgo1 was expressed in aged eggs, reaching the same low levels as in young eggs (Fig. 5B-5D). The Sgo1 N61I mutant that was unable to bind to PP2A (65, 67, 68) was unable to rescue PSSC, showing that Sgo1 prevents PSSC by mediating PP2A recruitment to the pericentromeric region (Fig. 5E-5G). Sgo2 supplementation resulted in a reduction of PSSC as well, but to a lesser degree than Sgo1 (Fig. 5B). Supplementation of Sgo1 together with Sgo2 completely abolished PSSC in aged eggs, accompanied by a significant reduction in inter-kinetochore distances (Fig. 5E-5G). Co-expression of Sgo1 with MajSat RNA also reduced the incidence of PSSC along with the inter-kinetochore distance in aged eggs. Supplementation of MajSat RNA alone only had a minor effect on PSSC rates (Fig. 5E-5G).

### Sgo1 increases Rec8 and PP2A on chromosomes in aged oocytes and eggs

The inventors next asked if Sgo1 prevents PSSC in aged oocytes by increasing the levels of Rec8 and PP2A. Supplementation of Sgo1 significantly increased Rec8 and PP2A levels at MI centromeres compared to untreated oocytes (Fig. 6A, 6B). In contrast, Sgo2 expression in aged oocytes did not significantly alter PP2A and Rec8 levels in aged MI oocytes, consistent with a weaker effect on PSSC in aged mouse oocytes (Fig. 6A, 6B).

Sgo1 supplementation also increased the levels of Rec8 and PP2A in aged eggs during metaphase II (Fig. 6C, 6D). Sgo1 and Sgo2 co-supplementation also resulted in higher Rec8 and PP2A levels in aged compared to untreated controls (Fig. 6C, 6D). Together, these results show that supplementing aged mouse oocytes with Sgo1 alone or Sgo2 alone, or a combination of Sgo1 and Sgo2 can rescue PSSC by promoting the recruitment of PP2A and protecting Rec8 in the centromeric and pericentromeric region. Based on this data alone, supplementation with Sgo1 and/or Sgo2 is expected to rescue PSSC in other mammalian oocytes, such as human, as demonstrated in mice in this Example. Data from human oocytes is provided in Example 3.

### Example 3

### Aged human oocytes lose Sgo1 at the inner centromere and pericentromere bridge

Next, the inventors investigated if similar cohesion protection and aging mechanisms might play a role during PSSC in human eggs. Sgo1 localized to a large region around the pericentromere in both oocytes at MI and eggs at MII stage, consistent with a previous study (69). To examine if centromeric transcription is required for protecting sister chromatid cohesion in human oocytes, human oocytes were treated with the transcription inhibitor, triptolide, and examined chromosomes in MII. Triptolide increased the incidence of PSSC in eggs from young women and caused a decrease in Sgo1 levels (Fig. 7B, 7C).

Next, the inventors investigated if Sgo1 levels decline in human oocytes with advanced female age. To this end, Sgo1 levels on chromosomes from oocytes of women who were younger, and older than 35 years, were quantified, corresponding to reproductively young and advanced age groups (69). Sgo1 levels were significantly reduced on old chromosomes compared to young chromosomes (Fig. 7A), indicating that Sgo1 levels also declined in aged human oocytes. It was further observed, that exogenous Sgo1 localizes to the human MI and MII centromeric regions.

The inventors also introduced mRNA encoding Sgo1 into human oocytes at the GV or MI stage and subsequently matured the oocytes in vitro to the MII stage (Fig. 8A). The inventors investigated whether similar cohesion protection and aging mechanisms might be involved in PSSC in human oocytes. Sgo1 localized to a large region around the pericentromere in both MI and MII human oocytes consistent with a previous study (8). In vitro introduction of Sgo1 reduced the percentage of eggs with PSSC (Fig. 8B). The percentage of eggs with PSSC was reduced from 64% in the control to 43% upon introduction of exogenous Sgo1 (Fig. 8B). The distances between sister chromatid kinetochores at MII were reduced from 2.2 µm in control eggs to 1.5 µm upon introduction of exogenous Sgo1 (Fig. 8C), indicating increased cohesion in the centromeric region.

The inventors next investigated whether Sgo1 levels in human oocytes decrease with advancing female age. The inventors quantified Sgo1 levels on chromosomes from oocytes from women who were younger than 35 or aged equal and older than 35 years, corresponding to reproductively young and advanced age groups (8). Sgo1 levels were significantly reduced on old chromosomes compared to young chromosomes (Fig. 8D), indicating that Sgo1 levels decrease in aged human oocytes.

### Example 4

### Sgo1 supplementation reduces PSSC in human aged eggs

The inventors then examined further whether supplementation of exogenous Sgo1 can reduce PSSC in human eggs. To this end, the incidence of PSSC in human eggs after microinjection of two different concentrations of Sgo1 mRNA was examined (treated with 20 or 80 ng/µl). The inventors found a significant reduction in PSSC when Sgo1 was expressed in human eggs at a concentration of 20 ng/µl (Fig. 8E, 8G). Strikingly, Sgo1 mRNA expression (20 ng/µl) significantly reduced the percentage of eggs with PSSC and frequency of PSSC in eggs from aged women (≥35 years, Fig. 8G, 8J), in line with our observations with aged mouse eggs. Eggs supplemented with the higher concentration of Sgo1 mRNA (80 ng/µl) showed moderate PSSC reduction (Figure 9B). The inventors only had very few oocytes from donors aged 35 and older in the 80 ng/µl treatment group, which likely explains the above average rate of PSSC-free eggs in the control group (7 oocytes) and below average rate of PSSC-free eggs in the treated group (3 oocytes) (Fig. 9C). In addition, PSSC was significantly reduced in eggs treated with Sgo1 mRNA when eggs treated with both concentrations were combined together into one graph (Treated (20 or 80 ng/µl Sgo1 mRNA)) (Fig. 10A, 10D). Furthermore, the inventors found a consistent reduction in the percentage of eggs with PSSC when the inventors only compared untreated and treated eggs from the same donor by pairwise comparison (20 ng/µl alone, 80 ng/µl alone, and 20 or 80 ng/µl together) eggs (Fig. 8K, 9G, and 10G, respectively).

Taken together, this data demonstrates that supplementation of exogenous Sgo1 reduces PSSC in human eggs. Furthermore, this data show that supplementing eggs with Sgo1 efficiently reduces PSSC in human eggs, suggesting it as a therapeutic strategy to prevent the age-related increase in PSSC and resulting aneuploidy in human eggs and embryos. Therefore, supplementation of Sgo1 is expected to be a therapeutic strategy to prevent the age-related increase in aneuploidy in mammalian eggs, particularly in human oocytes. In particular, it can be used to increase the fraction of euploid eggs and is therefore expected to increase the success rates of IVF, to reduce miscarriages, and to prevent trisomies and monosomies, such as trisomy 21 (Down syndrome), trisomy 13 (Edwards syndrome) or trisomy 18 (Patau syndrome) and Turner syndrome.

### Discussion

As women get older, they often have problems with infertility (1, 3, 6, 13, 70). This decline in fertility is due to an age-related increase in aneuploidy in eggs (3, 71-74). As a result, women over the age of 35 often fail to conceive and have an increased rate of miscarriages. In vitro fertilization (IVF) and related assisted reproductive technologies (ART, also known as assisted reproductive techniques) have helped to restore fertility in couples who are unable to conceive. However, higher rates of aneuploidy in eggs of women with ageing have led to lower IVF success rates. To the knowledge of the inventors, there is currently no treatment commercially available to prevent age-related aneuploidy in eggs. The inventors found supplementing aged oocytes with the cohesin protection factor, Shugoshin (Sgo), which is greatly reduced in aged oocytes, can reduce PSSC. Exogenous Shugoshin can protect sister chromatid cohesion by efficiently restoring PP2A and Rec8 to the centromeres.

Among the two mammalian Sgo proteins, exogenous Sgo1 showed a greater reduction in PSSC than Sgo2 in aged mouse oocytes, as demonstrated herein. While Sgo2 supplementation partially rescued PSSC, Sgo1 reduced PSSC to levels observed in young mice. Sgo1 localizes to a larger area around the pericentromere than Sgo2, helping to recruit additional PP2A at meiosis I to protect Rec8. Furthermore, mammalian Sgo1 and Sgo2 proteins form distinct complexes with different PP2A subunits and complexes and may help to protect distinct centromeric PP2A populations pools within aged oocytes (65). Consistent with this, it was found that co-expression of Sgo1 and Sgo2 in aged oocytes further showed PP2A and Rec8 restoration at metaphase II stage (Fig. 6C, 6D).

The inventors also found that Sgo1-PP2A-mediated protection of centromeric cohesion requires centromeric transcription. A decrease in centromeric transcription in aged oocytes was found (Fig. 2H, 2I). MajSat RNAs alone could not rescue PSSC in aged oocytes, but could in triptolide-treated oocytes. This suggests that MajSat RNAs alone cannot restore the levels of Sgo1 protein lost from aged oocytes. Consistent with this, the inventors found that supplementation of satellite RNA (such as major satellite RNA, e.g., MajSat RNA) together with Sgo1 showed improved PSSC rescue in aged eggs.

Loss of chromosome cohesion with age in human oocytes has been associated with increased aneuploidy. Sgo2 protein is particularly lost from pericentromere bridges in aged human oocytes (22). In this invention it was found that there is a decrease in Sgo1 protein in human oocytes from women of advanced reproductive age, as well as in aged mouse oocytes. Exogenous Sgo1 localized to human pericentromeres, decreased inter kinetochore distances on MII chromosomes, and reduced PSSC in human eggs. Introducing Sgo1 and/or Sgo2 can therefore be utilized as a therapeutic approach to stabilize chromosome architecture, which is expected to greatly help prevent the age-related increase in chromosome segregation errors and aneuploidy in aged human oocytes and eggs (Fig. 8C).

### Materials and Methods

### Oocyte and follicle collection and culture

All mice were maintained in a specific pathogen-free environment and handled according to the guidelines of the Max Planck Institute for Multidisciplinary Sciences Animal House in accordance with international animal welfare standards (guidelines and recommendations of the Federation of Laboratory Animal Science Associations) and the German Animal Welfare Act (TSchG). 8-12-week-old 129S6 mice were sacrificed by cervical dislocation and ovaries were collected in M2 medium supplemented with 750 µM dibutyryl cyclic AMP (dbcAMP) to maintain oocytes in prophase I arrest. Aged mice used in the study were 60-65 weeks old. Isolation and selection of GV stage oocytes with a central nucleus was performed by puncturing mouse ovaries with a hypodermic needle and culturing them in M2 with dbcAMP media covered with paraffin oil (NidaCon #NO-400K). For meiotic maturation experiments, oocytes were washed out of dbcAMP. For follicle isolation, 10-12-day old (C57BL x CBA) F1 females were used as previously described (51) with some modifications. Ovaries were collected in MEM-alpha HEPES GlutaMAX (ThermoFisher, 42360024) supplemented with 0.1x penicillin-G/streptomycin (Gibco). Ovaries were briefly dissociated with 2 mg/ml Collagenase IV (Thermo Fisher Scientific 17104019) for 5 min and then mechanically pipetted several times. Follicles were washed free of collagenase and selected to be approximately 100 µm in size and surrounded by uniform layers of granulosa cells. Follicles were microinjected with a maximum volume of 6pl of siRNA. After microinjection, follicles were plated on collagen-coated inserts (Corning, 3491/3493) with MEM-alpha GlutaMAX (ThermoFisher, 41090028) supplemented with 5% fetal bovine serum (FBS) (Gibco, 16000044), 1x insulin-transferrin-selenium (ITS) (ThermoFisher Scientific, 41400045), 0.01 µg/ml bovine follicle stimulating hormone, oFSH (National Hormone and Peptide Programme, NDDK-oFSH-20) and 0.1x penicillin-G/streptomycin (Gibco) at 37°C/5% CO2. The medium in the wells surrounding the inserts was changed every 3 days. After 9-10 days of in vitro culture, GV oocytes were harvested in modified M2 medium containing 10% FBS instead of 4 mg/ml bovine serum albumin (BSA).

### Preparation and culture of human oocytes

The use of unfertilized human oocytes in this study was approved by the Medical Association of Lower Saxony under reference 15/2016. All human oocytes used in this study were obtained from Clinic for Reproductive Medicine, Brno, Czech Republic., Bourn Hall Fertility Clinic; Bourn, United Kingdom, and Kinderwunschzentrum Göttingen; Göttingen, Germany. The use of unfertilized human oocytes in this study was approved by the UK's National Research Ethics Service under the REC reference 11/EE/0346 (IRAS project ID 84952) and by German authorities under the Ärztekammer Niedersachsen (Lower Saxony). Only immature oocytes unsuitable for ICSI treatment were used in this study. All patients gave informed consent to donate their surplus oocytes for use in this study. Oocytes were collected and incubated in G-MOPS plus medium (Vitrolife, 10130) under paraffin oil (NidaCon #NO-400K) at 37°C within 1 to 4 hours after ovarian retrieval, as previously described (87). Vitrified human oocytes were thawed using Kitazato Oocyte/Embryo Thawing Media (VT602). Cryotop^{®} strips containing vitrified oocytes were immersed in thawing solution (TS) for 1 minute. Oocytes were washed out of TS by transfer to Diluent Solution (DS) in a well of Repro Plate-K1 (Ref. 83006/83007) and incubated in DS for 3 minutes. Oocytes were transferred to Wash Solution 1 (WS1) for 5 minutes, followed by a final wash with Wash Solution 2 (WS2, G-MOPS plus). In the final step, oocytes were incubated in G-MOPS plus (Vitrolife) medium in a 37°C incubator for 2 hours to allow complete recovery. Oocytes were incubated in G-MOPS plus media supplemented with DMSO/triptolide for approximately 24 hours until they reached the MII stage.

### Expression constructs and in vitro mRNA synthesis

To generate mRNA constructs for time-lapse imaging in oocytes, coding sequences of mSgo1, mSgo2, hSgo1, MajSat satellites were amplified from oocyte cDNA libraries generated using the SensiFAST cDNA synthesis kit (Bioline: BIO-65053). Primers used for plasmid construction are listed in the list of primers below. First, pENTR/dTOPO-Sgo1, pENTR/dTOPO-Sgo1N61I, pENTR/dTOPO-hSgo1, pENTR/dTOPO-Sgo2, pENTR/dTOPO-H2B, pENTR/dTOPO-Map4-MTBD, were generated for subcloning into pGEMHE-mClover3-gateway destination vectors using primers P1-10 and Invitrogen^{™}pENTR^{™}/TEV/D-TOPO^{™} Cloning Kit (Thermo Fisher Scientific, #K252520). Amplified sequences were subcloned into pGEMHE-mClover3 and pGEMHE-SNAP Gateway destination vectors using the Gateway^{™} LR Clonase^{™} II Enzyme Mix (Thermo Fisher Scientific, 11791020) to generate pGEMHE-mSgo1-mClover, pGEMHE-mSgo1N61I-mClover, pGEMHE-Sgo2-mClover, pGEMHE-hSgo1-mClover, pGEMHE-SNAP-H2B and pGEMHE-Map4-MTBD-mCover, constructs. To generate the hSgo1 mRNA construct, the vsv-Sgo1-EGFP plasmid was used (Addgene, 108494). Using restriction digestion with XhoI and NotI, the hSgo1 construct was subcloned into the pGEM-HE vector backbone for mRNA synthesis. All mRNAs were prepared using the HiScribe T7 ARCA mRNA Kit (NEB# E2065S) according to the manufacturer's instructions. Synthesized mRNAs were quantified using a Qubit RNA HS Assay Kit (Thermo Fisher Scientific # Q32852).

The *Mus musculus* major satellite repeat (MajSat) targeting sequence, pGEM-T-MajSat, was a generous gift from Dr Maria-Elena Torres-Padilla. Unlabeled MajSat sense and antisense RNA of the 234bp repeat (SEQ ID NO: 6) of the mouse major satellite were transcribed using the MaxiScript kit (AM1312, Ambion). The pGEMT-MajSat plasmid was linearized with ScaI and NgoMIV followed by in vitro transcription of the products using either T7 (for sense) or SP6 (for antisense) promoter. For generation of fluorescently labelled mouse satellite RNA, linearized plasmid DNA or PCR products containing a double-stranded T7 promoter region upstream of the target sequence were used. The HighYield T7 Cy3 RNA Labelling Kit (RNT-101-CY3) was used to label amplified sequences with Cy3-labelled UTP (UTP-X-Cy3, 35%), followed by DNA removal using Turbo^{™}DNAse (ThermoFisher). Purification of cy3-uridine-labelled RNA was performed using Monarch^{®} RNA Cleanup Kit (NEB) (T2040L). Double-stranded RNA (dsRNA) was produced by bidirectional amplification using the MAXIscript^{™} SP6/T7 Transcription Kit (Thermofisher, AM1322) and the T7 and SP6 promoters. Equal moles of complementary single-stranded RNA were mixed in Tris buffer (pH 7.4) 10 mM, EDTA 0.1 mM for 4 h at 37C. Residual ssRNA and proteins in the mixture were removed by RNase mixture (RNase A 1ug/ml and RNase T1 1ug/ml) and incubated for 30 min at 37°C and Proteinase K (140 ug/ml) for 15 min at 37°C, respectively. The purified RNA was stored at -80°C.

### Microinjection of mouse oocytes and immature follicles

Oocytes and follicles were microinjected according to previously described protocols (8). Oocytes and follicles were loaded onto an injection tray assembled by superimposing two coverslips separated by one (for GV oocytes) and two (for follicles) layers of 100 µm double-sided adhesive tape. For GV oocytes, 4 pl of the mRNAs were injected at the following concentrations mSgo2-mClover3 at 65 ng/µl, mSgo1-mClover3 at 75 ng/µl, hSgo1-mClover3 at 70 ng/µl, mMajSat-cy3 at 40 ng/µl, mMajSat dsRNA at 20 ng/µl, SNAP-H2B at 5 ng/µl, mClover3-Map4-MTBD at 90 ng/µl. Oocytes were allowed to express the mRNAs for 3 hours before dbcAMP was washed out to allow meiotic maturation and imaging. Human oocytes were then microinjected with either 175 fmol or 547 fmol of hSgo1-mClover3 mRNA. Oocytes were cultured in G-MOPS Plus for 24 to 72 hours until the oocytes had formed the first polar body and subsequently fixed 6 to 9 hours after polar body extrusion.

Knockdown with short interfering RNAs was performed by microinjecting short interfering RNAs (siRNAs) into 10-12-day old F1 females according to previously established protocols (52). The following siRNAs were used for Sgo1 knockdown. In this study, 2 different RNAi mixtures were used against *Mm*Sgol1 (NM_028232) from Qiagen (5'-CAGCAAATTGCTGTTGAAGAA-3', SI01416674; 5'-CAGGAGAATTGCAGAGTACAA-3', SI01416667), see SEQ ID NO: 27 and 28. AllStars Negative Control (1027281, Qiagen) was used as a control. Mouse follicles were microinjected with 6 pl of siRNAs at a needle concentration of 2 mM as described previously.

### Imaging of oocytes using confocal microscopy

For confocal imaging, live mouse oocytes were imaged in pre-warmed M2 media and fixed oocytes in PBS with paraffin oil (NidaCon #NO-400K) in a 35 mm glass bottom dish with a #1 coverslip (MatTek). Imaging with triptolide supplemented media was performed in Nunc^{™} Cell-Culture Treated Multidishes (Thermo Fisher, 176740) without oil. Confocal images were acquired using LSM 800/900/980 laser scanning confocal microscopes (Zeiss) equipped with an environmental incubator box and a 40× C-Apochromat 1.2 NA water immersion objective. Automatic 3D tracking using Autofocus screen was implemented for time-lapse imaging with a temporal resolution of 5 min (77,78). Images were acquired under identical imaging conditions on the same microscope for the control and experimental groups. Airyscan images were acquired using LSM800/900/980 confocal laser scanning microscopes equipped with an Airyscan module (Zeiss) and processed in ZEN (Zeiss) after acquisition. Care was taken to ensure that imaging conditions, including laser power, pixel dwell time and detector gain, did not cause phototoxicity, photobleaching or saturation.

To inhibit transcription in oocytes, Triptolide (T3652, MilliporeSigma), and α-amanitin (A2263, MilliporeSigma) were used at 20 µM for 2 hours and 20 µg/ml for 8 hours, respectively. Isolated oocytes were divided into control and treatment groups and then incubated with pre-warmed M2 media containing dbcAMP, inhibitor (according to Table 1), SiRDNA (as chromosomal marker, 1:5000 dilution, 50 nM). For time-lapse imaging of oocytes, DNA was stained by incubating oocytes with M2 media containing 150 nM SiR-DNA (SpyroChrome: SC007).

### Immunofluorescence

To obtain mouse metaphase I and II spindles, oocytes were incubated in dbcAMP-free medium for ~6 hours and ~16 hours after release, respectively. Oocytes were fixed for 30 minutes at 37°C in 100 mM HEPES (pH 7) (titrated with KOH), 50 mM EGTA (pH 7) (titrated with KOH), 10 mM MgSO4, 2% formaldehyde (MeOH-free), 0.2% Triton X-100, according to previously published methods (79, 80). Fixed oocytes were extracted with phosphate-buffered saline (PBS) with 0.5% Triton X-100 (PBT) overnight at 4°C and blocked in PBT with 5% BSA (PBT-BSA) for 6 hours at room temperature. All primary antibody incubations were performed overnight at 4°C in 5% PBT-BSA. After washing 3 times for 10 minutes with 5% PBT-BSA, oocytes were incubated with the secondary antibody mixture (20 µg/ml) for 1 hour at room temperature. Hoechst 33342 (Molecular Probes) was used at 100 µM for DNA staining with the final secondary antibody. Primary antibodies used for immunofluorescence analysis were as follows Human anti-centromere (15234, Antibodies Incorporated), rat anti-α-tubulin (sc-53030, Santa Cruz), rabbit anti-GFP (Ab6556, Abcam), mouse anti-GM130 (610822, BD Biosciences), rabbit anti-RNA pol II-pS2 (AB5095, Abcam), mouse anti-RNA pol II-pS5 (ab5408, Abcam), mouse anti-RNA pol II antibody (39097, Active Motif), rabbit anti-CENP-A(C51A7, Cell Signaling), rabbit anti-Sgol2 (orb499806, Biorbyt), rabbit anti-Sgol1 (polyclonal, donated by Dr. Tomoya Kitajima), mouse anti-Sgol1 (HOO151648-M01, Abnova), rabbit anti-PP2A catalytic subunit (05-421, Merck Millipore), rabbit anti-PP2A catalytic subunit (2038S, Cell Signaling), rabbit anti-Rec8 (produced by Cambridge Research Biochemicals, antigen protein corresponding to Mus musculus Rec8 (aa25-286)), rabbit anti-Sgo1 (produced by Cambridge Research Biochemicals, antigen peptide corresponding to Mus musculus Sgo1 (aa 2-19)). Alexa Fluor 405, 488, 568 or 647 conjugated anti-human IgG, donkey IgG, mouse IgG, rabbit IgG or rat IgG (Thermo Fisher Scientific) were used as secondary antibodies.

### Chromosome spreads

To obtain chromosome spreads of metaphase I and metaphase II chromosomes, oocytes were incubated in dbcAMP-free medium for ~6 hours and ~16 hours after release, respectively. To remove the zona, oocytes were washed briefly with 3-4 drops of Tyrode's acid (T1788, Sigma) followed by washing with M2 medium. Oocytes were swollen by incubation with a hypotonic solution (50% FBS, 50% ddH2O) on agarose gels (0.5% agarose in PBS) for 14 min at 37°C. Oocytes were fixed by dropping 12 µL of fixative (1% PFA, 0.15% Triton X-100, 3 mM DTT) into each well of a 15-well slide (096041505, MP Biomedicals) in a humidified box. After overnight chromosome attachment to the slides, the slides were air dried and washed twice sequentially for 5 minutes each with spreading wash solution (0.08% Kodak Photoflo in 1L PBS, 74257, Laborimpex), PBS and immune wash solution (0.2% BSA, 0.1% Tween-20 in PBS). In case of CENPA antibody (cell signaling C51A7) lambda phosphatase treatment was performed before for 45 minutes at 30°C (3µL lambda Phosphatase, 30µL 10x PMP, 30µL MnCl2 stock, 237µl water). Spreads were blocked with 10% FBS, 2.5% BSA and 0.1% Tween-20 in PBS for 30 minutes at room temperature and antibody incubations were performed in PBT-BSA with 10 mg/ml primary antibodies or 20 mg/ml secondary antibodies for 1 hour at room temperature. Hoechst 33342 (Molecular Probes) was used at 100 µM for DNA staining with the final secondary antibody. Slides were washed three times with a cleaning solution (0.01% Tween-20 in PBS), mounted with a 1:1 PBS-glycerol solution and sealed with nail varnish.

### Detection of nascent transcription in oocytes

The Click-iT^{™} RNA Alexa Fluor^{™} 488 Imaging Kit (ThermoFisher, C10329) was used. Isolated oocytes were transferred and washed in M2 (+dbcAMP) media supplemented with 5-ethynyluridine (EU, 1 mM) and incubated at 37°C for 3 hours. Oocytes were fixed as previously described, followed by extraction with PBT. The oocytes were then transferred to Click Reaction solution (172 µL Click Reaction Buffer, 8 µL CuSO4, 0.48 µL Alexa Fluo, 20 µL 1:10 diluted React Additive and 2 µL 10% TritonX-100) in the Terasaki plate and incubated for 30 minutes at room temperature. The oocytes were then washed with Click Rinse (Click Rinse solution diluted 1:100 with 10% TritonX-100) and PBS-Triton 0.1%. After two washes with each solution, oocytes were incubated in PBS-Triton 0.1% supplemented with Hoechst (100 µM) for 30 min in the dark.

### Detection of satellite RNAs by RNA FISH

Detection of satellite transcripts was performed by RNA FISH using FISH probes against major and minor satellite repeats (PNAbio). The probes used in this study are Cy3-O-GCGAGGAAAACTGAAAAAGG (MajSat antisense), FAM-O-TTGCCATATTCCACGTCC (MajSat sense), Cy3-O_TCCCGTTTCCAACGAAT (MinSat antisense), FAM-O-ATTCGTTGGAAACGGGA (MinSat sense) (from PNAbio), see SEQ ID NOs: 29-32. For metaphase I and II oocyte chromosome spreads, slides were washed twice briefly with 0.1% Tween-20 in RNase-free PBS followed by RNase-free PBS for 5 minutes at room temperature. For controls, slides were treated with RNase solution (100 µg/mL) for 30 minutes at 37°C and washed twice for 5 minutes with RNase-free PBS. PNA probes were used at 500 nM in freshly prepared hybridisation solution (60% formamide, 0.5% blocking reagent (11096176001, Roche) in 20 mM Tris pH 7.4). Pre-warmed PNA probes were added to pre-warmed slides with chromosome spreads for 10 minutes at 85°C on a heating block. Slides were placed in a humidified chamber for 10 hours in the dark for probe hybridisation. Slides were sequentially washed in the dark with 50% (v/v) formamide in 2× SSC at 45°C for 15 minutes, 0.2× SSC at 63°C for 15 minutes, 2× SSC at 45°C for 5 minutes, and finally 2× SSC at room temperature for 5 minutes. For immunofluorescence, slides were washed in 1× PBS and blocked with 5% BSA (w/v) and 0.1% (v/v) Triton X-100 in 1× PBS for 1 hour at room temperature. Primary and secondary antibodies were incubated for 1 hour at room temperature. Slides were washed with RNase-free PBS before incubation with Hoechst solution for 1 hour at room temperature and washed three times with RNase-free PBS. 50% (v/v) glycerol-RNase-free PBS was used as mounting medium and sealed with coverslips (#1.5, VWR, 15165452) and nail varnish.

### Immunoblotting

For the detection of Sgo1 and RNA Pol II-pS2, 50 GV mouse oocytes were probed per lane. The harvested cells were washed briefly with PBS and equal numbers of oocytes from the control and experimental groups were snap-frozen in liquid nitrogen. For oocyte lysis, cells were snap frozen and thawed a total of 3 times before mixing 5 µl NuPAGE^{™} LDS Sample Buffer (NP0007, ThermoFisher Scientific) supplemented with 100 mM DTT and 15 µl cell lysate and boiled at 95°C for 5 minutes. Protein samples, together with a pre-stained protein ladder (26619, ThermoFisher Scientific), were resolved on a 10-well NuPAGE 4-12% bis-tris protein gel, 1.0 mm thick (NP0322BOX, ThermoFisher Scientific) with 1X NuPAGE MOPS running buffer (NP0001, Thermo Fisher Scientific). Proteins were transferred to a 0.45 µm PVDF membrane (LC2005, Thermo Fisher Scientific) using 1X NuPAGE transfer buffer (NP0006, Thermo Fisher Scientific) containing 20% methanol at 110 V for 1.5 hours. Blocking and antibody incubations were performed in PBS containing 5% skimmed milk and 0.05% Tween-20. The following primary antibodies were used: rabbit anti-Sgo1 (polyclonal, donated by Dr Tomoya Kitajima, 1 µg/ml), rabbit anti-RNA Pol II-pS2 (AB5095, Abcam, 2 µg/ml) and rabbit anti-Ddb1 (ab109027, Abcam, 0.04 µg/ml). Primary antibodies were incubated overnight at 4°C. Anti-rabbit HRP (Abcam, #ab205718) was used as secondary antibody and incubated for 1 hour at room temperature. Immunoblots were developed using SuperSignal West Femto Maximum Sensitivity Substrate Femto (34094, ThermoFisher Scientific) and imaged using an Amersham Imager 600 (GE Healthcare).

### Image analysis and quantification

Images were analysed in Fiji (https://imagej.nih.gov/ijl), Imaris (Bitplane), Arivis Vision4D or ZEN blue (ZEISS). The exported data were further processed in Microsoft Excel and GraphPad Prism 9. In particular, a Gaussian filter was applied for noise reduction and maximum intensity projection of z-stacks (Zen, Zeiss). Airyscan processing was used when images were acquired using an Airyscan detector (Zen, Zeiss). Illustrations were created using Adobe Illustrator. Automated spot detection was used to identify kinetochore centers based on local maxima. For some cells, chromosome segmentation was performed in Imaris 9.3 and Imaris 10.0 using scripts written in Matlab R2018b and R2021a. Sister kinetochores were paired using filaments to generate seeds for individual chromosomes. Manual corrections were made where necessary. Inter-sister kinetochore distances were obtained by manual pairwise measurements between detected spots. PSSC was recorded when single chromatids with a single kinetochore were observed. Intensity measurements of RNA and proteins on chromosome spreads were performed using Fiji and Imaris (Bitplane). For centromeric proteins, automated spot detection was used to identify kinetochore centers based on local maxima and mean intensities were measured at the kinetochore. For proteins localized in an extended region around kinetochores, the region of interest was segmented in Fiji and a threshold was applied to mask the pericentromere. After thresholding, a binary mask was generated and multiplied by the original region of interest. The background was drawn manually and the mean background intensity was subtracted. Relative fluorescence intensity was calculated by normalization to the mean of the control cells.

Quantification of Rec8 and PP2A at the pericentromere in metaphase II eggs was performed using Arivis Vision4D. The machine learning segmenter was used to draw objects around the pericentromere and the background was drawn manually. After background subtraction, the mean intensity was normalized to the mean of control cells and relative intensity was plotted using GraphPad Prism 9. For quantification of Sgo1 levels in human oocytes, data were first processed with Huygens deconvolution for both Sgo1 and CREST channels to minimise noise. The same deconvolution parameters were used for both control and experimental data sets (maximum iteration 40, acuity 36.05). Total Sgo1 intensity on chromosomes was measured in Imaris after background subtraction and normalized to the mean of control cells.

### Statistical analysis

Standard deviation was used for error bars in box plots. Statistical significance based on unpaired, two-tailed Student's t-test (for comparison of two groups) was calculated in GraphPad Prism 9. All data were obtained from at least two independent experiments. P values are indicated with * sign (ns = not significant, *p≤0.05, **p≤0.01, ***p≤0.001, ****p≤0.0001). Statistical analyses were performed with GraphPad. Statistical significance was calculated using unpaired two-tailed Student's t-test (for absolute values) and two-tailed Fisher's exact test (for categorical values). All box plots show median (horizontal black line), mean (small black squares), 25th and 75th percentiles (boxes), 10th and 90th percentiles (whiskers), percentiles (whiskers), and the 1st and 99th percentiles (circles). We tested the total number of intact sister chromatids against the total number of PSSC errors between two conditions, as indicated in each Figure. Each experimental condition reported in this study includes at least two technical replicates, with most having 3 replicates. Donated human oocyte material is limited in quantity and therefore constrained sample size.

### Expression constructs and in vitro mRNA synthesis

To generate the hSgo1 mRNA construct, the vsv-Sgo1-EGFP plasmid was used (Addgene, 108494). Using restriction digestion with XhoI and NotI, the hSgo1 construct was subcloned into the pGEM-HE vector backbone for mRNA synthesis. All mRNAs were prepared using the HiScribe T7 ARCA mRNA Kit (NEB, E2065S) according to the manufacturer's instructions. Synthesized mRNAs were quantified using a Qubit RNA HS Assay Kit (Thermo Fisher Scientific, Q32852). All RNAs were analyzed by gel electrophoresis before injection. Briefly, RNA was incubated at 70°C for 3 min in formamide loading buffer (Thermo-Fisher:R0641) and run alongside RiboRuler HR RNA ladder (Thermo-Fisher: SM1821). Gels were visualized with SYBR-safe stain (ThermoFisher, S33102).

### Preparation and culture of human oocytes

The use of unfertilized human oocytes in this study was approved by the UK's National Research Ethics Service under the REC reference 11/EE/0346 (IRAS project ID 84952). All human oocytes used in these experiments were obtained from Bourn Hall Fertility Clinic; Bourn, United Kingdom. Only immature oocytes unsuitable for ICSI treatment were used in this study. All patients gave informed consent to donate their surplus oocytes for use in this study. Oocytes were collected and incubated in G-MOPS plus medium (Vitrolife, 10130) and G-IVF plus medium (Vitrolife, 10134) under paraffin oil (NidaCon, NO-400K) at 37°C within 1 to 4 hours after ovarian retrieval, as previously described (87). Oocytes were then microinjected with 4 pl of either 20 or 80 ng/µl of hSgo1-mClover3 mRNA. Oocytes microinjected with 20 or 80 ng/µl of hSgo1-mClover3 mRNA were cultured in G-MOPS Plus or G-IVF Plus, respectively, for 24 to 72 hours until the formation of a polar body, indicating maturation to metaphase II. Eggs were fixed 6 to 9 hours after polar body extrusion and resuspended in PBS supplemented with 0.02% sodium azide until preparation for immunofluorescence.

### Immunofluorescence

Human oocytes were fixed for 30 minutes at 37°C in 100 mM HEPES (pH 7) (titrated with KOH), 50 mM EGTA (pH 7) (titrated with KOH), 10 mM MgSO4, 2% formaldehyde (MeOH-free), 0.2% Triton X-100, according to previously published methods (79). Fixed oocytes were extracted with phosphate-buffered saline (PBS) with 0.5% Triton X-100 (PBT) and blocked in PBT with 5% BSA (PBT-BSA) overnight at 4°C. All primary antibody incubations were performed overnight at 4°C in 5% PBT-BSA. After washing 3 times for 10 minutes with 5% PBT-BSA, oocytes were incubated with the secondary antibody mixture (20 µg/ml) for 1 hour at room temperature. Hoechst 33342 (Molecular Probes) was used at 100 µM for DNA staining with the final secondary antibody. Primary antibodies used for immunofluorescence analysis were as follows: human anti-centromere (15234, Antibodies Incorporated), mouse anti-Sgol1 (HOO151648-M01, Abnova), and rabbit anti-PP2A catalytic subunit (2038S, Cell Signaling). Alexa Fluor 405, 488, 568, or 647 conjugated anti-human IgG, donkey IgG, mouse IgG, and rabbit IgG (Thermo Fisher Scientific) were used as secondary antibodies.

### Microscopy

Confocal images of fixed immunofluorescence-labeled human eggs were acquired on LSM-900/980 confocal laser scanning microscopes equipped with a 40×C-Apochromat 1.2 NA water immersion objective. Super-resolution images were acquired on LSM900/980 microscopes equipped with an Airyscan module (Zeiss) and processed in ZEN (Zeiss) after acquisition.

Images were acquired under identical imaging conditions on the same microscope for the control and experimental groups.

Image analysis and quantification Images were analyzed in Imaris (Bitplane), or ZEN blue (ZEISS). The exported data were further processed in Microsoft Excel and GraphPad Prism 9. In particular, a Gaussian filter was applied for noise reduction and maximum intensity projection of z-stacks (Zen, Zeiss). Airyscan processing was used when images were acquired using an Airyscan detector (Zen, Zeiss). Illustrations were created using Adobe Illustrator. Automated spot detection was used to identify kinetochore centers based on local maxima. For some cells, chromosome segmentation was performed in Imaris 10.0.1 using scripts written in Matlab R2021a. Sister kinetochores were paired using filaments to generate seeds for individual chromosomes. Manual corrections were made where necessary. Manual pairwise measurements obtained inter-kinetochore distances between detected spots. PSSC was recorded when single chromatids with a single kinetochore were observed with no chromosome signal being present between two sister chromatids.

### List of protein sequences

| **SEQ ID NO** | **Name** | **Species** | **Sequence** |
|---|---|---|---|
| 1 | Shugoshin 1 ("Sgo1") | *Homo sapiens* | AKERCLKKSFQDSLEDIKKRMKEKRNKNLAEIGKRRSFIAAPCQIITNTSTLLK NYQDNNKMLVLALENEKSKVKEAQDIILQLRKECYYLTCQLYALKGKLTSQQT VEPAQNQEICSSGMDPNSDDSSRNLFVKDLPQIPLEETELPGQGESFQIEDQI PTIPQDTLGVDFDSGEAKSTDNVLPRTVSVRSSLKKHCNSICQFDSLDDFETS HLAGKSFEFERVGFLDPLVNMHIPENVQHNACQWSKDQVNLSPKLIQPGTFT KTKEDILESKSEQTKSKQRDTQERKREEKRKANRRKSKRMSKYKENKSENKK TVPQKKMHKSVSSNDAYNFNLEEGVHLTPFRQKVSNDSNREENNESEVSLCE SSGSGDDSDDLYLPTCKYIQNPTSNSDRPVTRPLAKRALKYTDEKETEGSKPT KTPTTTPPETQQSPHLSLKDITNVSLYPVVKIRRLSLSPKKNKASPAVALPKRR CTASVNYKEPTLASKLRRGDPFTDLCFLNSPIFKQKKDLRRSKKSMKQIQ |
| 2 | Shugoshin 2 ("Sgo2") | *Homo sapiens* | MECPVMETGSLFTSGIKRHLKDKRISKTTKLNVSLASKIKTKILNNSSIFKISLK HNNRALAQALSREKENSRRITTEKMLLQKEVEKLNFENTFLRLKLNNLNKKLID IEALMNNNLITAIEMSSLSEFHQSSFLLSASKKKRISKQCKLMRLPFARVPLTSN DDEDEDKEKMQCDNNIKSKTLPDIPSSGSTTQPLSTQDNSEVLFLKENNQNV YGLDDSEHISSIVDVPPRESHSHSDQSSKTSLMSEMRNAQSIGRRWEKPSPS NVTERKKRGSSWESNNLSADTPCATVLDKQHISSPELNCNNEINGHTNETNT EMQRNKQDLPGLSSESAREPNAECMNQIEDNDDFQLQKTVYDADMDLTASE VSKIVTVSTGIKKKSNKKTNEHGMKTFRKVKDSSSEKKRERSKRQFKNSSDVD IGEKIENRTERSDVLDGKRGAEDPGFIFNNEQLAQMNEQLAQVNELKKMTLQ |
| | | | TGFEQGDRENVLCNKKEKRITNEQEETYSLSQSSGKFHQESKFDKGQNSLTC NKSKASRQTFVIHKLEKDNLLPNQKDKVTIYENLDVTNEFHTANLSTKDNGNL CDYGTHNILDLKKYVTDIQPSEQNESNINKLRKKVNRKTEIISGMNHMYEDN DKDVVHGLKKGNFFFKTQEDKEPISENIEVSKELQIPALSTRDNENQCDYRTQ NVLGLQKQITNMYPVQQNESKVNKKLRQKVNRKTEIISEVNHLDNDKSIEYTV KSHSLFLTQKDKEIIPGNLEDPSEFETPALSTKDSGNLYDSEIQNVLGVKHGHD MQPACQNDSKIGKKPRLNVCQKSEIIPETNQIYENDNKGVHDLEKDNFFSLTP KDKETISENLQVTNEFQTVDLLIKDNGNLCDYDTQNILELKKYVTDRKSAEQN ESKINKLRNKVNWKTEIISEMNQIYEDNDKDAHVQESYTKDLDFKVNKSKQK LECQDIINKHYMEVNSNEKESCDQILDSYKVVKKRKKESSCKAKNILTKAKNKL ASQLTESSQTSISLESDLKHITSEADSDPGNPVELCKTQKQSTTTLNKKDLPFV EEIKEGECQVKKVNKMTSKSKKRKTSIDPSPESHEVMERILDSVQGKSTVSEQ ADKENNLENEKMVKNKPDFYTKAFRSLSEIHSPNIQDSSFDSVREGLVPLSVS SGKNVIIKENFALECSPAFQVSDDEHEKMNKMKFKVNRRTQKSGIGDRPLQD LSNTSFVSNNTAESENKSEDLSSERTSRRRRCTPFYFKEPSLRDKMRR |
| 4 | Shugoshin 1 ("Sgo1") | *Mus musculus* | MAKERCQKRSFQDTLEDIKNRMKEKRNKNLAGIGKRKSFIVAPGQVPTNTAT LLRYYQDNNRLLVLALENEKSKVREAQDVILQLRKECYYLTCQLYALKEKLTSR QSEETTQNWKGRPSDVVSSIDNTTRDLSGKSLQQIAVEETDCPYQTTEPSPA VTPETQGCDFDSGKVESTDEVLPRTISIRRHLRKDFSNISHSTTLEDCKASPRV AQSLEVKGSRCREVTVTLHRLENVCLWNKDQISLCSRLINPAKITETEVILSSK PEQIESKHKRARKRRAEQRRTKQRCKSKSSLRSKGNKNKDKQGLPPTTLDGG IGSCDAYDFNLKGTVHPTPFRQKMNNGCNKETDSSNSEVSDLECSTSEDESD DLYLPPSKRLRDYRESERAVTRPRSKRGLQYPDGKERKEVLPSTAPTGIPPET QESPRCSLKDVTNILQCPRVKIRKPSLPPKRREDSPAVALTKRRCSTIKSYKEP TLASKLRRGDPFTDLCFLNSPIFKQKRGMRCPKRRTKQTQ |
| 5 | Shugoshin 2 ("Sgo2") | *Mus musculus* | MEYPGIKVDTVTSGIQRRVKGRIAKTNLNVSLASKIKAKILNNSSIFKISLKHNN RALARALSKEKENSRRITTEKMQLQKEVEKLNFENTFLRLKLNTLNKKLVEIES HVSNDLLTAIEISSLSEFHQGSFLLSATKKQRNSKQCKPAHLPYARVLLTSEND DDDGADDKWQTKCNNRTISKTSPDSTSSVSRQPSSLHQCNLKAFPPKEDNQ KTCGSGHLEHTSSVDILPNESHSDQSPKSSLSEMKTAPSPSLRREKLSHGNVT MRKKCVSSTPDILYVTDLDHQPTSSPGSNWNNEIHGHTNETSNNTQRNAEC FLDLPSESSSEPDAKRMELVQKNTDSFHFQKTVYDAADMELTATDIGKIVAVS KSKKNQNKKKADCRKETFRKVKGASSDKKRESSKRECKDGSEVGAEEEADAA RAERGAGVLDGRGDSEEPNCISSTEQPSQVNTQKKRTLQNSSDQENIQNTKR RQTYTTDEQEETNPFSRHSVKFLQDGKFDLCQKTLHHNLSKPSRQTFVIRKSE KDNLFPNQEDKDTISENLEVTNEFHIDDLSIEANENVCDHETQTMLDLKKSVS AQQNQTKINKTKQKINRRTKIISVMSQVYEDNDKDIHVLEKDNFPFHTQANK ETTSGNLESSKEFESPLLFTRDNGSLRDCKTQNVLDLHKQIPDLYPDRNESQI |
| | | | SKIPRQKVNRKTEVISGVKCFSNDQGVHCSEKDKSLLLQKDKDFPGTLKDLSE FDTPAFCNKDSAKSCDYKSEMLLGLKKHDPNMQPACQDDSKAGKKLRQKVN RKTEIISKITQIHENDRGSTHDSLNKKLCQKVNISKIISQMNQIYETINEDGNG FKSSIKDCEDIKSCDFGEINSNKKENYDPIQDPCTLVKKTKRKGSCKAGSSLAG AKNRCGLQLTDSSQVQSVPLDSGLRHHPNEADSGPGEQTNLPKMQKQSAGR SLGDAFSVSLGKEGSRPAKAVSKMTPKSKKRKLPLGCSPETHGTVEITPNTDL AKAVDSQQTEKENYLEKEKIAKRKPDFCTKVLKPLSETCSSNIKNSSLDSMCKS SLPLSISSRKTLMLEESSSLESTCIFQVGDAAHEKITTGTRNPHHRTQKSTPGS RTSLVLVDTSSVSDTNPANPENESEGQSSHPMRRKRQCVPLNLTEPSLRSKM RR |

### List of Primers

| **SEQ ID NO** | **Gene name** | **Primer direction** | **Sequence** |
|---|---|---|---|
| 15 | MmSgo2 | Forward primer | 5'-CACCATGGAGTACCCAGGGATAAAAGTT-3' |
| 16 | MmSgo2 | Reverse primer | 5'-CACCATGGGACAAACACCAAA-3' |
| 17 | MmSgo1 | Forward primer | 5'-CACCATGGCTAAGGAAAGGTGTCAGAA-3' |
| 18 | MmSgo1 | Reverse primer | 5'-TTACTGTGTTTGCTTGGTTCTTCTTTTAGGACA-3' |
| 19 | HsSgo1 | Forward primer | 5'-GCCAAGGAAAGATGCCTGAA-3' |
| 20 | HsSgo1 | Reverse primer | 5'-TTGTATTTGTTTCATACTTTTTT-3' |
| 21 | MmSgo1-N61I | Forward primer | 5'-AGATAACATCAGGTTGTTAGTC-3' |
| 22 | MmSgo1-N61I | Forward primer | 5'-AACCTGATGTTATCTTGGTAATATC-3' |
| 23 | MmSgo1-N61I | Reverse primer | 5'-CTAACTCACATTAATTGCGTTG-3' |
| 24 | MmSgo1-N61I | Reverse primer | 5'-ATTAATGTGAGTTAGCTCACTC-3' |
| 25 | dsRNA-control | Forward primer | 5'-AACATGTGAGCAAAAGGCCAG-3' |
| 26 | dsRNA-control | Reverse primer | 5'-CCCAGCTTGGAGCGAACGAC-3' |

### List of RNAs used

| SEQ ID NO | **Name** | **Species** | **Sequence** |
|---|---|---|---|
| 3 | Human alpha satellite RNA sequenc e | *Homo sapiens* | **Original SEQ:** |
| | | | CAUUCUCAGAAACUUCUUUGUGAUGUGUCCAUUCAACUCACAGAGUUG AACCUUUCUUUUGAUAGAGCAGUUUUGAAACACUCUUUUUGUAGAAUC UGCAAGUGGAUAUUUGGAGCGCUUUGAGGCCUAUGGUGGAAAAGGAAA UAUCUUCACAUAAAAACUAGACAGAAG |
| | | | **in sequence listing:** |
| | | | cattctcagaaacttctttgtgatgtgtccattcaactcacagagttgaacctttcttttgatagagc agttttgaaacactctttttgtagaatctgcaagtggatatttggagcgctttgaggcctatggtgg aaaaggaaatatcttcacataaaaactagacagaag |
| 6 | Mouse major satellite RNA sequenc e | *Mus musculus* | **Original seq:** |
| | | | GGACCUGGAAUAUGGCGAGAAAACUGAAAAUCACGGAAAAUGAGAAAUACA CACUUUAGGACGUGAAAUAUGGCGAGGAAAACUGAAAAAGGUGGAAAAUUU AGAAAUGUCCACUGUAGGACGUGGAAUAUGGCAAGAAAACUGAAAAUCAUG GAAAAUGAGAAACAUCCACUUGACGACUUGAAAAAUGACGAAAUCACUAAA AAACGUGAAAAAUGAGAAAUGCACACUGAA |
| | | | **in sequence listing:** |
| | | | ggacctggaatatggcgagaaaactgaaaatcacggaaaatgagaaatacacactttaggacgtg aaatatggcgaggaaaactgaaaaaggtggaaaatttagaaatgtccactgtaggacgtggaatat ggcaagaaaactgaaaatcatggaaaatgagaaacatccacttgacgacttgaaaaatgacgaaa tcactaaaaaacgtgaaaaatgagaaatgcacactgaa |
| 7 | Mouse minor satellite RNA | *Mus musculus* | **Original sequence:** |
| | | | GGAAAAUGAUAAAAACCACACUUUAGAACAUAUUAGAUGAGUGAGUUACAC UGAAAACACAUUCGUUGGAAACCGGCAUUGUAGACAGUGUAUAUCAUGAGU AUACUAGAAACAU |
| | | | **in sequence listing:** |
| | | | ggaaaatgataaaaaccacactttagaacatattagatgagtgagttacactgaaaacacattcgttg gaaaccggcattgtagacagtgtatatcatgagtatactagaaacat |
| 27 | RNAi 1 against *Mm*Sgol 1 | synthetic | 5'-CAGCAAATTGCTGTTGAAGAA-3' |
| 28 | RNAi 2 against *Mm*Sgol 1 | synthetic | 5'-CAGGAGAATTGCAGAGTACAA-3' |

### Relevant genomic DNA sequences:

| **SEQ ID NO** | **Name** | **Species** | **Sequence** |
|---|---|---|---|
| 8 | Shugoshin 1 ("Sgo1") cDNA | *Homo sapiens* | GCCAAGGAAAGATGCCTGAAAAAGTCCTTTCAAGATAGTCTTGAAGAC ATAAAGAAGCGAATGAAAGAGAAAAGGAATAAAAACTTGGCAGAGATT GGCAAACGCAGGTCTTTTATAGCTGCACCATGCCAAATAATCACCAACA CTTCTACACTGCTGAAAAATTACCAAGACAACAACAAAATGTTAGTTTT AGCTTTGGAAAATGAAAAATCCAAAGTGAAAGAAGCCCAAGATATCATC CTACAGCTGAGAAAAGAATGTTACTATCTCACATGTCAGCTATATGCAT TGAAAGGAAAACTTACATCACAACAAACAGTAGAACCTGCACAAAATCA AGAAATATGTTCCTCTGGAATGGACCCCAATAGTGATGACAGCTCCAG AAATTTATTTGTGAAGGATTTACCGCAAATTCCTCTTGAAGAAACTGAA CTTCCAGGACAAGGAGAATCATTTCAAATAGAAGATCAGATACCTACTA TTCCTCAAGACACACTGGGAGTTGATTTTGATTCAGGTGAAGCTAAGT CTACTGATAATGTCTTACCTAGAACTGTATCTGTTCGTAGCAGTTTAAA GAAACATTGTAACAGTATATGTCAGTTTGATAGCTTGGATGATTTTGAA ACCAGTCATTTGGCAGGGAAGTCTTTTGAATTCGAAAGAGTTGGATTT TTAGACCCACTAGTAAACATGCACATACCTGAAAATGTACAACACAATG CTTGTCAATGGAGCAAGGACCAAGTTAACTTATCACCAAAGCTGATTCA GCCAGGAACGTTTACTAAAACAAAAGAAGACATTTTAGAATCTAAATCT GAACAAACTAAAAGTAAGCAAAGAGATACACAAGAAAGAAAAAGAGAA GAGAAAAGAAAAGCTAACAGGAGAAAATCAAAACGTATGTCAAAATATA AAGAGAATAAAAGCGAAAATAAAAAAACTGTTCCCCAAAAAAAAATGCA CAAATCTGTCAGTTCCAATGATGCTTACAATTTTAATTTGGAAGAGGGT GTTCATCTTACTCCTTTCCGACAAAAAGTGAGCAATGACTCTAATAGAG AAGAAAACAACGAGTCTGAAGTGAGCCTCTGTGAATCAAGTGGTTCAG GAGATGATTCCGATGACCTCTATTTGCCCACTTGCAAGTACATTCAGAA TCCCACGAGCAATTCAGATAGACCAGTCACCAGGCCTCTAGCTAAAAGA GCACTGAAATACACAGATGAAAAAGAGACGGAGGGTTCTAAGCCAACA AAAACTCCTACCACTACACCACCTGAAACTCAGCAGTCACCTCATCTTA GCCTGAAGGATATCACCAATGTCTCCTTGTATCCTGTTGTGAAAATCAG AAGACTTTCTCTTTCTCCAAAAAAGAATAAAGCAAGCCCAGCAGTGGCT CTGCCTAAACGTAGGTGCACAGCCAGCGTGAACTATAAGGAGCCCACC CTCGCTTCGAAACTGAGAAGAGGGGACCCTTTTACAGATTTGTGTTTTT TGAATTCTCCTATTTTCAAGCAGAAAAAGGATTTGAGACGTTCTAAAAA AAGTATGAAACAAATACAA |
| 9 | Shugoshin 2 ("Sgo2") cDNA | *Homo sapiens* | ATGGAGTGCCCAGTGATGGAAACTGGCTCACTTTTTACCTCAGGAATTAAG AGACATTTGAAAGACAAAAGAATTTCAAAGACTACTAAGTTGAATGTTTCTC TTGCTTCAAAAATAAAAACAAAAATACTAAATAATTCTTCTATTTTCAAAAT ATCTTTAAAGCACAACAACAGGGCATTAGCTCAGGCTCTTAGTAGAGAAAA AGAGAATTCTCGAAGAATTACAACTGAAAAGATGCTATTGCAAAAAGAAGT AGAGAAACTGAATTTTGAGAACACATTTCTTCGCCTAAAGCTAAATAACTTG AATAAGAAGCTTATAGACATAGAAGCTCTCATGAACAATAACTTGATAACT GCAATTGAAATGAGCAGTCTTTCTGAGTTCCATCAGAGTTCCTTTCTACTG TCAGCTAGCAAGAAGAAACGAATTAGTAAACAGTGCAAGTTGATGCGTCTT CCATTTGCAAGGGTTCCATTAACTTCAAATGATGATGAAGATGAAGATAAA GAGAAAATGCAGTGTGACAACAATATTAAATCAAAGACATTACCTGATATT CCCTCTTCAGGATCAACAACACAACCTTTATCAACTCAGGATAATTCGGAAG TGTTATTTCTTAAAGAAAATAATCAAAATGTATATGGTTTAGATGATTCAGA ACATATTTCTTCTATAGTTGATGTACCTCCCAGAGAAAGCCATTCCCACTCA GACCAAAGTTCTAAGACTTCTCTAATGAGTGAGATGAGAAACGCCCAGTCT ATTGGCCGCAGATGGGAGAAACCATCTCCTAGTAATGTGACTGAAAGGAAG AAGCGTGGGTCATCTTGGGAATCAAATAATCTTTCTGCAGACACTCCCTGT GCAACAGTTTTAGATAAACAACACATTTCAAGTCCAGAATTAAATTGCAATA ATGAGATAAATGGTCATACTAATGAAACAAATACTGAAATGCAAAGAAATA AACAGGATCTTCCTGGCTTATCTTCTGAGTCTGCCAGAGAACCTAATGCAG AGTGCATGAATCAAATTGAGGATAATGATGACTTTCAATTGCAGAAAACTG TGTATGATGCTGACATGGATTTAACTGCTAGTGAAGTCAGCAAAATTGTCA CAGTCTCAACAGGCATTAAAAAGAAAAGTAATAAAAAAACAAATGAACATG GAATGAAAACTTTCAGAAAAGTGAAAGATTCCAGCTCTGAAAAAAAGAGAG AAAGATCAAAGAGACAGTTTAAAAATAGTTCAGATGTCGATATTGGGGAAA AGATTGAAAACAGGACAGAAAGATCTGATGTCCTGGATGGCAAAAGGGGT GCAGAAGATCCCGGTTTTATTTTCAATAATGAACAGCTGGCTCAGATGAAT GAACAGCTGGCTCAGGTGAATGAACTAAAGAAAATGACCCTTCAAACTGGC TTTGAACAAGGTGACAGAGAAAATGTACTGTGTAATAAAAAGGAGAAAAGA ATAACAAATGAGCAAGAGGAAACATACTCTTTATCCCAAAGTTCAGGTAAA TTTCACCAGGAGAGTAAATTTGATAAGGGTCAGAATTCCCTAACTTGTAAT AAAAGTAAAGCTTCTAGACAGACATTTGTGATTCACAAATTAGAAAAAGAT AACTTACTCCCAAACCAAAAGGATAAAGTAACCATTTATGAAAACCTAGACG TCACAAATGAATTTCACACAGCCAATCTTTCCACCAAAGATAATGGAAATTT ATGTGATTATGGGACCCACAATATATTGGATTTGAAAAAGTATGTCACTGA TATTCAACCCTCAGAGCAAAATGAATCAAACATTAATAAGCTTAGAAAGAAA GTAAACCGGAAGACAGAAATAATTTCTGGAATGAACCACATGTATGAGGAT AATGATAAAGATGTGGTGCATGGCCTAAAAAAAGGTAATTTTTTTTTCAAA |
| | | | ACCCAAGAGGATAAAGAACCTATCTCTGAAAACATAGAAGTTTCCAAAGAG CTTCAAATCCCAGCTCTTTCTACTAGAGATAATGAAAATCAATGTGACTATA GGACCCAGAATGTGTTGGGTTTGCAAAAGCAGATCACCAATATGTACCCCG TTCAGCAAAATGAATCAAAAGTTAATAAGAAGCTTAGGCAGAAAGTAAATC GGAAGACAGAAATAATTTCTGAAGTGAATCATTTAGATAATGACAAAAGTA TAGAATACACAGTTAAAAGTCACTCACTCTTTTTAACGCAAAAAGATAAGGA AATCATCCCTGGAAACCTAGAAGACCCAAGTGAGTTTGAAACACCTGCTCT TTCTACCAAAGATAGTGGAAACCTGTATGATTCTGAGATTCAAAATGTTTT GGGGGTGAAACATGGCCATGATATGCAACCTGCTTGTCAAAATGATTCAAA AATAGGTAAGAAGCCTAGACTAAATGTATGTCAAAAGTCAGAAATAATTCC TGAAACCAACCAAATATATGAGAATGATAACAAAGGTGTACATGACCTAGA AAAAGATAACTTCTTCTCTCTAACCCCAAAGGATAAAGAAACAATTTCTGAA AATCTACAAGTCACAAATGAATTTCAAACAGTTGATCTTCTCATCAAAGATA ATGGAAATTTATGTGATTATGACACCCAGAATATATTGGAGTTGAAAAAGT ATGTTACTGATAGGAAATCTGCTGAGCAAAATGAATCAAAAATAAATAAGC TCAGGAATAAAGTGAATTGGAAGACAGAAATAATTTCTGAAATGAACCAGA TATATGAGGATAATGATAAAGATGCACATGTCCAAGAAAGCTATACAAAAG ATCTTGATTTTAAAGTAAATAAATCTAAACAAAAACTTGAATGCCAAGACAT TATCAATAAACACTATATGGAAGTCAACAGTAATGAAAAGGAAAGTTGTGA TCAAATTTTAGATTCCTACAAAGTAGTTAAAAAACGTAAGAAAGAATCATCA TGCAAGGCAAAGAACATTTTGACAAAAGCTAAGAACAAACTTGCTTCACAG TTAACAGAATCTTCACAGACATCTATCTCCTTAGAATCTGATTTAAAACATA TTACTAGTGAAGCAGATTCTGATCCAGGAAACCCAGTTGAACTATGTAAGA CTCAGAAGCAAAGCACTACCACTTTGAATAAAAAAGATCTCCCTTTTGTGG AAGAAATAAAAGAAGGAGAGTGTCAGGTTAAAAAGGTAAATAAAATGACAT CTAAGTCAAAGAAAAGGAAGACCTCCATAGATCCTTCTCCAGAGAGCCATG AAGTAATGGAAAGAATACTTGACAGCGTTCAGGGAAAGTCTACTGTATCTG AACAAGCTGATAAGGAAAACAATTTGGAGAATGAGAAAATGGTCAAAAATA AGCCAGACTTTTACACAAAGGCATTTAGATCTTTGTCTGAGATACATTCACC TAACATACAAGATTCTTCCTTTGACAGTGTTCGTGAAGGTTTAGTACCTTT GAGCGTTTCTTCTGGTAAAAATGTGATAATAAAAGAAAATTTTGCCTTGGA GTGCTCCCCAGCCTTTCAAGTAAGTGATGATGAGCATGAGAAGATGAACAA GATGAAATTTAAAGTCAACCGGAGAACCCAAAAATCAGGAATAGGTGATAG ACCATTACAGGACTTGTCAAATACCAGTTTTGTTTCAAATAACACTGCTGAA TCTGAAAATAAGTCAGAAGATCTATCTTCAGAACGGACAAGCAGAAGAAGA AGGTGTACTCCTTTCTATTTTAAAGAGCCAAGCCTCAGAGACAAGATGAGA AGATGA |
| 10 | Shugoshin 1 ("Sgo1") cDNA | *Mus musculus* | ATGGCTAAGGAAAGGTGTCAGAAAAGGTCCTTTCAAGATACCCTTGAAGAC ATTAAGAATCGAATGAAAGAAAAAAGGAATAAAAATTTGGCGGGGATTGGG AAACGCAAGTCCTTTATTGTTGCACCGGGCCAAGTACCCACTAACACTGCT ACACTACTGAGATATTACCAAGATAACAACAGGTTGTTAGTCTTGGCTTTG GAAAATGAGAAATCCAAAGTGAGAGAAGCACAGGATGTCATCCTGCAACTG AGAAAAGAATGCTACTACCTTACTTGTCAGCTGTATGCATTGAAAGAGAAG CTAACTTCCCGACAAAGTGAAGAAACTACTCAGAACTGGAAAGGACGTCCC TCAGACGTGGTCTCCAGCATTGACAATACGACCAGGGACTTGTCAGGGAAG TCCTTACAGCAAATTGCTGTTGAAGAAACTGATTGTCCTTACCAAACCACAG AACCAAGTCCTGCTGTTACTCCAGAGACACAGGGTTGCGATTTTGATTCAG GTAAAGTTGAGTCTACTGATGAAGTCTTACCCAGAACTATATCTATCCGTC GCCATTTAAGGAAAGATTTTAGTAATATAAGCCACTCCACGACTTTGGAGG ATTGTAAAGCCAGTCCAAGAGTGGCACAGTCTCTGGAAGTTAAAGGAAGTA GATGTAGAGAAGTAACCGTAACCCTGCACAGACTTGAAAATGTTTGTCTGT GGAACAAAGACCAAATTAGCTTATGTTCTAGACTGATTAACCCAGCAAAGA TTACTGAAACAGAAGTCATTTTATCATCTAAACCTGAACAAATAGAAAGCAA GCATAAACGTGCACGAAAAAGAAGAGCAGAGCAAAGAAGAACCAAGCAGAG ATGCAAATCAAAATCCTCATTGAGGAGTAAGGGGAACAAAAACAAAGATAA GCAGGGTTTACCCCCTACTACACTGGATGGAGGTATTGGTTCCTGTGATGC TTACGATTTTAATCTAAAAGGGACGGTCCACCCCACCCCTTTCCGACAAAAA ATGAACAATGGCTGCAACAAAGAAACGGATAGCAGCAACTCAGAAGTGAGT GACCTCGAATGCAGTACCTCTGAGGATGAGTCTGATGACCTCTACCTGCCT CCCTCCAAGCGCTTGCGAGACTACAGAGAGTCAGAGAGAGCAGTTACCAGG CCTCGGTCTAAAAGAGGACTTCAGTACCCAGATGGGAAAGAGAGGAAGGA GGTGCTGCCATCTACAGCTCCTACTGGTATCCCACCTGAGACTCAAGAGTC ACCTCGTTGTAGCCTAAAGGATGTCACCAATATCCTGCAGTGTCCTAGAGT GAAGATCAGGAAGCCTTCTCTGCCTCCAAAGCGGCGTGAAGACAGCCCAGC AGTGGCTCTGACTAAACGCAGGTGTAGCACCATCAAAAGCTATAAAGAGCC AACACTCGCTTCAAAGCTAAGAAGAGGGGACCCTTTCACGGACTTGTGTTT CTTGAATTCTCCTATTTTCAAGCAGAAAAGGGGTATGAGATGTCCTAAAAG AAGAACCAAGCAAACACAGTAA |
| 11 | Shugoshin 2 ("Sgo2") cDNA | *Mus musculus* | ATGGAGTACCCAGGGATAAAAGTTGACACTGTTACCTCTGGAATTCAGAGA CGAGTGAAGGGCAGAATTGCAAAGACAAATTTGAATGTTTCTCTTGCTTCA AAGATCAAAGCAAAAATATTAAACAATTCTTCTATTTTCAAGATCTCTCTAA AGCACAACAACAGAGCATTAGCGCGGGCCCTTAGTAAAGAGAAAGAGAATT CTCGAAGAATTACTACCGAAAAGATGCAATTACAGAAAGAAGTAGAGAAAC TGAATTTTGAGAATACCTTTCTTCGCTTAAAGTTAAATACCTTGAATAAGAA GCTTGTAGAAATAGAATCGCATGTGAGCAATGATTTGTTAACTGCAATTGA |
| | | | AATAAGCAGTCTTTCTGAGTTCCACCAAGGTTCTTTTCTCCTGTCAGCTACC AAGAAACAAAGGAACAGTAAGCAGTGCAAGCCTGCGCATCTTCCATATGCA AGAGTTCTGTTAACTTCAGAAAATGATGATGATGATGGTGCTGATGATAAA TGGCAGACAAAGTGTAACAACAGAACTATATCAAAGACCTCACCTGATAGT ACCTCTTCAGTATCAAGACAACCTTCATCCTTACATCAGTGCAATTTGAAAG CATTCCCTCCTAAAGAAGATAATCAGAAGACATGTGGGTCAGGTCATTTAG AACATACTTCAAGTGTTGATATACTTCCTAATGAGAGCCACTCAGATCAAAG TCCTAAGAGTTCTCTGAGTGAGATGAAAACTGCTCCATCTCCCAGCCTCAG AAGGGAAAAATTATCACATGGTAATGTGACTATGAGGAAGAAGTGTGTGTC TTCAACTCCAGACATTCTGTATGTGACAGATTTAGATCACCAACCAACTTCA AGTCCAGGATCAAATTGGAATAATGAGATACATGGTCATACTAATGAAACC AGCAATAACACGCAAAGAAATGCCGAGTGTTTTCTTGACTTACCTTCTGAG TCTTCCAGTGAGCCTGACGCAAAGCGCATGGAGCTAGTGCAGAAGAACACC GATAGCTTTCACTTCCAGAAAACTGTATATGATGCCGCTGATATGGAGTTA ACTGCTACTGACATAGGCAAGATTGTAGCAGTTTCAAAAAGCAAGAAAAAT CAAAATAAGAAAAAGGCAGACTGTAGAAAGGAGACTTTCAGAAAAGTGAAA GGTGCAAGCTCTGATAAAAAGAGAGAAAGCTCAAAGAGAGAATGTAAAGAT GGTTCAGAAGTAGGTGCTGAGGAAGAGGCTGATGCAGCCAGAGCAGAAAG AGGCGCTGGTGTCCTGGATGGCAGAGGGGATTCAGAAGAGCCAAACTGCA TTTCCAGTACTGAGCAGCCATCTCAGGTAAACACGCAAAAGAAAAGAACCC TCCAGAACAGCTCAGATCAGGAGAACATTCAAAATACGAAGAGGAGGCAAA CATATACGACAGATGAGCAAGAGGAAACAAACCCTTTCTCCAGACATTCAG TCAAATTTCTTCAAGATGGTAAATTTGATCTGTGTCAGAAAACCCTACATCA TAATTTAAGTAAGCCTTCTCGACAGACATTTGTGATTCGTAAGTCAGAAAA AGATAACTTATTTCCAAATCAAGAAGATAAAGACACCATTTCTGAAAACCTA GAAGTTACAAATGAATTTCATATAGATGATCTTTCCATCGAAGCTAATGAAA ATGTATGTGACCATGAGACTCAGACAATGTTGGACTTGAAAAAGTCTGTCA GTGCTCAACAAAATCAAACAAAAATAAATAAGACTAAGCAGAAAATAAATCG AAGGACAAAAATAATTTCTGTCATGAGCCAAGTATATGAGGACAATGATAA AGATATTCACGTCCTAGAAAAAGACAACTTTCCCTTTCATACCCAAGCAAAT AAAGAAACCACCAGTGGAAACCTAGAAAGTTCAAAAGAATTTGAATCACCT CTTCTTTTCACAAGAGACAACGGAAGCTTACGTGACTGTAAGACCCAGAAT GTTCTGGATCTGCACAAGCAAATTCCTGATCTATACCCTGATCGGAATGAG TCCCAGATTAGCAAAATCCCTAGGCAAAAAGTAAATCGCAAGACAGAAGTA ATTTCTGGAGTGAAATGTTTTAGTAATGACCAAGGTGTTCATTGCTCAGAA AAGGATAAGTCTTTGTTACTACAAAAGGATAAAGACTTCCCAGGAACTTTA AAAGACTTAAGTGAGTTTGATACGCCTGCTTTTTGTAACAAAGATAGTGCA AAGTCGTGTGATTATAAGTCTGAAATGCTCTTGGGGTTGAAAAAACATGAC |
| | | | CCTAATATGCAACCTGCTTGTCAAGATGATTCAAAAGCAGGTAAGAAACTT AGACAAAAGGTAAATCGAAAAACAGAAATAATTTCTAAAATCACCCAAATAC ATGAAAATGATAGAGGAAGTACACATGACTCATTAAATAAGAAGCTCTGTC AGAAGGTTAATATATCAAAAATCATTTCTCAAATGAACCAAATATATGAGAC TATTAATGAAGATGGAAATGGCTTTAAAAGCTCTATCAAAGATTGCGAAGA TATTAAAAGTTGTGACTTTGGGGAAATCAACAGTAATAAAAAGGAAAATTA TGATCCAATTCAAGATCCTTGCACACTGGTTAAAAAAACAAAGAGAAAGGG ATCATGTAAAGCAGGGAGCAGTTTGGCAGGAGCTAAGAACAGGTGTGGTT TGCAGTTAACAGACTCTTCCCAGGTACAGTCTGTCCCCTTAGACTCTGGCT TAAGACACCATCCAAACGAAGCAGATTCTGGTCCTGGAGAGCAGACTAACC TGCCAAAGATGCAGAAACAAAGCGCTGGGAGGTCACTGGGAGATGCTTTCT CTGTGAGTCTGGGAAAAGAAGGAAGCCGCCCAGCCAAAGCAGTTAGTAAAA TGACACCCAAATCAAAGAAGAGAAAGCTCCCTCTCGGTTGTTCTCCTGAAA CCCACGGGACGGTGGAGATAACACCCAACACTGACCTCGCTAAGGCTGTTG ACTCCCAACAGACTGAGAAGGAGAACTATTTGGAGAAGGAGAAAATTGCCA AGAGGAAGCCAGATTTTTGTACAAAGGTGTTGAAACCTTTATCTGAGACAT GTTCATCTAACATAAAGAATTCTTCCTTGGACAGTATGTGTAAGAGTTCGC TACCTTTGAGTATTTCTTCTAGAAAAACCCTGATGCTGGAAGAAAGTTCTTC CCTGGAGAGTACATGCATCTTTCAAGTAGGTGATGCCGCTCATGAGAAGAT AACGACAGGCACACGTAATCCCCACCACAGGACACAGAAGTCGACACCGGG TAGCAGAACGTCCCTGGTCTTGGTGGATACCAGTTCTGTTTCAGATACCAA CCCTGCTAACCCCGAGAATGAGTCAGAAGGGCAGTCTTCACACCCAATGAG AAGGAAAAGACAGTGCGTCCCTCTCAACCTGACAGAGCCAAGCCTTAGAAG CAAGATGAGGAGA |
| 12 | Human alpha-satellite DNA sequence | *Homo sapiens* | CATTCTCAGAAACTTCTTTGTGATGTGTCCATTCAACTCACAGAGTTGAACC TTTCTTTTGATAGAGCAGTTTTGAAACACTCTTTTTGTAGAATCTGCAAGT GGATATTTGGAGCGCTTTGAGGCCTATGGTGGAAAAGGAAATATCTTCACA TAAAAACTAGACAGAAG |
| 13 | Mouse major satellite DNA sequence | *Mus musculus* | GGACCTGGAATATGGCGAGAAAACTGAAAATCACGGAAAATGAGAAATACA CACTTTAGGACGTGAAATATGGCGAGGAAAACTGAAAAAGGTGGAAAATTT AGAAATGTCCACTGTAGGACGTGGAATATGGCAAGAAAACTGAAAATCATG GAAAATGAGAAACATCCACTTGACGACTTGAAAAATGACGAAATCACTAAA AAACGTGAAAAATGAGAAATGCACACTGAA |
| 14 | Mouse minor satellite DNA | *Mus musculus* | GGAAAATGATAAAAACCACACTTTAGAACATATTAGATGAGTGAGTTACAC TGAAAACACATTCGTTGGAAACCGGCATTGTAGACAGTGTATATCATGAGT ATACTAGAAACAT |

### Labelled probes used

| | | | |
|---|---|---|---|
| 29 | MajSat antisense, 5'-Cy3-O-labelled | synthetic | Cy3-O-GCGAGGAAAACTGAAAAAGG |
| 30 | MajSat sense, 5'-FAM-O-labelled | synthetic | FAM-O-TTGCCATATTCCACGTCC |
| 31 | MinSat antisense, 5'-Cy3-O-labelled | synthetic | Cy3-O_TCCCGTTTCCAACGAAT |
| 32 | MinSat sense, 5'-FAM-O-labelled | synthetic | FAM-O-ATTCGTTGGAAACGGGA |

### List of References:

1. Hunt, P. A. The control of mammalian female meiosis: Factors that influence chromosome segregation. J. Assist. Reprod. Genet. 15, 246-252 (1998).
2. Gruhn, J. R. et al. Chromosome errors in human eggs shape natural fertility over reproductive life span. Science (80-. ). 365, 1466-1469 (2019).
3. Webster, A. & Schuh, M. Mechanisms of Aneuploidy in Human Eggs. Trends Cell Biol. 27, 55-68 (2017).
4. Herbert, M., Kalleas, D., Cooney, D., Lamb, M. & Lister, L. Aneuploid Oocytes and Trisomy Births. Cold Spring Harb Perspect Biol. 7, a017970 (2015).
5. Mihajlović, A. I., Haverfield, J. & FitzHarris, G. Distinct classes of lagging chromosome underpin age-related oocyte aneuploidy in mouse. Dev. Cell 56, 2273-2283.e3 (2021).
6. Chiang, T., Schultz, R. M. & Lampson, M. A. Meiotic origins of maternal age-related aneuploidy. Biol. Reprod. 86, 1-7 (2012).
7. Jones, K. T. Meiosis in oocytes: Predisposition to aneuploidy and its increased incidence with age. Hum. Reprod. Update 14, 143-158 (2008).
8. Zielinska, A. P. et al. Meiotic Kinetochores Fragment into Multiple Lobes upon Cohesin Loss in Aging Eggs. Curr. Biol. 29, 3749-3765.e7 (2019).
9. Angell, R. First-meiotic-division nondisjunction in human oocytes. Am. J. Hum. Genet. 61, 23-32 (1997).
10. Yun, Y., Lane, S. I. R. & Jones, K. T. Premature dyad separation in meiosis II is the major segregation error with maternal age in mouse oocytes. Dev. 141, 199-208 (2014).
11. Revenkova, E., Herrmann, K., Adelfalk, C. & Jessberger, R. Oocyte cohesin expression restricted to predictyate stages provides full fertility and prevents aneuploidy. Curr. Biol. 20, 1529-1533 (2010).
12. Merriman, J. A., Jennings, P. C., Mclaughlin, E. A. & Jones, K. T. Effect of aging on superovulation efficiency, aneuploidy rates, and sister chromatid cohesion in mice aged up to 15 months. Biol. Reprod. 86, 1-6 (2012).
13. Wolstenholme, J. & Angell, R. R. Maternal age and trisomy - A unifying mechanism of formation. Chromosoma 109, 435-438 (2000).
14. Haering, C. H., Löwe, J., Hochwagen, A. & Nasmyth, K. Molecular Architecture of SMC Proteins and the Yeast Cohesin Complex C-terminal domains forming a head would be part of. Mol. Cell 9, 773-788 (2002).
15. Nasmyth, K. & Haering, C. H. Cohesin: Its Roles and Mechanisms. Annu. Rev. Genet. 43, 525-558 (2009).
16. Gligoris, T. & Löwe, J. Structural Insights into Ring Formation of Cohesin and Related Sm Complexes. Trends Cell Biol. 26, 680-693 (2016).
17. Tachibana-Konwalski, K. et al. Rec8-containing cohesin maintains bivalents without turnover during the growing phase of mouse oocytes. Genes Dev. 24, 2505-2516 (2010).
18. Burkhardt, S. et al. Chromosome Cohesion Established by Rec8-Cohesin in Fetal Oocytes is Maintained without Detectable Turnover in Oocytes Arrested for Months in Mice. Curr. Biol. 26, 678-685 (2016).
19. Lister, L. M. et al. Age-related meiotic segregation errors in mammalian oocytes are preceded by depletion of cohesin and Sgo2. Current Biology vol. 20 1511-1521 (2010).
20. Garcia-Cruz, R. et al. Dynamics of cohesin proteins REC8, STAG3, SMC1β and SMC3 are consistent with a role in sister chromatid cohesion during meiosis in human oocytes. Hum. Reprod. 25, 2316-2327 (2010).
21. Chiang, T., Duncan, F. E., Schindler, K., Schultz, R. M. & Michael, A. NIH Public Access. Curr. Biol. 20, 1522-1528 (2011).
22. Mihalas BP, Pieper GH, Aboelenain M, Munro L, Srsen V, Currie CE, Kelly DA, Hartshorne GM, Telfer EE, McAinsh AD, Anderson RA, Marston AL. Age-dependent loss of cohesion protection in human oocytes. Curr Biol. 34(1):117-131.e5, (2024).
23. Barone, S. et al. Chromosome missegregation in single human oocytes is related to the age and gene expression profile. Int. J. Mol. Sci. 21, (2020).
24. Thomas, C., Cavazza, T. & Schuh, M. Aneuploidy in human eggs: Contributions of the meiotic spindle. Biochem. Soc. Trans. 49, 107-118 (2021).
25. Nakajima, M. et al. The complete removal of cohesin from chromosome arms depends on separase. J. Cell Sci. 120, 4188-4196 (2007).
26. Kudo, N. R. et al. Role of cleavage by separase of the Rec8 kleisin subunit of cohesin during mammalian meiosis I. J. Cell Sci. 122, 2686-2698 (2009).
27. Lee, J., Iwai, T., Yokota, T. & Yamashita, M. Temporally and spatially selective loss of Rec8 protein from meiotic chromosomes during mammalian meiosis. J. Cell Sci. 116, 2781-2790 (2003).
28. Luo, S. & Tong, L. Structure and Function of the Separase-Securin Complex. 217-232.
29. Miyazaki, W. Y. & Orr-Weaver, T. L. Sister-Chromatid Cohesion in Mitosis and Meiosis. Annu. Rev. Genet. 28, 167-187 (2003).
30. Petronczki, M. et al. ' nage a ' Quatre: Un Me The Molecular Biology of Chromosome Segregation in Meiosis Heredity: A Historical Introduction. 112, 423-440 (2003).
31. Llano, E. et al. Shugoshin-2 is essential for the completion of meiosis but not for mitotic cell division in mice. Genes Dev. 22, 2400-2413 (2008).
32. Lee, J. et al. Unified mode of centromeric protection by shugoshin in mammalian oocytes and somatic cells. Nat. Cell Biol. 10, 42-52 (2008).
33. Clift, D. & Marston, A. L. The role of shugoshin in meiotic chromosome segregation. Cytogenet. Genome Res. 133, 234-242 (2011).
34. Kitajima, T. S. et al. Shugoshin collaborates with protein phosphatase 2A to protect cohesin. Nature 441, 46-52 (2006).
35. Tang, Z., Sun, Y., Harley, S. E., Zou, H. & Yu, H. Human Bub1 protects centromeric sister-chromatid cohesion through Shugoshin during mitosis. Proc. Natl. Acad. Sci. U. S. A. 101, 18012-18017 (2004).
36. McGuinness, B. E., Hirota, T., Kudo, N. R., Peters, J. M. & Nasmyth, K. Shugoshin prevents dissociation of cohesin from centromeres during mitosis in vertebrate cells. PLoS Biol. 3, 0433-0449 (2005).
37. Salic, A., Waters, J. C. & Mitchison, T. J. Vertebrate shugoshin links sister centromere cohesion and kinetochore microtubule stability in mitosis. Cell 118, 567-578 (2004).
38. Yin, S. et al. Shugoshin1 may play important roles in separation of homologous chromosomes and sister chromatids during mouse oocyte meiosis. PLoS One 3, 1-7 (2008).
39. Perea-Resa, C. & Blower, M. D. Centromere Biology: Transcription Goes on Stage. Mol. Cell Biol. 38, (2018).
40. Talbert, P. B. & Henikoff, S. Transcribing Centromeres: Noncoding RNAs and Kinetochore Assembly. Trends Genet. 34, 587-599 (2018).
41. Smurova, K. & Wulf, P. De. Centromere and Pericentromere Transcription: Roles and Regulation in Sickness and in Health. 9, 1-26 (2018).
42. Ferri, F., Bouzinba-Segard, H., Velasco, G., Hubé, F. & Francastel, C. Non-coding murine centromeric transcripts associate with and potentiate Aurora B kinase. Nucleic Acids Res. 37, 5071-5080 (2009).
43. Grenfell, A. W., Heald, R. & Strzelecka, M. Mitotic noncoding RNA processing promotes kinetochore and spindle assembly in Xenopus. J. Cell Biol. 214, 133-141 (2016).
44. Chan, F. L. et al. Active transcription and essential role of RNA polymerase II at the centromere during mitosis. Proc. Natl. Acad. Sci. U. S. A. 109, 1979-1984 (2012).
45. Bobkov, G. O. M., Gilbert, N. & Heun, P. Centromere transcription allows CENP-A to transit from chromatin association to stable incorporation. J. Cell Biol. 217, 1957-1972 (2018).
46. Chen, Y., Zhang, Q., Teng, Z. & Liu, H. Centromeric transcription maintains centromeric cohesion in human cells. J. Cell Biol. 220, (2021).
47. Hsieh, C., Xia, J. & Lin, H. MIWI prevents aneuploidy during meiosis by cleaving excess satellite RNA. EMBO J. 39, 1-26 (2020).
48. Yadav, R. P., Mäkelä, J. A., Hyssälä, H., Cisneros-Montalvo, S. & Kotaja, N. DICER regulates the expression of major satellite repeat transcripts and meiotic chromosome segregation during spermatogenesis. Nucleic Acids Res. 48, 7135-7153 (2020).
49. Liu, H., Qu, Q. & Rice, A. Mitotic Transcription Installs Sgo1 at Centromeres to Coordinate Chromosome Segregation Article Mitotic Transcription Installs Sgo1 at Centromeres to Coordinate Chromosome Segregation. Mol. Cell 59, 426-436 (2015).
50. Liu, H. Insights into centromeric transcription in mitosis. Transcription 7, 21-25 (2016).
51. Wu, T., Lane, S. I. R., Morgan, S. L., Tang, F. & Jones, K. T. Loss of centromeric RNA activates the spindle assembly checkpoint in mammalian female meiosis I. J. Cell Biol. 220, (2021).
52. Santhanam, B. et al. Live imaging RNAi screen reveals genes essential for meiosis in mammalian oocytes. Nature 524, 239-242 (2015).
53. Bensaude, O. Inhibiting eukaryotic transcription: Which compound to choose? How to evaluate its activity? Transcription 2, 103-108 (2011).
54. Baumann, C. et al. Helicase LSH/Hells regulates kinetochore function, histone H3/Thr3 phosphorylation and centromere transcription during oocyte meiosis. Nat. Commun. 11, (2020). 55. Komissarov, A. S., Gavrilova, E. V., Demin, S. J., Ishov, A. M. & Podgornaya, O. I. tandemly repeated DNA families in the mouse genome. BMC Genomics 12, 531 (2011).
56. Guenatri, M., Bailly, D., Maison, C. & Almouzni, G. Mouse centric and pericentric satellite repeats form distinct functional heterochromatin. J. Cell Biol. 166, 493-505 (2004).
57. Lica, L. M., Narayanswami, S. & Hamkalo, B. A. Mouse satellite DNA, centromere structure, and sister chromatid pairing. J. Cell Biol. 103, 1145-1151 (1986).
58. De La Fuente, R., Baumann, C. & Viveiros, M. M. ATRX contributes to epigenetic asymmetry and silencing of major satellite transcripts in the maternal genome of the mouse embryo. Dev. 142, 1806-1817 (2015).
59. Kerrebrock, A. W., Miyazaki, W. Y., Birnby, D. & Orr-Weaver, T. L. The Drosophila mei-S332 gene promotes sister-chromatid cohesion in meiosis following kinetochore differentiation. Genetics 130, 827-841 (1992).
60. Watanabe, Y., Kitajima, T. S., Hyman, T., Yanagida, M. & Hirano, T. Shugoshin protects cohesion complexes at centromeres. Philos. Trans. R. Soc. B Biol. Sci. 360, 515-521 (2005).
61. Rivera, T. & Losada, A. Shugoshin regulates cohesion by driving relocalization of PP2A in Xenopus extracts. Chromosoma 118, 223-233 (2009).
62. Katis, V. L. et al. Rec8 Phosphorylation by Casein Kinase 1 and Cdc7-Dbf4 Kinase Regulates Cohesin Cleavage by Separase during Meiosis. Dev. Cell 18, 397-409 (2010).
63. Rumpf, C. et al. Casein kinase 1 is required for efficient removal of Rec8 during meiosis I. Cell Cycle 9, 2657-2662 (2010).
64. Tang, Z. et al. PP2A Is Required for Centromeric Localization of Sgo1 and Proper Chromosome Segregation. Dev. Cell 10, 575-585 (2006).
65. Xu, Z. et al. Structure and Function of the PP2A-Shugoshin Interaction. Mol. Cell 35, 426-441 (2009).
66. Lister, L. M. et al. Age-related meiotic segregation errors in mammalian oocytes are preceded by depletion of cohesin and Sgo2. Curr. Biol. 20, 1511-1521 (2010).
67. Kerrebrock, A. W., Moore, D. P., Wu, J. S. & Orr-Weaver, T. L. Mei-S332, a drosophila protein required for sister-chromatid cohesion, can localize to meiotic centromere regions. Cell 83, 247- 256 (1995).
68. Moore, D. P., Page, A. W., Tang, T. T. L., Kerrebrock, A. W. & Orr-Weaver, T. L. The cohesion protein MEI-S332 localizes to condensed meiotic and mitotic centromeres until sister chromatid separate. J. Cell Biol. 140, 1003-1012 (1998).
69. Zielinska, A. P., Holubcova, Z., Blayney, M., Elder, K. & Schuh, M. Sister kinetochore splitting and precocious disintegration of bivalents could explain the maternal age effect. Elife 4, 1-19 (2015).
70. Charalambous, C., Webster, A. & Schuh, M. Aneuploidy in mammalian oocytes and the impact of maternal ageing. Nat. Rev. Mol. Cell Biol. 24, 27-44 (2023).
71. Hassold T & Hunt P. To err (meiotically) is human: the genesis of human aneuploidy. Nat. Rev. Genet. 2, 280-91 (2001).
72. Kuliev, A., Cieslak, J. & Verlinsky, Y. Frequency and distribution of chromosome abnormalities in human oocytes. Cytogenet. Genome Res. 111, 193-198 (2005).
73. Kuliev, A., Zlatopolsky, Z., Kirillova, I., Spivakova, J. & Cieslak Janzen, J. Meiosis errors in over 20,000 oocytes studied in the practice of preimplantation aneuploidy testing. Reprod. Biomed. Online 22, 2-8 (2011).
74. Wartosch, L. et al. Origins and mechanisms leading to aneuploidy in human eggs. Prenat. Diagn. 41, 620-630 (2021).
75. Dumont, M. et al. Human chromosome-specific aneuploidy is influenced by DNA -dependent centromeric features . EMBO J. 39, 1-21 (2020).
76. Santenard, A. et al. Heterochromatin formation in the mouse embryo requires critical residues of the histone variant H3.3. Nat. Cell Biol. 12, 853-862 (2010).
77. Rabut, G. & Ellenberg, J. Automatic real-time three-dimensional cell tracking by fluorescence microscopy. J. Microsc. 216, 131-137 (2004).
78. Politi, A. Z. et al. Quantitative mapping of fluorescently tagged cellular proteins using FCS-calibrated four-dimensional imaging. Nat. Protoc. 13, 1445-1464 (2018).
79. Schuh M, Ellenberg J. Self-organization of MTOCs replaces centrosome function during acentrosomal spindle assembly in live mouse oocytes. Cell. 2007 Aug 10;130(3):484-98.
80. Strickland L, von Dassow G, Ellenberg J, Foe V, Lenart P, Burgess D. Light microscopy of echinoderm embryos. Methods Cell Biol. 2004;74:371-409.
81. Ninomiya, K., Yamazaki, T., & Hirose, T. (2023). Satellite RNAs: emerging players in subnuclear architecture and gene regulation. The EMBO Journal, 42(18), e114331.
82. Rošić S, Köhler F, Erhardt S. Repetitive centromeric satellite RNA is essential for kinetochore formation and cell division. J Cell Biol. 2014 Nov 10;207(3):335-49. doi: 10.1083/jcb.201404097. Epub 2014 Nov 3. Erratum in: J Cell Biol. 2014 Dec 8;207(5):673.
83. Blower MD. Centromeric Transcription Regulates Aurora-B Localization and Activation. Cell Rep. 2016 May 24;15(8):1624-33.
84. Ling YH, Yuen KWY. Point centromere activity requires an optimal level of centromeric noncoding RNA. Proc Natl Acad Sci U S A. 2019 Mar 26;116(13):6270-6279.
85. Cerutti F, Gamba R, Mazzagatti A, Piras FM, Cappelletti E, Belloni E, Nergadze SG, Raimondi E, Giulotto E. The major horse satellite DNA family is associated with centromere competence. Mol Cytogenet. 2016 Apr 27;9:35.
86. Escudeiro A, Adega F, Robinson TJ, Heslop-Harrison JS, Chaves R. Conservation, Divergence, and Functions of Centromeric Satellite DNA Families in the Bovidae. Genome Biol Evol. 2019 Apr 1;11(4):1152-1165
87. Holubcová Z, Blayney M, Elder K, Schuh M. Human oocytes. Error-prone chromosome-mediated spindle assembly favors chromosome segregation defects in human oocytes. Science. 2015 Jun 5;348(6239):1143-7.

## Claims

1. An *in vitro* method of introducing (i) mRNA encoding Shugoshin 1 ("Sgo1") protein or (ii) Sgo1 protein into an oocyte or an egg,
wherein the method is used in assisted reproductive technology.

2. The method of claim 1, wherein the method does not include nuclear transfer.

3. The method of claims 1 or 2, wherein (i) the mRNA encoding Sgo1 protein or (ii) the Sgo1 protein reduces aneuploidy compared to an oocyte or an egg not having (i) mRNA encoding Sgo1 protein or (ii) Sgo1 protein introduced.

4. The method of any of the preceding claims, wherein (ii) Sgo1 protein binds PP2A protein and/or at the centromere, preferably assessed by microscopic co-localization.

5. The method of any of the preceding claims, wherein (a) mRNA encoding Sgo2 protein or (b) Sgo2 protein is additionally introduced into the oocyte or the egg, preferably wherein the mRNA encoding Sgo2 protein or the Sgo2 protein reduces aneuploidy compared to an oocyte or an egg not having introduced the mRNA encoding Sgo2 protein or Sgo2 protein.

6. The method of any of the preceding claims, wherein a satellite RNA is additionally introduced into the oocyte or egg; preferably wherein the satellite RNA is alpha satellite RNA.

7. The method of any of the preceding claims, where the oocyte or egg is a human oocyte or a human egg and wherein the human oocyte or the human egg is an aged oocyte or an aged egg, preferably wherein the aged oocyte or the aged egg is at least 35 years old.

8. The method of any preceding claims, wherein
(I) the oocyte or the egg prior to carrying out the method has at least a 5% increased probability of having a premature separation of sister chromatids (PSSC), preferably at least 10%, more preferably at least 15%, more preferably at least 20%, more preferably at least 25%, and even more preferably at least 35% compared to an average oocyte or average egg in reproductive prime age;
(II) the oocyte or the egg has at least 5% reduction in natural Sgo1 levels, preferably at least 10% reduction, more preferably at least 15% reduction, even more preferably at least 25% reduction and even more preferably at least 35% reduction compared to an average oocyte or average egg in reproductive prime age;
(III) the oocyte or the egg after having carried out the method has at least a 5% reduced probability of having a premature separation of sister chromatids (PSSC), preferably at least 10%, more preferably at least 15%, more preferably at least 20%, more preferably at least 25%, and even more preferably at least 35% compared to an oocyte or an egg not undergoing the method;
(IV) the oocyte or the egg prior to carrying out the method has at least 10% reduction in PP2A levels, preferably at least 20% reduction, more preferably at least 35% reduction and even more preferably at least 50% reduction compared to an average oocyte and average egg in reproductive prime age;
(V) the oocyte or the egg prior to carrying out the method has at least 15% reduction in Rec8 levels at centromeres, preferably at least 25% reduction, more preferably at least 35% reduction and even more preferably at least 55% reduction compared to an average oocyte or an average egg in reproductive prime age;
(VI) the oocyte or the egg prior to carrying out the method has reduced transcription during meiotic divisions, in particular centromeric transcription, by at least 10%, preferably 20%, more preferably at least 30% compared to an average oocyte or an average egg in reproductive prime age; and/or
(VII) the oocyte or the egg prior to carrying out the method has an increased interkinetochore distance by at least 10% when compared to an average oocyte or an average egg in reproductive prime age, more preferably by at least 20%, and the most preferably by at least 30% when compared to an average oocyte or an average egg in reproductive prime age;
preferably wherein the reproductive prime age for a human oocyte or a human egg is from 18 to 29 years, more preferably from 20 to 27 years, even more preferably from 23 to 25 years.

9. The method of any preceding claims, wherein the mRNA encoding the Sgo1 or the Sgo1 protein, preferably in combination with (A) the mRNA encoding Sgo2 or Sgo2 protein and/or (B) the satellite RNA, is/are introduced to the oocyte or the egg during prophase arrest, meiosis I, or meiosis II, in particular during the germinal vesicle stage, meiosis I prometaphase, meiosis I metaphase, meiosis I anaphase, meiosis I telophase, meiosis II metaphase, even more preferably between the germinal vesicle stage and anaphase I, and most preferably during germinal vesicle stage, meiosis I prometaphase or meiosis I metaphase.

10. An *in vitro* method of introducing (i) Sgo2 protein or (ii) mRNA encoding Sgo2 protein into an oocyte or an egg, preferably wherein the mRNA encoding Sgo2 protein or the mRNA Sgo2 protein is defined in claim 5, and/or wherein the oocyte or the egg is further defined in claims 7 or 8; and/or wherein a satellite RNA is additionally introduced into the oocyte or the egg as further defined in claim 6.

11. An (i) mRNA encoding Sgo1 protein or (ii) Sgo1 protein for use in a method of preventing infertility, miscarriage, implantation failure, or embryonic arrest, which are caused by or associated with a chromosomal aberration, wherein the mRNA encoding Sgo1 protein or Sgo1 protein is introduced into a zygote; preferably wherein the chromosomal aberration is aneuploidy, even more preferably the aneuploidy is a trisomy or a monosomy such as trisomy 21 (Down syndrome), trisomy 13 (Edwards syndrome), trisomy 18 (Patau syndrome), or Turner syndrome (X chromosome monosomy).

12. The (i) the mRNA encoding Sgo1 protein or (ii) Sgo1 protein for use in a method according to claim 11, wherein (i) said mRNA encoding Sgo1 protein or (ii) said Sgo1 protein is introduced in combination with (a) mRNA encoding Sgo2 protein or (b) Sgo2 protein; and/or wherein (i) said mRNA encoding Sgo1 protein or (ii) Sgo1 protein is introduced in combination with satellite RNA.

13. A pharmaceutical composition comprising an mRNA encoding Sgo1 protein or an Sgo1 protein, wherein the pharmaceutical composition additionally comprises (I) an mRNA encoding Sgo2 protein or a Sgo2 protein, (II) an mRNA encoding PP2A or PP2A protein and/or (III) a satellite RNA, and wherein the pharmaceutical composition is suitable for introduction into an oocyte, an egg or a zygote.

14. The pharmaceutical composition of claim 13, wherein
I. the Sgo1 and the Sgo2 form a complex after introduction into the oocyte or the egg;
II. the Sgo1 and the PP2A form a complex after introduction into the oocyte or the egg; and/or
III. the Sgo1 protein and the satellite RNA are forming a complex after introduction into the oocyte or the egg,
preferably wherein the Sgo1, the Sgo2, the PP2A and/or the satellite RNA form a complex after introduction into the oocyte or the egg.

15. A kit comprising the pharmaceutical composition of claims 13 or 14, preferably wherein a needle, capillary or injection pen for microinjection comprises the pharmaceutical composition, preferably additionally comprising instructions for use.
